# EUROPEAN PATENT SPECIFICATION

(11) **EP 1 860 941 B1**
(45) Date of publication and mention of the grant of the patent: **12.11.2008**
(21) Application number: 06725075.3
(22) Date of filing: 15.03.2006
(51) Int. Cl.: A01N 43/40, C07D 213/42

(54) **BIPHENYL-N-(4-PYRIDYL) METHYLSUFONAMIDES**
BIPHENYL-N-(4-PYRIDYL) METHYLSULFONAMIDE
BIPHENYL-N-(4-PYRIDYL)METHYLSULFONAMIDES

(30) Priority: 16.03.2005 US 662411 P; 20.04.2005 DE 102005018464; 30.05.2005 EP 05011598; 24.02.2006 US 776551 P
(43) Date of publication of application: 05.12.2007
(73) Proprietor: BASF SE, 67056 Ludwigshafen (DE)
(72) Inventor: GRAMMENOS, Wassilios, 67071 Ludwigshafen (DE); RHEINHEIMER, Joachim, 67063 Ludwigshafen (DE); LOHMANN, Jan Klaas, 68167 Mannheim (DE); GROTE, Thomas, 67157 Wachenheim (DE); PUHL, Michael, 68623 Lampertheim (DE); KORADIN, Christopher, 67067 Ludwigshafen (DE); BAUMANN, Ernst, 67373 Dudenhofen (DE); VON DEYN, Wolfgang, 67435 Neustadt (DE); LANGEWALD, Jürgen, 68165 Mannheim (DE); GÖTZ, Norbert, 67547 Worms (DE); CULBERTSON, Deborah L., Fuquay Varina, NC 27526 (US); ANSPAUGH, Douglas D., Apex, NC 27502 (US); OLOUMI-SADEGHI, Hassan, Raleigh, NC 27614 (US); COTTER, Henry Van Tuyl, Raleigh, NC 27613 (US); KUHN, David G., Apex, NC 27502 (US)
(86) International application number: PCT/EP2006/060753
(87) International publication number: WO 2006/097489

(56) References cited:
- EP-A- 0 778 267
- WO-A-20/05033081

## Description

The present invention relates to biphenylsulfonamides of the formula I 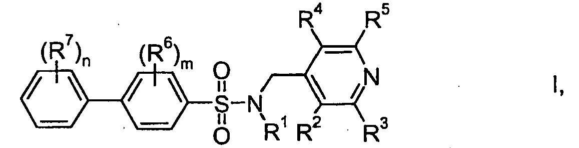 in which the substituents are as defined below:
- R¹: is hydrogen, C₁₋C₄-alkyl, C₁-C₄-alkoxy, C₂-C₄-alkenyl, C₂-C₄-alkynyl or benzyl;
- R², R³, R⁴, R⁵: independently of one another are hydrogen, halogen, C₁-C₄-alkyl, halomethyl, C₁-C₄-alkoxy, halomethoxy, C₁-C₄-alkylthio, C₁-C₄-alkylamino or di-(C₁-C₄-alkyl)amino;
- R⁶, R⁷: independently of one another are hydrogen, hydroxy, cyano, nitro, amino, halogen, C₁-C₈-alkyl, C₁-C₆-haloalkyl, C₁-C₈-alkoxy, C₁-C₈-haloalkoxy, C₁-C₆-alkylthio, (C₁-C₄-alkyl)carbonyl, (C₁-C₄-alkoxy)carbonyl, -C(R⁸)=NOR⁹, C₁-C₄-alkylamino, di(C₁-C₄-alkyl)amino, (C₁-C₄-alkyl)aminocarbonyl, di(C₁-C₄-alkyl)aminocarbonyl, phenyl or phenoxy, where the rings may carry one to three groups R¹⁰,
or
two radicals R⁶ or two radicals R⁷, together with two adjacent ring members of the phenyl ring to which they are attached, may form an annellated cyclopentyl, cyclohexyl or phenyl ring, whereas these annellated rings may carry themselves one or two radicals R¹⁰;
- R¹⁰: is halogen, cyano, nitro, C₁-C₄-alkyl, C₁-C₄-alkoxy, halomethyl or halomethoxy;
- R⁸: C₁-C₄-alkyl;
- R⁹: C₁-C₈-alkyl, C₁-C₄-haloalkyl, C₂-C₄-alkenyl, C₂-C₄-haloalkenyl, C₂-C₄-alkynyl, C₂-C₄-haloalkynyl or benzyl;
- m: is zero, 1, 2, 3 or 4;
- n: is zero, 1, 2, 3, 4 or 5;
with the proviso that m and n are not simultaneously zero if R², R³, R⁴ and R⁵ are all hydrogen;
and the N-oxides and the agriculturally acceptable salts and the veterinarily acceptable salts of the compounds I.

Moreover, the invention relates to processes for preparing these compounds, to compositions comprising them and to their use for controlling phytopathogenic harmful fungi.

The present invention also relates to the use of the biphenylsulfonamides I for combating arthropodal pests (harmful arthropodes) and for protecting materials against infestation and/or destruction by said pests.

WO 2005/033081 describes various pyridinosulfonamides. EP-A 206 581 and Lieb. Ann. Chem. 641 (1990) disclose individual 4-pyridinemethylsulfonamides. The compounds described in the publications mentioned are suitable for controlling harmful fungi.

The compounds according to this invention differ from those described in WO 2005/033081 by the substitution pattern of the biphenyl group.

However, in many cases their action is unsatisfactory. Based upon this, it is the object of the present invention to provide compounds having improved action and/or a broadened activity spectrum.

We have found that this object is achieved by the compounds defined at the outset. Furthermore, we have found processes and intermediates for their preparation, compositions comprising them and methods for controlling harmful fungi using the compounds I.

Compared to the known compounds, the compounds of the formula I have increased efficacy against harmful fungi.

Surprisingly, the compounds I, their N-oxides and salts are also suitable for controlling arthropodal pests, in particular for combating insects.

Likewise, the compounds I, their N-oxides and salts are also useful for combating arachnids.

The term "combating" as used herein comprises controlling, i.e. killing of pests and also protecting plants, non-living materials or seeds from an attack or infestation by said pests.

Accordingly, the invention further provides compositions for combating such pests, preferably in the form of directly sprayable solutions, emulsions, pastes, oil dispersions, powders, materials for scattering, dusts or in the form of granules, which comprises a pesticidally effective amount of at least one compound of the formula I or at least an N-oxide or a salt thereof and at least one carrier which may be liquid and/or solid and which is prefarably agronomically acceptable, and/or at least one surfactant.

Furthermore, the invention provides a method for combating such pests, which comprises contacting said pests, their habitat, breeding ground, food supply, plant, seed, soil, area, material or environment in which the animal pests are growing or may grow, or the materials, plants, seeds, soils, surfaces or spaces to be protected from an attack of or infestation by said pest, with a pesticidally effective amount of a compound of the formula I as defined herein or an N-oxide or a salt thereof.

The invention provides in particular a method for protecting crops, including seeds, from attack or infestation by arthropodal pests, said method comprises contacting a crop with a pesticidally effective amount of at least one compound of formula I as defined herein or with an N-oxide or a salt thereof.

The invention also provides a method for protecting non-living materials from attack or infestation by the aforementioned pests, which method comprises contacting the non-living material with a pesticidally effective amount of at least one compound of formula I as defined herein or with an N-oxide or a salt thereof.

Suitable compounds of the formula I encompass all possible stereoisomers (cis/trans isomers, enantiomers) which may occur and mixtures thereof. Stereoisomeric centers are e.g. the carbon and nitrogen atom of the -C(R⁸)=NOR⁹ moiety as well as asymmetric carbon atoms in the radicals R¹, R², R³, R⁴ and/or R⁵ etc. The present invention provides both the pure enantiomes or diastereomers or mixtures thereof, the pure cis- and trans-isomers and the mixtures thereof. The compounds of the general formula I may also exist in the form of different tautomers. The invention comprises the single tautomers, if seperable, as well as the tautomer mixtures.

The compounds of the formula I may be present in various crystal modifications which may differ in their biological activity. They also form part of the subject matter of the present invention.

Salts of the compounds of the formula I are preferably agriculturally acceptable salts. They can be formed in a customary method, e.g. by reacting the compound with an acid of the anion in question if the compound of formula I has a basic functionality or by reacting an acidic compound of formula I with a suitable base.

Suitable agriculturally useful salts are especially the salts of those cations or the acid addition salts of those acids whose cations and anions, respectively, do not have any adverse effect on the action of the compounds according to the present invention. Suitable cations are in particular the ions of the alkali metals, preferably lithium, sodium and potassium, of the alkaline earth metals, preferably calcium, magnesium and barium, and of the transition metals, preferably manganese, copper, zinc and iron, and also ammonium (NH₄⁺) and substituted ammonium in which one to four of the hydrogen atoms are replaced by C₁-C₄-alkyl, C₁-C₄-hydroxyalkyl, C₁-C₄-alkoxy, C₁-C₄-alkoxy-C₁-C₄-alkyl, hydroxy-C₁-C₄-alkoxy-C₁-C₄-alkyl, phenyl or benzyl. Examples of substituted ammonium ions comprise methylammonium, isopropylammonium, dimethylammonium, diisopropylammonium, trimethylammonium, tetramethylammonium, tetraethylammonium, tetrabutylammonium, 2-hydroxyethylammonium, 2-(2-hydroxyethoxy)ethylammonium, bis(2-hydroxyethyl)ammonium, benzyltrimethylammonium and benzyltriethylammonium, furthermore phosphonium ions, sulfonium ions, preferably tri(C₁-C₄-alkyl)sulfonium, and sulfoxonium ions, preferably tri(C₁-C₄-alkyl)sulfoxonium.

Anions of useful acid addition salts are primarily chloride, bromide, fluoride, hydrogen sulfate, sulfate, dihydrogen phosphate, hydrogen phosphate, phosphate, nitrate, hydrogen carbonate, carbonate, hexafluorosilicate, hexafluorophosphate, benzoate, and the anions of C₁-C₄-alkanoic acids, preferably formate, acetate, propionate and butyrate. They can be formed by reacting the compounds of the formulae Ia and Ib with an acid of the corresponding anion, preferably of hydrochloric acid, hydrobromic acid, sulfuric acid, phosphoric acid or nitric acid.

The organic moieties mentioned in the above definitions of the variables are - like the term halogen - collective terms for individual listings of the individual group members. The prefix Cₙ-Cₘ indicates in each case the possible number of carbon atoms in the group.
halogen: fluorine, chlorine, bromine and iodine;
alkyl and all alkyl moieties in alkylcarbonyl, alkylamino, dialkylamino, alkylaminocarbonyl, dialkylaminocarbonyl: saturated straight-chain or branched hydrocarbon radicals having 1 to 4, 6 or 8 carbon atoms, for example C₁-C₆-alkyl, such as methyl, ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 2,2-dimethylpropyl, 1-ethylpropyl, hexyl, 1,1-dimethylpropyl, 1,2-dimethylpropyl, 1-methylpentyl, 2-methylpentyl, 3-methylpentyl, 4-methylpentyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1,1,2-trimethylpropyl, 1,2,2-trimethylpropyl, 1-ethyl-1-methylpropyl and 1-ethyl-2-methylpropyl;
haloalkyl: straight-chain or branched alkyl groups having 1 to 2 or 4 carbon atoms (as mentioned above), where some or all of the hydrogen atoms in these groups may be replaced by halogen atoms as mentioned above: in particular C₁-C₂-haloalkyl, such as chloromethyl, bromomethyl, dichloromethyl, trichloromethyl, fluoromethyl, difluoromethyl, trifluoromethyl, chlorofluoromethyl, dichlorofluoromethyl, chlorodifluoromethyl, 1-chloroethyl, 1-bromoethyl, 1-fluoroethyl, 2-fluoroethyl, 2,2-difluoroethyl, 2,2,2-trifluoroethyl, 2-chloro-2-fluoroethyl, 2-chloro-2,2-difluoroethyl, 2,2-dichloro-2-fluoroethyl, 2,2,2-trichloroethyl, pentafluoroethyl or 1,1,1-trifluoroprop-2-yl;
alkenyl: unsaturated straight-chain or branched hydrocarbon radicals having 2 to 4, 6 or 8 carbon atoms and one or two double bonds in any position, for example C₂-C₆-alkenyl, such as ethenyl, 1-propenyl, 2-propenyl, 1-methylethenyl, 1-butenyl, 2-butenyl, 3-butenyl, 1-methyl-1-propenyl, 2-methyl-1-propenyl, 1-methyl-2-propenyl, 2-methyl-2-propenyl, 1-pentenyl, 2-pentenyl, 3-pentenyl, 4-pentenyl, 1-methyl-1-butenyl, 2-methyl-1-butenyl, 3-methyl-1-butenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-propenyl, 1,2-dimethyl-1-propenyl, 1,2-dimethyl-2-propenyl, 1-ethyl-1-propenyl, 1-ethyl-2-propenyl, 1-hexenyl, 2-hexenyl, 3-hexenyl, 4-hexenyl, 5-hexenyl, 1-methyl-1-pentenyl, 2-methyl-1-pentenyl, 3-methyl-1-pentenyl, 4-methyl-1-pentenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1-dimethyl-2-butenyl, 1,1-dimethyl-3-butenyl, 1,2-dimethyl-1-butenyl, 1,2-dimethyl-2-butenyl, 1,2-dimethyl-3-butenyl, 1,3-dimethyl-1-butenyl, 1,3-dimethyl-2-butenyl, 1,3-dimethyl-3-butenyl, 2,2-dimethyl-3-butenyl, 2,3-dimethyl-1-butenyl, 2,3-dimethyl-2-butenyl, 2,3-dimethyl-3-butenyl, 3,3-dimethyl-1-butenyl, 3,3-dimethyl-2-butenyl, 1-ethyl-1-butenyl, 1-ethyl-2-butenyl, 1-ethyl-3-butenyl, 2-ethyl-1-butenyl, 2-ethyl-2-butenyl, 2-ethyl-3-butenyl, 1,1,2-trimethyl-2-propenyl, 1-ethyl-1-methyl-2-propenyl, 1-ethyl-2-methyl-1-propenyl and 1-ethyl-2-methyl-2-propenyl;
haloalkenyl: unsaturated straight-chain or branched hydrocarbon radicals having 2 to 6 carbon atoms and one or two double bonds in any position (as mentioned above), where in these groups some or all of the hydrogen atoms may be replaced by halogen atoms as mentioned above, in particular by fluorine, chlorine and bromine;
alkynyl: straight-chain or branched hydrocarbon groups having 2 to 4, 6 or 8 carbon atoms and one or two triple bonds in any position, for example C₂-C₆-alkynyl, such as ethynyl, 1-propynyl, 2-propynyl, 1-butynyl, 2-butynyl, 3-butynyl, 1-methyl-2-propynyl, 1-pentynyl, 2-pentynyl, 3-pentynyl; 4-pentynyl, 1-methyl-2-butynyl, 1-methyl-3-butynyl, 2-methyl-3-butynyl, 3-methyl-1-butynyl, 1,1-dimethyl-2-propynyl, 1-ethyl-2-propynyl, 1-hexynyl, 2-hexynyl, 3-hexynyl, 4-hexynyl, 5-hexynyl, 1-methyl-2-pentynyl, 1-methyl-3-pentynyl, 1-methyl-4-pentynyl, 2-methyl-3-pentynyl, 2-methyl-4-pentynyl, 3-methyl-1-pentynyl, 3-methyl-4-pentynyl, 4-methyl-1-pentynyl, 4-methyl-2-pentynyl, 1,1-dimethyl-2-butynyl, 1,1-dimethyl-3-butynyl, 1,2-dimethyl-3-butynyl, 2,2-dimethyl-3-butynyl, 3,3-dimethyl-1-butynyl, 1-ethyl-2-butynyl, 1-ethyl-3-butynyl, 2-ethyl-3-butynyl and 1-ethyl-1-methyl-2-propynyl;
haloalkynyl: unsaturated straight-chain or branched hydrocarbon groups having 2 to 4 carbon atoms and one triple bond in any position (as mentioned above), where in these groups some or all of the hydrogen atoms may be replaced by halogen atoms as mentioned above, in particular by fluorine, chlorine and/or bromine;
five- to ten-membered saturated, partially unsaturated or aromatic heterocycle which contains one to four heteroatoms from the group consisting of O, N and S:
- 5- or 6-membered heterocyclyl which contains one to three nitrogen atoms and/or one oxygen or sulfur atom or one or two oxygen and/or sulfur atoms, for example 2-tetrahydrofuranyl, 3-tetrahydrofuranyl, 2-tetrahydrothienyl, 3-tetrahydrothienyl, 2-pyrrolidinyl, 3-pyrrolidinyl, 3-isoxazolidinyl, 4-isoxazolidinyl, 5-isoxazolidinyl, 3-isothiazolidinyl, 4-isothiazolidinyl, 5-isothiazolidinyl, 3-pyrazolidinyl, 4-pyrazolidinyl, 5-pyrazolidinyl, 2-oxazolidinyl, 4-oxazolidinyl, 5-oxazolidinyl, 2-thiazolidinyl, 4-thiazolidinyl, 5-thiazolidinyl, 2-imidazolidinyl, 4-imidazolidinyl, 2-pyrrolin-2-yl, 2-pyrrolin-3-yl, 3-pyrrolin-2-yl, 3-pyrrolin-3-yl, 2-piperidinyl, 3-piperidinyl, 4-piperidinyl, 1,3-dioxan-5-yl, 2-tetrahydropyranyl, 4-tetrahydropyranyl, 2-tetrahydrothienyl, 3-hexahydropyridazinyl, 4-hexahydropyridazinyl, 2-hexahydropyrimidinyl, 4-hexahydropyrimidinyl, 5-hexahydropyrimidinyl and 2-piperazinyl;
- 5-membered heteroaryl which contains one to four nitrogen atoms or one to three nitrogen atoms and one sulfur or oxygen atom: 5-membered heteroaryl groups which, in addition to carbon atoms, may contain one to four nitrogen atoms or one to three nitrogen atoms and one sulfur or oxygen atom as ring members, for example 2-thienyl, 3-thienyl, 3-pyrazolyl, 4-pyrazolyl, 5-pyrazolyl, 2-oxazolyl, 4-oxazolyl, 5-oxazolyl, 2-thiazolyl, 4-thiazolyl, 5-thiazolyl, 2-imidazolyl, 4-imidazolyl and 1,3,4-triazol-2-yl;
- 6-membered heteroaryl which contains one to three or one to four nitrogen atoms: 6-membered heteroaryl groups which, in addition to carbon atoms, may contain one to three or one to four nitrogen atoms as ring members, for example 2-pyridinyl, 3-pyridinyl, 4-pyridinyl, 3-pyridazinyl, 4-pyridazinyl, 2-pyrimidinyl, 4-pyrimidinyl, 5-pyrimidinyl and 2-pyrazinyl.

The scope of the present invention includes the (R)- and (S)-isomers and the racemates of compounds of the formula I having chiral centers.

With a view to the intended use of the biphenylsulfonamides of the formula I, particular preference is given to the following meanings of the substituents, in each case on their own or in combination:

The invention preferably provides compounds of the formula I in which R¹ is hydrogen or methyl, in particular hydrogen.

Preference is also given to compounds of the formula I in which R², R³, R⁴ and R⁵ independently of one another are hydrogen, methyl, ethyl, fluorine, chlorine, CF₃, OCH₃, OC₂H₅, OCHF₂, OCF₃, SCH₃ or SC₂H₅, or in particular hydrogen.

In addition, preference is also given to compounds of the formula I in which at least one, in particular one or two, radical(s) selected from R², R³, R⁴ and R⁵ is/are not hydrogen. In this case, that radical(s) mean preferably chlorine, methyl or methoxy.

In this regard, preference is given to compounds I in which R² and R⁵ are hydrogen.

Likewise, preference is given to compounds I in which R⁴ and R⁵ are hydrogen.

Preference is likewise given to compounds of the formula I which are substituted by two identical groups selected from R², R³; R⁴ and R⁵.

R⁶ is preferably halogen, in particular chlorine and fluorine; C₁-C₄-alkyl, in particular methyl and ethyl; C₁-C₄-alkoxy, in particular methoxy and ethoxy; C₁-C₄-haloalkyl, in particular trifluoromethyl; C₁-C₄-haloalkoxy, in particular difluoromethoxy and trifluoromethoxy; C₁-C₄-thioalkyl, in particular thiomethyl; (C₁-C₄-alkoxy)carbonyl, in particular methoxycarbonyl and ethoxycarbonyl.
Particularly preferred are compounds I with R⁶ being halogen, C₁-C₄-alkyl, C₁-C₄-haloalkyl, C₁-C₄-alkoxy or C₁-C₄-haloalkoxy.

R⁷ is preferably halogen, in particular chlorine and fluorine; C₁-C₈-alkyl, in particular methyl, ethyl, isopropyl, tert.-butyl; C₁-C₈-alkoxy, in particular methoxy, ethoxy, isopropoxy, tert.-butoxy; C₁-C₄-haloalkyl, in particular trifluoromethyl and pentafluoroethyl; C₁-C₈-thioalkyl, in particular thiomethyl and thioethyl; C(R⁸)=NOR⁹, where R⁸ is preferably C₁-C₄-alkyl, in particular methyl and ethyl, and R⁹ is preferably C₁-C₆-alkyl, allyl, propargyl and benzyl; (C₁-C₈-alkoxy)carbonyl, in particular methoxycarbonyl and ethoxycarbonyl; (C₁-C₈-alkyl)carbonyl, in particular methylcarbonyl and ethylcarbonyl; (C₁-C₈-alkyl)aminocarbonyl; di(C₁-C₈-alkyl)aminocarbonyl; and phenyl, which may be substituted by halogen, such as chlorine or fluorine, C₁-C₄-alkyl, such as methyl and ethyl; C₁-C₄-alkoxy, such as methoxy and ethoxy; or halomethyl, such as trifluoromethyl. Particularly preferred are compounds I with R⁷ being halogen, C₁-C₄-alkyl, C₁-C₄-haloalkyl, C₁-C₄-alkoxy or C₁-C₄-haloalkoxy, in particular fluorine, chlorine, methyl, halomethyl, methoxy or halomethoxy.

Preference is also given to compounds I in which one R⁷ is in the para-position of the respective phenyl ring.

Further preferred embodiments of the formula I are, in each case per se, compounds of the formulae I.1 to I.5, where the variables are as defined for formula I:

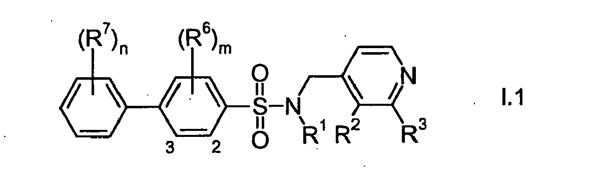

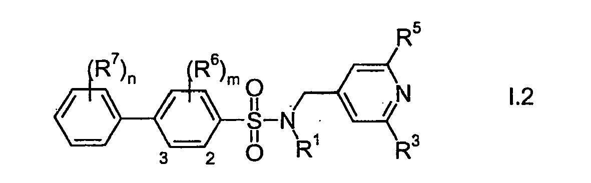

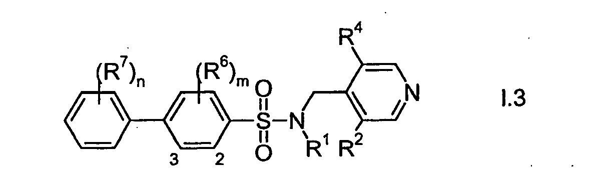

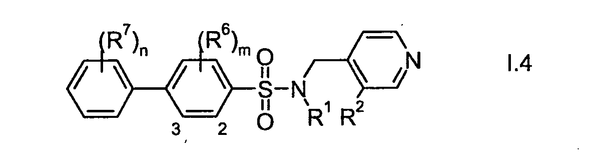

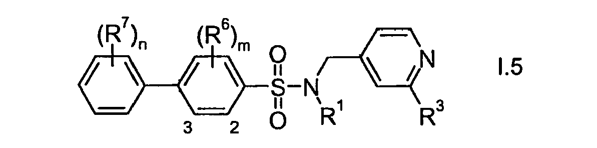

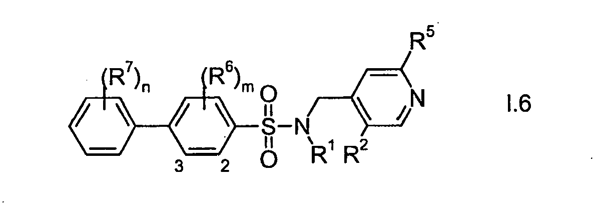

In a further preferred embodiment of the compounds according to the invention, the index m is zero.

In a further preferred embodiment of the compounds according to the invention, the index n is zero.

In another preferred embodiment of the compounds according to the invention, the index n is one to five, in particular two to five.

In one embodiment of the compounds according to the invention, one R⁶ is located ortho to the sulfonyl group; these compounds correspond to formula IA:

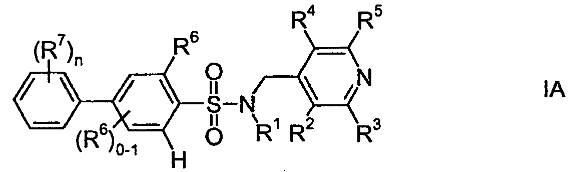

In a further embodiment of the compounds according to the invention, one R⁶ is located meta to the sulfonyl group; these compounds correspond to formula IB:

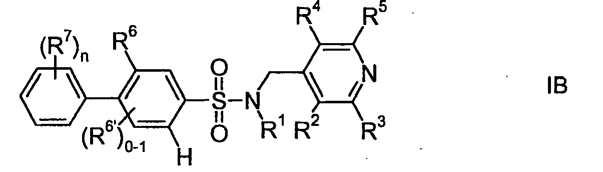

In further preferred embodiments of the compounds according to the invention, one group R⁷ is located in the 4-position (formula IC), in the 2-position (formula ID), two identical or different groups R⁷ are located in the 2,4-position (formula IE) or in the 3,4-position (formula IF) or in the 2,5-position (formula IG).
Particular preference is given to the compounds of the formula IC.

Furthermore, preference is given to the compounds of the formula ID.

In particular with a view to their use, preference is given to compounds I compiled in the tables below. Moreover, the groups mentioned for a substituent in the tables are per se, independently of the combination in which they are mentioned, a particularly preferred embodiment of the substituent in question.

### Table 1

Compounds of the formula I in which R¹, R², R³, R⁴ and R⁵ are hydrogen and the combination of (R⁶)ₘ and (R⁷)ₙ for a compound corresponds in each case to one row of Table A

### Table 2

Compounds of the formula I in which R¹ is methyl, R², R³, R⁴ and R⁵ are hydrogen and the combination of (R⁶)ₘ and (R⁷)ₙ for a compound corresponds in each case to one row of Table A

### Table 3

Compounds of the formula I.1 in which R¹ is hydrogen, R² and R³ are methyl and the combination of (R⁶)ₘ and (R⁷)ₙ for a compound corresponds in each case to one of rows A-1 to A-879 of Table A

### Table 4

Compounds of the formula I.1 in which R¹, R² and R³ are methyl and the combination of (R⁶)ₘ and (R⁷)ₙ for a compound corresponds in each case to one of rows A-1 to A-879 of Table A

### Table 5

Compounds of the formula I.1 in which R¹ is hydrogen, R² and R³ are fluorine and the combination of (R⁶)ₘ and (R⁷)ₙ for a compound corresponds in each case to one of rows A-1 to A-879 of Table A

### Table 6

Compounds of the formula I.1 in which R¹ is hydrogen, R² and R³ are chlorine and the combination of (R⁶)ₘ and (R⁷)ₙ for a compound corresponds in each case to one of rows A-1 to A-879 of Table A

### Table 7

Compounds of the formula I.1 in which R¹ is hydrogen, R² and R³ are methoxy and the combination of (R⁶)ₘ and (R⁷)ₙ for a compound corresponds in each case to one of rows A-1 to A-879 of Table A

### Table 8

Compounds of the formula I.1 in which R¹ is hydrogen, R² and R³ are trifluoromethoxy and the combination of (R⁶)ₘ and (R⁷)ₙ for a compound corresponds in each case to one of rows A-1 to A-879 of Table A

### Table 9

Compounds of the formula I.1 in which R¹ is hydrogen, R² and R³ are trifluoromethyl and the combination of (R⁶)ₘ and (R⁷)ₙ for a compound corresponds in each case to one of rows A-1 to A-879 of Table A

### Table 10

Compounds of the formula 1.2 in which R¹ is hydrogen, R³ and R⁵ are methyl and the combination of (R⁶)ₘ and (R⁷)ₙ for a compound corresponds in each case to one of rows A-1 to A-879 of Table A

### Table 11

Compounds of the formula 1.2 in which R¹ is hydrogen, R³ and R⁵ are fluorine and the combination of (R⁶)ₘ and (R⁷)ₙ for a compound corresponds in each case to one of rows A-1 to A-879 of Table A

### Table 12

Compounds of the formula I.2 in which R¹ is hydrogen, R³ and R⁵ are chlorine and the combination of (R⁶)ₘ and (R⁷)ₙ for a compound corresponds in each case to one of rows A-1 to A-879 of Table A

### Table 13

Compounds of the formula 1.2 in which R¹ is hydrogen, R³ and R⁵ are methoxy and the combination of (R⁶)ₘ and (R⁷)ₙ for a compound corresponds in each case to one of rows A-1 to A-879 of Table A

### Table 14

Compounds of the formula I.2 in which R¹ is hydrogen, R³ and R⁵ are trifluoromethoxy and the combination of (R⁶)ₘ and (R⁷)ₙ for a compound corresponds in each case to one of rows A-1 to A-879 of Table A

### Table 15

Compounds of the formula 1.2 in which R¹ is hydrogen, R³ and R⁵ are trifluoromethyl and the combination of (R⁶)ₘ and (R⁷)ₙ for a compound corresponds in each case to one of rows A-1 to A-879 of Table A

### Table16

Compounds of the formula I.3 in which R¹ is hydrogen, R² and R⁴ are methyl and the combination of (R⁶)ₘ and (R⁷)ₙ for a compound corresponds in each case to one of rows A-1 to A-879 of Table A

### Table 17

Compounds of the formula 1.3 in which R¹ is hydrogen, R² and R⁴ are fluorine and the combination of (R⁶)ₘ and (R⁷)ₙ for a compound corresponds in each case to one of rows A-1 to A-879 of Table A

### Table 18

Compounds of the formula I.3 in which R¹ is hydrogen, R² and R⁴ are chlorine and the combination of (R⁶)ₘ and (R⁷)ₙ for a compound corresponds in each case to one of rows A-1 to A-879 of Table A

### Table 19

Compounds of the formula 1.3 in which R¹ is hydrogen, R² and R⁴ are methoxy and the combination of (R⁶)ₘ and (R⁷)ₙ for a compound corresponds in each case to one of rows A-1 to A-879 of Table A

### Table 20

Compounds of the formula 1.3 in which R¹ is hydrogen, R² and R⁴ are trifluoromethoxy and the combination of (R⁶)ₘ and (R⁷)ₙ for a compound corresponds in each case to one of rows A-1 to A-879 of Table A

### Table 21

Compounds of the formula 1.3 in which R¹ is hydrogen, R² and R⁴ are trifluoromethyl and the combination of (R⁶)ₘ and (R⁷)ₙ for a compound corresponds in each case to one of rows A-1 to A-879 of Table A

### Table 22

Compounds of the formula I.4 in which R¹ is hydrogen, R² is methyl and the combination of (R⁶)ₘ and (R⁷)ₙ for a compound corresponds in each case to one of rows A-1 to A-879 of Table A

### Table 23

Compounds of the formula I.4 in which R¹ and R² are methyl and the combination of (R⁶)ₘ and (R⁷)ₙ for a compound corresponds in each case to one of rows A-1 to A-879 of Table A

### Table 24

Compounds of the formula I.4 in which R¹ is hydrogen, R² is fluorine and the combination of (R⁶)ₘ and (R⁷)ₙ for a compound corresponds in each case to one of rows A-1 to A-879 of Table A

### Table 25

Compounds of the formula I.4 in which R¹ is methyl, R² is fluorine and the combination of (R⁶)ₘ and (R⁷)ₙ for a compound corresponds in each case to one of rows A-1 to A-879 of Table A

### Table 26

Compounds of the formula I.4 in which R¹ is hydrogen, R² is chlorine and the combination of (R⁶)ₘ and (R⁷)ₙ for a compound corresponds in each case to one of rows A-1 to A-879 of Table A

### Table 27

Compounds of the formula I.4 in which R¹ is methyl, R² is chlorine and the combination of (R⁶)ₘ and (R⁷)ₙ for a compound corresponds in each case to one of rows A-1 to A-879 of Table A

### Table 28

Compounds of the formula I.4 in which R¹ is hydrogen, R² is methoxy and the combination of (R⁶)ₘ and (R⁷)ₙ for a compound corresponds in each case to one of rows A-1 to A-879 of Table A

### Table 29

Compounds of the formula I.4 in which R¹ is methyl, R² is methoxy and the combination of (R⁶)ₘ and (R⁷)ₙ for a compound corresponds in each case to one of rows A-1 to A-879 of Table A

### Table 30

Compounds of the formula I.4 in which R¹ is hydrogen, R² is ethoxy and the combination of (R⁶)ₘ and (R⁷)ₙ for a compound corresponds in each case to one of rows A-1 to A-879 of Table A

### Table 31

Compounds of the formula I.4 in which R¹ is hydrogen, R² is trifluoromethoxy and the combination of (R⁶)ₘ and (R⁷)ₙ for a compound corresponds in each case to one of rows A-1 to A-879 of Table A

### Table 32

Compounds of the formula I.4 in which R¹ is methyl, R² is trifluoromethoxy and the combination of (R⁶)ₘ and (R⁷)ₙ for a compound corresponds in each case to one of rows A-1 to A-879 of Table A

### Table 33

Compounds of the formula I.4 in which R¹ is hydrogen, R² is trifluoromethyl and the combination of (R⁶)ₘ and (R⁷)n for a compound corresponds in each case to one of rows A-1 to A-879 of Table A

### Table 34

Compounds of the formula I.4 in which R¹ is methyl, R² is trifluoromethyl and the combination of (R⁶)ₘ and (R⁷)ₙ for a compound corresponds in each case to one of rows A-1 to A-879 of Table A

### Table 35

Compounds of the formula 1.5 in which R¹ is hydrogen, R³ is methyl and the combination of (R⁶)ₘ and (R⁷)ₙ for a compound corresponds in each case to one of rows A-1 to A-879 of Table A

### Table 36

Compounds of the formula 1.5 in which R¹ is methyl, R³ is methyl and the combination of (R⁶)ₘ and (R⁷)ₙ for a compound corresponds in each case to one of rows A-1 to A-879 of Table A

### Table 37

Compounds of the formula 1.5 in which R¹ is hydrogen, R³ is fluorine and the combination of (R⁶)ₘ and (R⁷)ₙ for a compound corresponds in each case to one of rows A-1 to A-879 of Table A

### Table 38

Compounds of the formula 1.5 in which R¹ is methyl, R³ is fluorine and the combination of (R⁶)ₘ and (R⁷)ₙ for a compound corresponds in each case to one of rows A-1 to A-879 of Table A

### Table 39

Compounds of the formula 1.5 in which R¹ is hydrogen, R³ is chlorine and the combination of (R⁶)ₘ and (R⁷)ₙ for a compound corresponds in each case to one of rows A-1 to A-879 of Table A

### Table 40

Compounds of the formula 1.5 in which R¹ is methyl, R³ is chlorine and the combination of (R⁶)ₘ and (R⁷)ₙ for a compound corresponds in each case to one of rows A-1 to A-879 of Table A

### Table 41

Compounds of the formula I.5 in which R¹ is hydrogen, R³ is methoxy and the combination of (R⁶)ₘ and (R⁷)ₙ for a compound corresponds in each case to one of rows A-1 to A-879 of Table A

### Table 42

Compounds of the formula 1.5 in which R¹ is methyl, R³ is methoxy and the combination of (R⁶)ₘ and (R⁷)ₙ for a compound corresponds in each case to one of rows A-1 to A-879 of Table A

### Table 43

Compounds of the formula 1.5 in which R¹ is hydrogen, R³ is ethoxy and the combination of (R⁶)ₘ and (R⁷)ₙ for a compound corresponds in each case to one of rows A-1 to A-879 of Table A

### Table 44

Compounds of the formula 1.5 in which R¹ is hydrogen, R³ is trifluoromethoxy and the combination of (R⁶)ₘ and (R⁷)ₙ for a compound corresponds in each case to one of rows A-1 to A-879 of Table A

### Table 45

Compounds of the formula 1.5 in which R¹ is methyl, R³ is trifluoromethoxy and the combination of (R⁶)ₘ and (R⁷)ₙ for a compound corresponds in each case to one of rows A-1 to A-879 of Table A

### Table 46

Compounds of the formula 1.5 in which R¹ is hydrogen, R³ is trifluoromethyl and the combination of (R⁶)ₘ and (R⁷)ₙ for a compound corresponds in each case to one of rows A-1 to A-879 of Table A

### Table 47

Compounds of the formula 1.5 in which R¹ is methyl, R³ is trifluoromethyl and the combination of (R⁶)ₘ and (R⁷)ₙ for a compound corresponds in each case to one of rows A-1 to A-879 of Table A

### Table 48

Compounds of the formula I.6 in which R¹ is hydrogen, R² and R⁵ are methyl and the combination of (R⁶)ₘ and (R⁷)ₙ for a compound corresponds in each case to one of rows A-1 to A-879 of Table A

### Table 49

Compounds of the formula I.6 in which R¹ is hydrogen, R² and R⁵ are fluorine and the combination of (R⁶)ₘ and (R⁷)ₙ for a compound corresponds in each case to one of rows A-1 to A-879 of Table A

### Table 50

Compounds of the formula 1.6 in which R¹ is hydrogen, R² and R⁵ are chlorine and the combination of (R⁶)ₘ and (R⁷)ₙ for a compound corresponds in each case to one of rows A-1 to A-879 of Table A

### Table 51

Compounds of the formula I.6 in which R¹ is hydrogen, R² and R⁵ are methoxy and the combination of (R⁶)ₘ and (R⁷)ₙ for a compound corresponds in each case to one of rows A-1 to A-879 of Table A

### Table 52

Compounds of the formula 1.6 in which R¹ is hydrogen, R² and R⁵ are trifluoromethoxy and the combination of (R⁶)ₘ and (R⁷)ₙ for a compound corresponds in each case to one of rows A-1 to A-879 of Table A

### Table 53

Compounds of the formula 1.6 in which R¹ is hydrogen, R² and R⁵ are trifluoromethyl and the combination of (R⁶)ₘ and (R⁷)ₙ for a compound corresponds in each case to one of rows A-1 to A-879 of Table A

**Table A**

| **No.** | **(R⁶)ₘ** | **(R⁷)n** |
|---|---|---|
| A-1 | 2-F | - |
| A-2 | 2-Cl | - |
| A-3 | 2-CH₃ | - |
| A-4 | 2-CF₃ | - |
| A-5 | 2-OCH₃ | - |
| A-6 | 3-F | - |
| A-7 | 3-Cl | - |
| A-8 | 3-CH₃ | - |
| A-9 | 3-CF₃ | - |
| A-10 | 3-OCH₃ | - |
| A-11 | - | 2-CH3 |
| A-12 | 2-F | 2-CH₃ |
| A-13 | 2-Cl | 2-CH₃ |
| A-14 | 2-CH₃ | 2-CH₃ |
| A-15 | 2-CF₃ | 2-CH₃ |
| A-16 | 2-OCH₃ | 2-CH₃ |
| A-17 | 3-F | 2-CH₃ |
| A-18 | 3-Cl | 2-CH₃ |
| A-19 | 3-CH₃ | 2-CH₃ |
| A-20 | 3-CF₃ | 2-CH₃ |
| A-21 | 3-OCH₃ | 2-CH₃ |
| A-22 | - | 2-CH₂CH₃ |
| A-23 | 2-F | 2-CH₂CH₃ |
| A-24 | 2-Cl | 2-CH₂CH₃ |
| A-25 | 2-CH₃ | 2-CH₂CH₃ |
| A-26 | 2-CF₃ | 2-CH₂CH₃ |
| A-27 | 2-OCH₃ | 2-CH₂CH₃ |
| A-28 | 3-F | 2-CH₂CH₃ |
| A-29 | 3-Cl | 2-CH₂CH₃ |
| A-30 | 3-CH₃ | 2-CH₂CH₃ |
| A-31 | 3-CF₃ | 2-CH₂CH₃ |
| A-32 | 3-OCH₃ | 2-CH₂CH₃ |
| A-33 | - | 2-Br |
| A-34 | 2-F | 2-Br |
| A-35 | 2-Cl | 2-Br |
| A-36 | 2-CH₃ | 2-Br |
| A-37 | 2-CF₃ | 2-Br |
| A-38 | 2-OCH₃ | 2-Br |
| A-39 | 3-F | 2-Br |
| A-40 | 3-Cl | 2-Br |
| A-41 | 3-CH₃ | 2-Br |
| A-42 | 3-CF₃ | 2-Br |
| A-43 | 3-OCH₃ | 2-Br |
| A-44 | - | 2-Cl |
| A-45 | 2-F | 2-Cl |
| A-46 | 2-Cl | 2-Cl |
| A-47 | 2-CH₃ | 2-Cl |
| A-48 | 2-CF₃ | 2-Cl |
| A-49 | 2-OCH₃ | 2-Cl |
| A-50 | 3-F | 2-Cl |
| A-51 | 3-Cl | 2-Cl |
| A-52 | 3-CH₃ | 2-Cl |
| A-53 | 3-CF₃ | 2-Cl |
| A-54 | 3-OCH₃ | 2-Cl |
| A-55 | - | 2-F |
| A-56 | 2-F | 2-F |
| A-57 | 2-Cl | 2-F |
| A-58 | 2-CH₃ | 2-F |
| A-59 | 2-CF₃ | 2-F |
| A-60 | 2-OCH₃ | 2-F |
| A-61 | 3-F | 2-F |
| A-62 | 3-Cl | 2-F |
| A-63 | 3-CH₃ | 2-F |
| A-64 | 3-CF₃ | 2-F |
| A-65 | 3-OCH₃ | 2-F |
| A-66 | - | 2-CN |
| A-67 | 2-F | 2-CN |
| A-68 | 2-Cl | 2-CN |
| A-69 | 2-CH₃ | 2-CN |
| A-70 | 2-CF₃ | 2-CN |
| A-71 | 2-OCH₃ | 2-CN |
| A-72 | 3-F | 2-CN |
| A-73 | 3-Cl | 2-CN |
| A-74 | 3-CH₃ | 2-CN |
| A-75 | 3-CF₃ | 2-CN |
| A-76 | 3-OCH₃ | 2-CN |
| A-77 | - | 2-NO₂ |
| A-78 | 2-F | 2-NO₂ |
| A-79 | 2-Cl | 2-NO₂ |
| A-80 | 2-CH₃ | 2-NO₂ |
| A-81 | 2-CF₃ | 2-NO₂ |
| A-82 | 2-OCH₃ | 2-NO₂ |
| A-83 | 3-F | 2-NO₂ |
| A-84 | 3-Cl | 2-NO₂ |
| A-85 | 3-CH₃ | 2-NO₂ |
| A-86 | 3-CF₃ | 2-NO₂ |
| A-87 | 3-OCH₃ | 2-NO₂ |
| A-88 | - | 2-OCH₃ |
| A-89 | 2-F | 2-OCH₃ |
| A-90 | 2-Cl | 2-OCH₃ |
| A-91 | 2-CH₃ | 2-OCH₃ |
| A-92 | 2-CF₃ | 2-OCH₃ |
| A-93 | 2-OCH₃ | 2-OCH₃ |
| A-94 | 3-F | 2-OCH₃ |
| A-95 | 3-Cl . | 2-OCH₃ |
| A-96 | 3-CH₃ | 2-OCH₃ |
| A-97 | 3-CF₃ | 2-OCH₃ |
| A-98 | 3-OCH₃ | 2-OCH₃ |
| A-99 | - | 2-CF₃ |
| A-100 | 2-F | 2-CF₃ |
| A-101 | 2-Cl | 2-CF₃ |
| A-102 | 2-CH₃ | 2-CF₃ |
| A-103 | 2-CF₃ | 2-CF₃ |
| A-104 | 2-OCH₃ | 2-CF₃ |
| A-105 | 3-F | 2-CF₃ |
| A-106 | 3-Cl | 2-CF₃ |
| A-107 | 3-CH₃ | 2-CF₃ |
| A-108 | 3-CF₃ | 2-CF₃ |
| A-109 | 3-OCH₃ | 2-CF₃ |
| A-110 | H | 2-SCH₃ |
| A-111 | 2-F | 2-SCH₃ |
| A-112 | 2-Cl | 2-SCH₃ |
| A-113 | 2-CH₃ | 2-SCH₃ |
| A-114 | 2-CF₃ | 2-SCH₃ |
| A-115 | 2-OCH₃ | 2-SCH₃ |
| A-116 | 3-F | 2-SCH₃ |
| A-117 | 3-Cl | 2-SCH₃ |
| A-118 | 3-CH₃ | 2-SCH₃ |
| A-119 | 3-CF₃ | 2-SCH₃ |
| A-120 | 3-OCH₃ | 2-SCH₃ |
| A-121 | - | 2-C(=O)CH₃ |
| A-122 | 2-F | 2-C(=O)CH₃ |
| A-123 | 2-Cl | 2-C(=O)CH₃ |
| A-124 | 2-CH₃ | 2-C(=O)CH₃ |
| A-125 | 2-CF₃ | 2-C(=O)CH₃ |
| A-126 | 2-OCH₃ | 2-C(=O)CH₃ |
| A-127 | 3-F | 2-C(=O)CH₃ |
| A-128 | 3-Cl | 2-C(=O)CH₃ |
| A-129 | 3-CH₃ | 2-C(=O)CH₃ |
| A-130 | 3-CF₃ | 2-C(=O)CH₃ |
| A-131 | 3-OCH₃ | 2-C(=O)CH₃ |
| A-132 | - | 2-C(=NOCH₃)CH₃ |
| A-133 | 2-F | 2-C(=NOCH₃)CH₃ |
| A-134 | 2-Cl | 2-C(=NOCH₃)CH₃ |
| A-135 | 2-CH₃ | 2-C(=NOCH₃)CH₃ |
| A-136 | 2-CF₃ | 2-C(=NOCH₃)CH₃ |
| A-137 | 2-OCH₃ | 2-C(=NOCH₃)CH₃ |
| A-138 | 3-F | 2-C(=NOCH₃)CH₃ |
| A-139 | 3-Cl | 2-C(=NOCH₃)CH₃ |
| A-140 | 3-CH₃ | 2-C(=NOCH₃)CH₃ |
| A-141 | 3-CF₃ | 2-C(=NOCH₃)CH₃ |
| A-142 | 3-OCH₃ | 2-C(=NOCH₃)CH₃ |
| A-143 | - | 3-CH₃ |
| A-144 | 2-F | 3-CH₃ |
| A-145 | 2-Cl | 3-CH₃ |
| A-146 | 2-CH₃ | 3-CH₃ |
| A-147 | 2-CF₃ | 3-CH₃ |
| A-148 | 2-OCH₃ | 3-CH₃ |
| A-149 | 3-F | 3-CH₃ |
| A-150 | 3-Cl | 3-CH₃ |
| A-151 | 3-CH₃ | 3-CH₃ |
| A-152 | 3-CF₃ | 3-CH₃ |
| A-153 | 3-OCH₃ | 3-CH₃ |
| A-154 | - | 3-CH₂CH₃ |
| A-155 | 2-F | 3-CH₂CH₃ |
| A-156 | 2-Cl | 3-CH₂CH₃ |
| A-157 | 2-CH₃ | 3-CH₂CH₃ |
| A-158 | 2-CF₃ | 3-CH₂CH₃ |
| A-159 | 2-OCH | 3-CH₂CH₃ |
| A-160 | 3-F | 3-CH₂CH₃ |
| A-161 | 3-Cl | 3-CH₂CH₃ |
| A-162 | 3-CH₃ | 3-CH₂CH₃ |
| A-163 | 3-CF₃ | 3-CH₂CH₃ |
| A-164 | 3-OCH₃ | 3-CH₂CH₃ |
| A-165 | - | 3-Br |
| A-166 | 2-F | 3-Br |
| A-167 | 2-Cl | 3-Br |
| A-168 | 2-CH₃ | 3-Br |
| A-169 | 2-CF₃ | 3-Br |
| A-170 | 2-OCH₃ | 3-Br |
| A-171 | 3-F | 3-Br |
| A-172 | 3-Cl | 3-Br |
| A-173 | 3-CH₃ | 3-Br |
| A-174 | 3-CF₃ | 3-Br |
| A-175 | 3-OCH₃ | 3-Br |
| A-176 | - | 3-Cl |
| A-177 | 2-F | 3-Cl |
| A-178 | 2-Cl | 3-Cl |
| A-179 | 2-CH₃ | 3-Cl |
| A-180 | 2-CF₃ | 3-Cl |
| A-181 | 2-OCH₃ | 3-Cl |
| A-182 | 3-F | 3-Cl |
| A-183 | 3-Cl | 3-Cl |
| A-184 | 3-CH₃ | 3-Cl |
| A-185 | 3-CF₃ | 3-Cl |
| A-186 | 3-OCH₃ | 3-Cl |
| A-187 | - | 3-F |
| A-188 | 2-F | 3-F |
| A-189 | 2-Cl | 3-F |
| A-190 | 2-CH₃ | 3-F |
| A-191 | 2-CF₃ | 3-F |
| A-192 | 2-OCH₃ | 3-F |
| A-193 | 3-F | 3-F |
| A-194 | 3-Cl | 3-F |
| A-195 | 3-CH₃ | 3-F |
| A-196 | 3-CF₃ | 3-F |
| A-197 | 3-OCH₃ | 3-F |
| A-198 | - | 3-CN |
| A-199 | 2-F | 3-CN |
| A-200 | 2-Cl | 3-CN |
| A-201 | 2-CH₃ | 3-CN |
| A-202 | 2-CF₃ | 3-CN |
| A-203 | 2-OCH₃ | 3-CN |
| A-204 | 3-F | 3-CN |
| A-205 | 3-Cl | 3-CN |
| A-206 | 3-CH₃ | 3-CN |
| A-207 | 3-CF₃ | 3-CN |
| A-208 | 3-OCH₃ | 3-CN |
| A-209 | - | 3-NO₂ |
| A-210 | 2-F | 3-NO₂ |
| A-211 | 2-Cl | 3-NO₂ |
| A-212 | 2-CH₃ | 3-NO₂ |
| A-213 | 2-CF₃ | 3-NO₂ |
| A-214 | 2-OCH₃ | 3-NO₂ |
| A-215 | 3-F | 3-NO₂ |
| A-216 | 3-Cl | 3-NO₂ |
| A-217 | 3-CH₃ | 3-NO₂ |
| A-218 | 3-CF₃ | 3-NO₂ |
| A-219 | 3-OCH₃ | 3-NO₂ |
| A-220 | - | 3-OCH₃ |
| A-221 | 2-F | 3-OCH₃ |
| A-222 | 2-Cl | 3-OCH₃ |
| A-223 | 2-CH₃ | 3-OCH₃ |
| A-224 | 2-CF₃ | 3-OCH₃ |
| A-225 | 2-OCH₃ | 3-OCH₃ |
| A-226 | 3-F | 3-OCH₃ |
| A-227 | 3-Cl | 3-OCH₃ |
| A-228 | 3-CH₃ | 3-OCH₃ |
| A-229 | 3-CF₃ | 3-OCH₃ |
| A-230 | 3-OCH₃ | 3-OCH₃ |
| A-231 | - | 3-CF₃ |
| A-232 | 2-F | 3-CF₃ |
| A-233 | 2-Cl | 3-CF₃ |
| A-234 | 2-CH₃ | 3-CF₃ |
| A-235 | 2-CF₃ | 3-CF₃ |
| A-236 | 2-OCH₃ | 3-CF₃ |
| A-237 | 3-F | 3-CF₃ |
| A-238 | 3-Cl | 3-CF₃ |
| A-239 | 3-CH₃ | 3-CF₃ |
| A-240 | 3-CF₃ | 3-CF₃ |
| A-241 | 3-OCH₃ | 3-CF₃ |
| A-242 | - | 3-SCH₃ |
| A-243 | 2-F | 3-SCH₃ |
| A-244 | 2-Cl | 3-SCH₃ |
| A-245 | 2-CH₃ | 3-SCH₃ |
| A-246 | 2-CF₃ | 3-SCH₃ |
| A-247 | 2-OCH₃ | 3-SCH₃ |
| A-248 | 3-F | 3-SCH₃ |
| A-249 | 3-Cl | 3-SCH₃ |
| A-250 | 3-CH₃ | 3-SCH₃ |
| A-251 | 3-CF₃ | 3-SCH₃ |
| A-252 | 3-OCH₃ | 3-SCH₃ |
| A-253 | - | 3-C(=O)CH₃ |
| A-254 | 2-F | 3-C(=O)CH₃ |
| A-255 | 2-Cl | 3-C(=O)CH₃ |
| A-256 | 2-CH₃ | 3-C(=O)CH₃ |
| A-257 | 2-CF₃ | 3-C(=O)CH₃ |
| A-258 | 2-OCH₃ | 3-C(=O)CH₃ |
| A-259 | 3-F | 3-C(=O)CH₃ |
| A-260 | 3-Cl | 3-C(=O)CH₃ |
| A-261 | 3-CH₃ | 3-C(=O)CH₃ |
| A-262 | 3-CF₃ | 3-C(=O)CH₃ |
| A-263 | 3-OCH₃ | 3-C(=O)CH₃ |
| A-264 | - | 3-C(=NOCH₃)CH₃ |
| A-265 | 2-F | 3-C(=NOCH₃)CH₃ |
| A-266 | 2-Cl | 3-C(=NOCH₃)CH₃ |
| A-267 | 2-CH₃ | 3-C(=NOCH₃)CH₃ |
| A-268 | 2-CF₃ | 3-C(=NOCH₃)CH₃ |
| A-269 | 2-OCH₃ | 3-C(=NOCH₃)CH₃ |
| A-270 | 3-F | 3-C(=NOCH₃)CH₃ |
| A-271 | 3-Cl | 3-C(=NOCH₃)CH₃ |
| A-272 | 3-CH₃ | 3-C(=NOCH₃)CH₃ |
| A-273 | 3-CF₃ | 3-C(=NOCH₃)CH₃ |
| A-274 | 3-OCH₃ | 3-C(=NOCH₃)CH₃ |
| A-275 | - | 4-CH₃ |
| A-276 | 2-F | 4-CH₃ |
| A-277 | 2-Cl | 4-CH₃ |
| A-278 | 2-CH₃ | 4-CH₃ |
| A-279 | 2-CF₃ | 4-CH₃ |
| A-280 | 2-OCH₃ | 4-CH₃ |
| A-281 | 3-F | 4-CH₃ |
| A-282 | 3-Cl | 4-CH₃ |
| A-283 | 3-CH₃ | 4-CH₃ |
| A-284 | 3-CF₃ | 4-CH₃ |
| A-285 | 3-OCH₃ | 4-CH₃ |
| A-286 | - | 4-CH₂CH₃ |
| A-287 | 2-F | 4-CH₂CH₃ |
| A-288 | 2-Cl | 4-CH₂CH₃ |
| A-289 | 2-CH₃ | 4-CH₂CH₃ |
| A-290 | 2-CF₃ | 4-CH₂CH₃ |
| A-291 | 2-OCH₃ | 4-CH₂CH₃ |
| A-292 | 3-F | 4-CH₂CH₃ |
| A-293 | 3-Cl | 4-CH₂CH₃ |
| A-294 | 3-CH₃ | 4-CH₂CH₃ |
| A-295 | 3-CF₃ | 4-CH₂CH₃ |
| A-296 | 3-OCH₃ | 4-CH₂CH₃ |
| A-297 | - | 4-Br |
| A-298 | 2-F | 4-Br |
| A-299 | 2-Cl | 4-Br |
| A-300 | 2-CH₃ | 4-Br |
| A-301 | 2-CF₃ | 4-Br |
| A-302 | 2-OCH₃ | 4-Br |
| A-303 | 3-F | 4-Br |
| A-304 | 3-Cl | 4-Br |
| A-305 | 3-CH₃ | 4-Br |
| A-306 | 3-CF₃ | 4-Br |
| A-307 | 3-OCH₃ | 4-Br |
| A-308 | - | 4-Cl |
| A-309 | 2-F | 4-Cl |
| A-310 | 2-Cl | 4-Cl |
| A-311 | 2-CH₃ | 4-Cl |
| A-312 | 2-CF₃ | 4-Cl |
| A-313 | 2-OCH₃ | 4-Cl |
| A-314 | 3-F | 4-Cl |
| A-315 | 3-Cl | 4-Cl |
| A-316 | 3-CH₃ | 4-Cl |
| A-317 | 3-CF₃ | 4-Cl |
| A-318 | 3-OCH₃ | 4-Cl |
| A-319 | - | 4-F |
| A-320 | 2-F | 4-F |
| A-321 | 2-Cl | 4-F |
| A-322 | 2-CH₃ | 4-F |
| A-323 | 2-CF₃ | 4-F |
| A-324 | 2-OCH₃ | 4-F |
| A-325 | 3-F | 4-F |
| A-326 | 3-Cl | 4-F |
| A-327 | 3-CH₃ | 4-F |
| A-328 | 3-CF₃ | 4-F |
| A-329 | 3-OCH₃ | 4-F |
| A-330 | - | 4-CN |
| A-331 | 2-F | 4-CN |
| A-332 | 2-Cl | 4-CN |
| A-333 | 2-CH₃ | 4-CN |
| A-334 | 2-CF₃ | 4-CN |
| A-335 | 2-OCH₃ | 4-CN |
| A-336 | 3-F | 4-CN |
| A-337 | 3-Cl | 4-CN |
| A-338 | 3-CH₃ | 4-CN |
| A-339 | 3-CF₃ | 4-CN |
| A-340 | 3-OCH₃ | 4-CN |
| A-341 | - | 4-NO₂ |
| A-342 | 2-F | 4-NO₂ |
| A-343 | 2-Cl | 4-NO₂ |
| A-344 | 2-CH₃ | 4-NO₂ |
| A-345 | 2-CF₃ | 4-NO₂ |
| A-346 | 2-OCH₃ | 4-NO₂ |
| A-347 | 3-F | 4-NO₂ |
| A-348 | 3-Cl | 4-NO₂ |
| A-349 | 3-CH₃ | 4-NO₂ |
| A-350 | 3-CF₃ | 4-NO₂ |
| A-351 | 3-OCH₃ | 4-NO₂ |
| A-352 | - | 4-OCH₃ |
| A-353 | 2-F | 4-OCH₃ |
| A-354 | 2-Cl | 4-OCH₃ |
| A-355 | 2-CH₃ | 4-OCH₃ |
| A-356 | 2-CF₃ | 4-OCH₃ |
| A-357 | 2-OCH₃ | 4-OCH₃ |
| A-358 | 3-F | 4-OCH₃ |
| A-359 | 3-Cl | 4-OCH₃ |
| A-360 | 3-CH₃ | 4-OCH₃ |
| A-361 | 3-CF₃ | 4-OCH₃ |
| A-362 | 3-OCH₃ | 4-OCH₃ |
| A-363 | - | 4-OCH₂CH₃ |
| A-364 | 2-F | 4-OCH₂CH₃ |
| A-365 | 2-Cl | 4-OCH₂CH₃ |
| A-366 | 2-CH₃ | 4-OCH₂CH₃ |
| A-367 | 2-CF₃ | 4-OCH₂CH₃ |
| A-368 | 2-OCH₃ | 4-OCH₂CH₃ |
| A-369 | 3-F | 4-OCH₂CH₃ |
| A-370 | 3-Cl | 4-OCH₂CH₃ |
| A-371 | 3-CH₃ | 4-OCH₂CH₃ |
| A-372 | 3-CF₃ | 4-OCH₂CH₃ |
| A-373 | 3-OCH₃ | 4-OCH₂CH₃ |
| A-374 | - | 4-OCH(CH₃)₂ |
| A-375 | 2-F | 4-OCH(CH₃)₂ |
| A-376 | 2-Cl | 4-OCH(CH₃)₂ |
| A-377 | 2-CH₃ | 4-OCH(CH₃)₂ |
| A-378 | 2-CF₃ | 4-OCH(CH₃)₂ |
| A-379 | 2-OCH₃ | 4-OCH(CH₃)₂ |
| A-380 | 3-F | 4-OCH(CH₃)₂ |
| A-381 | 3-Cl | 4-OCH(CH₃)₂ |
| A-382 | 3-CH₃ | 4-OCH(CH₃)₂ |
| A-383 | 3-CF₃ | 4-OCH(CH₃)₂ |
| A-384 | 3-OCH₃ | 4-OCH(CH₃)₂ |
| A-385 | - | 4-OC(CH₃)₃ |
| A-386 | 2-F | 4-OC(CH₃)₃ |
| A-387 | 2-Cl | 4-OC(CH₃)₃ |
| A-388 | 2-CH₃ | 4-OC(CH₃)₃ |
| A-389 | 2-CF₃ | 4-OC(CH₃)₃ |
| A-390 | 2-OCH₃ | 4-OC(CH₃)₃ |
| A-391 | 3-F | 4-OC(CH₃)₃ |
| A-392 | 3-Cl | 4-OC(CH₃)₃ |
| A-393 | 3-CH₃ | 4-OC(CH₃)₃ |
| A-394 | 3-CF₃ | 4-OC(CH₃)₃ |
| A-395 | 3-OCH₃ | 4-OC(CH₃)₃ |
| A-396 | - | 4-CF₃ |
| A-397 | 2-F | 4-CF₃ |
| A-398 | 2-Cl | 4-CF₃ |
| A-399 | 2-CH₃ | 4-CF₃ |
| A-400 | 2-CF₃ | 4-CF₃ |
| A-401 | 2-OCH₃ | 4-CF₃ |
| A-402 | 3-F | 4-CF₃ |
| A-403 | 3-Cl | 4-CF₃ |
| A-404 | 3-CH₃ | 4-CF₃ |
| A-405 | 3-CF₃ | 4-CF₃ |
| A-406 | 3-OCH₃ | 4-CF₃ |
| A-407 | - | 4-SCH₃ |
| A-408 | 2-F | 4-SCH₃ |
| A-409 | 2-Cl | 4-SCH₃ |
| A-410 | 2-CH₃ | 4-SCH₃ |
| A-411 | 2-CF₃ | 4-SCH₃ |
| A-412 | 2-OCH₃ | 4-SCH₃ |
| A-413 | 3-F | 4-SCH₃ |
| A-414 | 3-Cl | 4-SCH₃ |
| A-415 | 3-CH₃ | 4-SCH₃ |
| A-416 | 3-CF₃ | 4-SCH₃ |
| A-417 | 3-OCH₃ | 4-SCH₃ |
| A-418 | - | 4-C(=O)CH₃ |
| A-419 | 2-F | 4-C(=O)CH₃ |
| A-420 | 2-Cl | 4-C(=O)CH₃ |
| A-421 | 2-CH₃ | 4-C(=O)CH₃ |
| A-422 | 2-CF₃ | 4-C(=O)CH₃ |
| A-423 | 2-OCH₃ | 4-C(=O)CH₃ |
| A-424 | 3-F | 4-C(=O)CH₃ |
| A-425 | 3-Cl | 4-C(=O)CH₃ |
| A-426 | 3-CH₃ | 4-C(=O)CH₃ |
| A-427 | 3-CF₃ | 4-C(=O)CH₃ |
| A-428 | 3-OCH₃ | 4-C(=O)CH₃ |
| A-429 | - | 4-C(=NOCH₃)CH₃ |
| A-430 | 2-F | 4-C(=NOCH₃)CH₃ |
| A-431 | 2-Cl | 4-C(=NOCH₃)CH₃ |
| A-432 | 2-CH₃ | 4-C(=NOCH₃)CH₃ |
| A-433 | 2-CF₃ | 4-C(=NOCH₃)CH₃ |
| A-434 | 2-OCH₃ | 4-C(=NOCH₃)CH₃ |
| A-435 | 3-F | 4-C(=NOCH₃)CH₃ |
| A-436 | 3-Cl | 4-C(=NOCH₃)CH₃ |
| A-437 | 3-CH₃ | 4-C(=NOCH₃)CH₃ |
| A-438 | 3-CF₃ | 4-C(=NOCH₃)CH₃ |
| A-439 | 3-OCH₃ | 4-C(=NOCH₃)CH₃ |
| A-440 | - | 4-C₆H₅ |
| A-441 | 2-F | 4-C₆H₅ |
| A-442 | 2-Cl | 4-C₆H₅ |
| A-443 | 2-CH₃ | 4-C₆H₅ |
| A-444 | 2-CF₃ | 4-C₆H₅ |
| A-445 | 2-OCH₃ | 4-C₆H₅ |
| A-446 | 3-F | 4-C₆H₅ |
| A-447 | 3-Cl | 4-C₆H₅ |
| A-448 | 3-CH₃ | 4-C₆H₅ |
| A-449 | 3-CF₃ | 4-C₆H₅ |
| A-450 | 3-OCH₃ | 4-C₆H₅ |
| A-451 | - | 2,4-(CH₃)₂ |
| A-452 | 2-F | 2,4-(CH₃)₂ |
| A-453 | 2-Cl | 2,4-(CH₃)₂ |
| A-454 | 2-CH₃ | 2,4-(CH₃)₂ |
| A-455 | 2-CF₃ | 2,4-(CH₃)₂ |
| A-456 | 2-OCH₃ | 2,4-(CH₃)₂ |
| A-457 | 3-F | 2,4-(CH₃)₂ |
| A-458 | 3-Cl | 2,4-(CH₃)₂ |
| A-459 | 3-CH₃ | 2,4-(CH₃)₂ |
| A-460 | 3-CF₃ | 2,4-(CH₃)₂ |
| A-461 | 3-OCH₃ | 2,4-(CH₃)₂ |
| A-462 | - | 2,4-(CH₂CH₃)₂ |
| A-463 | 2-F | 2,4-(CH₂CH₃)₂ |
| A-464 | 2-Cl | 2,4-(CH₂CH₃)₂ |
| A-465 | 2-CH₃ | 2,4-(CH₂CH₃)₂ |
| A-466 | 2-CF₃ | 2,4-(CH₂CH₃)₂ |
| A-467 | 2-OCH₃ | 2,4-(CH₂CH₃)₂ |
| A-468 | 3-F | 2,4-(CH₂CH₃)₂ |
| A-469 | 3-Cl | 2,4-(CH₂CH₃)₂ |
| A-470 | 3-CH₃ | 2,4-(CH₂CH₃)₂ |
| A-471 | 3-CF₃ | 2,4-(CH₂CH₃)₂ |
| A-472 | 3-OCH₃ | 2,4-(CH₂CH₃)₂ |
| A-473 | - | 2,4-Br₂ |
| A-474 | 2-F | 2,4-Br₂ |
| A-475 | 2-Cl | 2,4-Br₂ |
| A-476 | 2-CH₃ | 2,4-Br₂ |
| A-477 | 2-CF₃ | 2,4-Br₂ |
| A-478 | 2-OCH₃ | 2,4-Br₂ |
| A-479 | 3-F | 2,4-Br₂ |
| A-480 | 3-Cl | 2,4-Br₂ |
| A-481 | 3-CH₃ | 2,4-Br₂ |
| A-482 | 3-CF₃ | 2,4-Br₂ |
| A-483 | 3-OCH₃ | 2,4-Br₂ |
| A-484 | - | 2,4-Cl₂ |
| A-485 | 2-F | 2,4-Cl₂ |
| A-486 | 2-Cl | 2,4-Cl₂ |
| A-487 | 2-CH₃ | 2,4-Cl₂ |
| A-488 | 2-CF₃ | 2,4-Cl₂ |
| A-489 | 2-OCH₃ | 2,4-Cl₂ |
| A-490 | 3-F | 2,4-Cl₂ |
| A-491 | 3-Cl | 2,4-Cl₂ |
| A-492 | 3-CH₃ | 2,4-Cl₂ |
| A-493 | 3-CF₃ | 2,4-Cl₂ |
| A-494 | 3-OCH₃ | 2,4-Cl₂ |
| A-495 | - | 2.4-F₂ |
| A-496 | 2-F | 2,4-F₂ |
| A-497 | 2-Cl | 2,4-F₂ |
| A-498 | 2-CH₃ | 2,4-F₂ |
| A-499 | 2-CF₃ | 2,4-F₂ |
| A-500 | 2-OCH₃ | 2,4-F₂ |
| A-501 | 3-F | 2,4-F₂ |
| A-502 | 3-Cl | 2,4-F₂ |
| A-503 | 3-CH₃ | 2,4-F₂ |
| A-504 | 3-CF₃ | 2,4-F₂ |
| A-505 | 3-OCH₃ | 2,4-F₂ |
| A-506 | 2-F | 2,4-(OCH₃)₂ |
| A-507 | 2-Cl | 2,4-(OCH₃)₂ |
| A-508 | 2-CH₃ | 2,4-(OCH₃)₂ |
| A-509 | 2-CF₃ | 2,4-(OCH₃)₂ |
| A-510 | 2-OCH₃ | 2,4-(OCH₃)₂ |
| A-511 | 3-F | 2,4-(OCH₃)₂ |
| A-512 | 3-Cl | 2,4-(OCH₃)₂ |
| A-513 | 3-CH₃ | 2,4-(OCH₃)₂ |
| A-514 | 3-CF₃ | 2,4-(OCH₃)₂ |
| A-515 | 3-OCH₃ | 2,4-(OCH₃)₂ |
| A-516 | - | 2,4-(CF₃)₂ |
| A-517 | 2-F | 2,4-(CF₃)₂ |
| A-518 | 2-Cl | 2,4-(CF₃)₂ |
| A-519 | 2-CH₃ | 2,4-(CF₃)₂ |
| A-520 | 2-CF₃ | 2,4-(CF₃)₂ |
| A-521 | 2-OCH₃ | 2,4-(CF₃)₂ |
| A-522 | 3-F | 2,4-(CF₃)₂ |
| A-523 | 3-Cl | 2,4-(CF₃)₂ |
| A-524 | 3-CH₃ | 2,4-(CF₃)₂ |
| A-525 | 3-CF₃ | 2,4-(CF₃)₂ |
| A-526 | 3-OCH₃ | 2,4-(CF₃)₂ |
| A-527 | - | 2,5-(CH₃)₂ |
| A-528 | 2-F | 2,5-(CH₃)₂ |
| A-529 | 2-Cl | 2,5-(CH₃)₂ |
| A-530 | 2-CH₃ | 2,5-(CH₃)₂ |
| A-531 | 2-CF₃ | 2,5-(CH₃)₂ |
| A-532 | 2-OCH₃ | 2,5-(CH₃)₂ |
| A-533 | 3-F | 2,5-(CH₃)₂ |
| A-534 | 3-Cl | 2,5-(CH₃)₂ |
| A-535 | 3-CH₃ | 2,5-(CH₃)₂ |
| A-536 | 3-CF₃ | 2,5-(CH₃)₂ |
| A-537 | 3-OCH₃ | 2,5-(CH₃)₂ |
| A-538 | - | 2,5-Cl₂ |
| A-539 | 2-F | 2,5-Cl₂ |
| A-540 | 2-Cl | 2,5-Cl₂ |
| A-541 | 2-CH₃ | 2,5-Cl₂ |
| A-542 | 2-CF₃ | 2,5-Cl₂ |
| A-543 | 2-OCH₃ | 2,5-Cl₂ |
| A-544 | 3-F | 2,5-Cl₂ |
| A-545 | 3-Cl | 2,5-Cl₂ |
| A-546 | 3-CH₃ | 2,5-Cl₂ |
| A-547 | 3-CF₃ | 2,5-Cl₂ |
| A-548 | 3-OCH₃ | 2,5-Cl₂ |
| A-549 | - | 2,5-F₂ |
| A-550 | 2-F | 2,5-F₂ |
| A-551 | 2-Cl | 2,5-F₂ |
| A-552 | 2-CH₃ | 2,5-F₂ |
| A-553 | 2-CF₃ | 2,5-F₂ |
| A-554 | 2-OCH₃ | 2,5-F₂ |
| A-555 | 3-F | 2,5-F₂ |
| A-556 | 3-Cl | 2,5-F₂ |
| A-557 | 3-CH₃ | 2.5-F₂ |
| A-558 | 3-CF₃ | 2,5-F₂ |
| A-559 | 3-OCH₃ | 2,5-F₂ |
| A-560 | - | 2,5-(OCH₃)₂ |
| A-561 | 2-F | 2,5-(OCH₃)₂ |
| A-562 | 2-Cl | 2,5-(OCH₃)₂ |
| A-563 | 2-CH₃ | 2,5-(OCH₃)₂ |
| A-564 | 2-CF₃ | 2,5-(OCH₃)₂ |
| A-565 | 2-OCH₃ | 2,5-(OCH₃)₂ |
| A-566 | 3-F | 2,5-(OCH₃)₂ |
| A-567 | 3-Cl | 2,5-(OCH₃)₂ |
| A-568 | 3-CH₃ | 2,5-(OCH₃)₂ |
| A-569 | 3-CF₃ | 2,5-(OCH₃)₂ |
| A-570 | 3-OCH₃ | 2,5-(OCH₃)₂ |
| A-571 | - | 2,5-(CF₃)₂ |
| A-572 | 2-F | 2,5-(CF₃)₂ |
| A-573 | 2-Cl | 2,5-(CF₃)₂ |
| A-574 | 2-CH₃ | 2,5-(CF₃)₂ |
| A-575 | 2-CF₃ | 2,5-(CF₃)₂ |
| A-576 | 2-OCH₃ | 2,5-(CF₃)₂ |
| A-577 | 3-F | 2,5-(CF₃)₂ |
| A-578 | 3-Cl | 2,5-(CF₃)₂ |
| A-579 | 3-CH₃ | 2,5-(CF₃)₂ |
| A-580 | 3-CF₃ | 2,5-(CF₃)₂ |
| A-581 | 3-OCH₃ | 2,5-(CF₃)₂ |
| A-582 | - | 2,5-(SCH₃)₂ |
| A-583 | 2-F | 2,5-(SCH₃)₂ |
| A-584 | 2-Cl | 2,5-(SCH₃)₂ |
| A-585 | 2-CH₃ | 2,5-(SCH₃)₂ |
| A-586 | 2-CF₃ | 2,5-(SCH₃)₂ |
| A-587 | 2-OCH₃ | 2,5-(SCH₃)₂ |
| A-588 | 3-F | 2,5-(SCH₃)₂ |
| A-589 | 3-Cl | 2,5-(SCH₃)₂ |
| A-590 | 3-CH₃ | 2,5-(SCH₃)₂ |
| A-591 | 3-CF₃ | 2,5-(SCH₃)₂ |
| A-592 | 3-OCH₃ | 2,5-(SCH₃)₂ |
| A-593 | - | 2,6-(CH₃)₂ |
| A-594 | 2-F | 2,6-(CH₃)₂ |
| A-595 | 2-Cl | 2,6-(CH₃)₂ |
| A-596 | 2-CH₃ | 2,6-(CH₃)₂ |
| A-597 | 2-CF₃ | 2,6-(CH₃)₂ |
| A-598 | 2-OCH₃ | 2,6-(CH₃)₂ |
| A-599 | 3-F | 2,6-(CH₃)₂ |
| A-600 | 3-Cl | 2,6-(CH₃)₂ |
| A-601 | 3-CH₃ | 2,6-(CH₃)₂ |
| A-602 | 3-CF₃ | 2,6-(CH₃)₂ |
| A-603 | 3-OCH₃ | 2,6-(CH₃)₂ |
| A-604 | - | 3,5-(CH₃)₂ |
| A-605 | 2-F | 3,5-(CH₃)₂ |
| A-606 | 2-Cl | 3,5-(CH₃)₂ |
| A-607 | 2-CH₃ | 3,5-(CH₃)₂ |
| A-608 | 2-CF₃ | 3,5-(CH₃)₂ |
| A-609 | 2-OCH₃ | 3,5-(CH₃)₂ |
| A-610 | 3-F | 3,5-(CH₃)₂ |
| A-611 | 3-Cl | 3,5-(CH₃)₂ |
| A-612 | 3-CH₃ | 3,5-(CH₃)₂ |
| A-613 | 3-CF₃ | 3,5-(CH₃)₂ |
| A-614 | 3-OCH₃ | 3,5-(CH₃)₂ |
| A-615 | - | 3,5-(CF₃)₂ |
| A-616 | 2-F | 3,5-(CF₃)₂ |
| A-617 | 2-Cl | 3,5-(CF₃)₂ |
| A-618 | 2-CH₃ | 3,5-(CF₃)₂ |
| A-619 | 2-CF₃ | 3,5-(CF₃)₂ |
| A-620 | 2-OCH₃ | 3,5-(CF₃)₂ |
| A-621 | 3-F | 3,5-(CF₃)₂ |
| A-622 | 3-Cl | 3,5-(CF₃)₂ |
| A-623 | 3-CH₃ | 3,5-(CF₃)₂ |
| A-624 | 3-CF₃ | 3,5-(CF₃)₂ |
| A-625 | 3-OCH₃ | 3,5-(CF₃)₂ |
| A-626 | - | 3-Cl, 4-F |
| A-627 | 2-F | 3-Cl, 4-F |
| A-628 | 2-Cl | 3-Cl, 4-F |
| A-629 | 2-CH₃ | 3-Cl, 4-F |
| A-630 | 2-CF₃ | 3-Cl, 4-F |
| A-631 | 2-OCH₃ | 3-Cl, 4-F |
| A-632 | 3-F | 3-Cl, 4-F |
| A-633 | 3-Cl | 3-Cl, 4-F |
| A-634 | 3-CH₃ | 3-Cl, 4-F |
| A-635 | 3-CF₃ | 3-Cl, 4-F |
| A-636 | 3-OCH₃ | 3-Cl, 4-F |
| A-637 | - | 3-CH₃, 4-F |
| A-638 | 2-F | 3-CH₃, 4-F |
| A-639 | 2-Cl | 3-CH₃, 4-F |
| A-640 | 2-CH₃ | 3-CH₃, 4-F |
| A-641 | 2-CF₃ | 3-CH₃, 4-F |
| A-642 | 2-OCH₃ | 3-CH₃, 4-F |
| A-643 | 3-F | 3-CH₃, 4-F |
| A-644 | 3-Cl | 3-CH₃, 4-F |
| A-645 | 3-CH₃ | 3-CH₃, 4-F |
| A-646 | 3-CF₃ | 3-CH₃, 4-F |
| A-647 | 3-OCH₃ | 3-CH₃, 4-F |
| A-648 | - | 2-Cl, 4-F |
| A-649 | 2-F | 2-Cl, 4-F |
| A-650 | 2-Cl | 2-Cl, 4-F |
| A-651 | 2-CH₃ | 2-Cl, 4-F |
| A-652 | 2-CF₃ | 2-Cl, 4-F |
| A-653 | 2-OCH₃ | 2-Cl, 4-F |
| A-654 | 3-F | 2-Cl, 4-F |
| A-655 | 3-Cl | 2-Cl, 4-F |
| A-656 | 3-CH₃ | 2-Cl, 4-F |
| A-657 | 3-CF₃ | 2-Cl, 4-F |
| A-658 | 3-OCH₃ | 2-Cl, 4-F |
| A-659 | - | 2-Cl, 4-OCH₃ |
| A-660 | 2-F | 2-Cl, 4-OCH₃ |
| A-661 | 2-Cl | 2-Cl, 4-OCH₃ |
| A-662 | 2-CH₃ | 2-Cl, 4-OCH₃ |
| A-663 | 2-CF₃ | 2-Cl, 4-OCH₃ |
| A-664 | 2-OCH₃ | 2-Cl, 4-OCH₃ |
| A-665 | 3-F | 2-Cl, 4-OCH₃ |
| A-666 | 3-Cl | 2-Cl, 4-OCH₃ |
| A-667 | 3-CH₃ | 2-Cl, 4-OCH₃ |
| A-668 | 3-CF₃ | 2-Cl, 4-OCH₃ |
| A-669 | 3-OCH₃ | 2-Cl, 4-OCH₃ |
| A-670 | - | 2-F, 4-Cl |
| A-671 | 2-F | 2-F, 4-Cl |
| A-672 | 2-Cl | 2-F, 4-Cl |
| A-673 | 2-CH₃ | 2-F, 4-Cl |
| A-674 | 2-CF₃ | 2-F, 4-Cl |
| A-675 | 2-OCH₃ | 2-F, 4-Cl |
| A-676 | 3-F | 2-F, 4-Cl |
| A-677 | 3-Cl | 2-F, 4-Cl |
| A-678 | 3-CH₃ | 2-F, 4-Cl |
| A-679 | 3-CF₃ | 2-F, 4-Cl |
| A-680 | 3-OCH₃ | 2-F, 4-Cl |
| A-681 | - | 2-F,4-Br |
| A-682 | 2-F | 2-F, 4-Br |
| A-683 | 2-Cl | 2-F, 4-Br |
| A-684 | 2-CH₃ | 2-F, 4-Br |
| A-685 | 2-CF₃ | 2-F, 4-Br |
| A-686 | 2-OCH₃ | 2-F, 4-Br |
| A-687 | 3-F | 2-F,4-Br |
| A-688 | 3-Cl | 2-F, 4-Br |
| A-689 | 3-CH₃ | 2-F, 4-Br |
| A-690 | 3-CF₃ | 2-F, 4-Br |
| A-691 | 3-OCH₃ | 2-F, 4-Br |
| A-692 | - | 2-F, 4-CH₃ |
| A-693 | 2-F | 2-F, 4-CH₃ |
| A-694 | 2-Cl | 2-F, 4-CH₃ |
| A-695 | 2-CH₃ | 2-F, 4-CH₃ |
| A-696 | 2-CF₃ | 2-F, 4-CH₃ |
| A-697 | 2-OCH₃ | 2-F, 4-CH₃ |
| A-698 | 3-F | 2-F, 4-CH₃ |
| A-699 | 3-Cl | 2-F, 4-CH₃ |
| A-700 | 3-CH₃ | 2-F, 4-CH₃ |
| A-701 | 3-CF₃ | 2-F, 4-CH₃ |
| A-702 | 3-OCH₃ | 2-F, 4-CH₃ |
| A-703 | - | 2-F, 4-CF₃ |
| A-704 | 2-F | 2-F, 4-CF₃ |
| A-705 | 2-Cl | 2-F, 4-CF₃ |
| A-706 | 2-CH₃ | 2-F, 4-CF₃ |
| A-707 | 2-CF₃ | 2-F, 4-CF₃ |
| A-708 | 2-OCH₃ | 2-F, 4-CF₃ |
| A-709 | 3-F | 2-F, 4-CF₃ |
| A-71 0 | 3-Cl | 2-F, 4-CF₃ |
| A-711 | 3-CH₃ | 2-F, 4-CF₃ |
| A-712 | 3-CF₃ | 2-F, 4-CF₃ |
| A-713 | 3-OCH₃ | 2-F, 4-CF₃ |
| A-714 | - | 2-F, 4-OCH₃ |
| A-715 | 2-F | 2-F, 4-OCH₃ |
| A-716 | 2-Cl | 2-F, 4-OCH₃ |
| A-717 | 2-CH₃ | 2-F, 4-OCH₃ |
| A-718 | 2-CF₃ | 2-F, 4-OCH₃ |
| A-719 | 2-OCH₃ | 2-F, 4-OCH₃ |
| A-720 | 3-F | 2-F, 4-OCH₃ |
| A-721 | 3-Cl | 2-F, 4-OCH₃ |
| A-722 | 3-CH₃ | 2-F, 4-OCH₃ |
| A-723 | 3-CF₃ | 2-F, 4-OCH₃ |
| A-724 | 3-OCH₃ | 2-F, 4-OCH₃ |
| A-725 | - | 2-CH₃, 4-OCH₃ |
| A-726 | 2-F | 2-CH₃, 4-OCH₃ |
| A-727 | 2-Cl | 2-CH₃, 4-OCH₃ |
| A-728 | 2-CH₃ | 2-CH₃, 4-OCH₃ |
| A-729 | 2-CF₃ | 2-CH₃, 4-OCH₃ |
| A-730 | 2-OCH₃ | 2-CH₃, 4-OCH₃ |
| A-731 | 3-F | 2-CH₃, 4-OCH₃ |
| A-732 | 3-Cl | 2-CH₃, 4-OCH₃ |
| A-733 | 3-CH₃ | 2-CH₃, 4-OCH₃ |
| A-734 | 3-CF₃ | 2-CH₃, 4-OCH₃ |
| A-735 | 3-OCH₃ | 2-CH₃, 4-OCH₃ |
| A-736 | - | 2-CH₃, 4-Cl |
| A-737 | 2-F | 2-CH₃, 4-Cl |
| A-738 | 2-Cl | 2-CH₃, 4-Cl |
| A-739 | 2-CH₃ | 2-CH₃, 4-Cl |
| A-740 | 2-CF₃ | 2-CH₃, 4-Cl |
| A-741 | 2-OCH₃ | 2-CH_{3,} 4-Cl |
| A-742 | 3-F | 2-CH₃, 4-Cl |
| A-743 | 3-Cl | 2-CH₃, 4-Cl |
| A-744 | 3-CH₃ | 2-CH₃, 4-Cl |
| A-745 | 3-CF₃ | 2-CH_{3,} 4-Cl |
| A-746 | 3-OCH₃ | 2-CH₃, 4-Cl |
| A-747 | - | 2-CH₃, 4-F |
| A-748 | 2-F | 2-CH₃, 4-F |
| A-749 | 2-Cl | 2-CH₃, 4-F |
| A-750 | 2-CH₃ | 2-CH₃, 4-F |
| A-751 | 2-CF₃ | 2-CH₃, 4-F |
| A-752 | 2-OCH₃ | 2-CH₃, 4-F |
| A-753 | 3-F | 2-CH₃, 4-F |
| A-754 | 3-Cl | 2-CH₃, 4-F |
| A-755 | 3-CH₃ | 2-CH₃, 4-F |
| A-756 | 3-CF₃ | 2-CH₃, 4-F |
| A-757 | 3-OCH₃ | 2-CH₃, 4-F |
| A-758 | - | 2-CH₃, 4-CF₃ |
| A-759 | 2-F | 2-CH₃, 4-CF₃ |
| A-760 | 2-Cl | 2-CH₃, 4-CF₃ |
| A-761 | 2-CH₃ | 2-CH₃, 4-CF₃ |
| A-762 | 2-CF₃ | 2-CH₃, 4-CF₃ |
| A-763 | 2-OCH₃ | 2-CH₃, 4-CF₃ |
| A-764 | 3-F | 2-CH₃, 4-CF₃ |
| A-765 | 3-Cl | 2-CH₃, 4-CF₃ |
| A-766 | 3-CH₃ | 2-CH₃, 4-CF₃ |
| A-767 | 3-CF₃ | 2-CH₃, 4-CF₃ |
| A-768 | 3-OCH₃ | 2-CH₃, 4-CF₃ |
| A-769 | - | 2-OCH₃, 4-F |
| A-770 | 2-F | 2-OCH₃, 4-F |
| A-771 | 2-Cl | 2-OCH₃, 4-F |
| A-772 | 2-CH₃ | 2-OCH₃, 4-F |
| A-773 | 2-CF₃ | 2-OCH₃, 4-F |
| A-774 | 2-OCH₃ | 2-OCH₃, 4-F |
| A-775 | 3-F | 2-OCH₃, 4-F |
| A-776 | 3-Cl | 2-OCH₃, 4-F |
| A-777 | 3-CH₃ | 2-OCH₃, 4-F |
| A-778 | 3-CF₃ | 2-OCH₃, 4-F |
| A-779 | 3-OCH₃ | 2-OCH₃, 4-F |
| A-780 | - | 2-OCH₃, 4-Cl |
| A-781 | 2-F | 2-OCH₃, 4-Cl |
| A-782 | 2-Cl | 2-OCH₃, 4-Cl |
| A-783 | 2-CH₃ | 2-OCH₃, 4-Cl |
| A-784 | 2-CF₃ | 2-OCH₃, 4-Cl |
| A-785 | 2-OCH₃ | 2-OCH₃, 4-Cl |
| A-786 | 3-F | 2-OCH₃, 4-Cl |
| A-787 | 3-Cl | 2-OCH₃, 4-Cl |
| A-788 | 3-CH₃ | 2-OCH₃, 4-Cl |
| A-789 | 3-CF₃ | 2-OCH₃, 4-Cl |
| A-790 | 3-OCH₃ | 2-OCH₃, 4-Cl |
| A-791 | - | 3-NO₂, 4-CH₃ |
| A-792 | 2-F | 3-NO₂, 4-CH₃ |
| A-793 | 2-Cl | 3-NO₂, 4-CH₃ |
| A-794 | 2-CH₃ | 3-NO₂, 4-CH₃ |
| A-795 | 2-CF₃ | 3-NO₂, 4-CH₃ |
| A-796 | 2-OCH₃ | 3-NO₂, 4-CH₃ |
| A-797 | 3-F | 3-NO₂, 4-CH₃ |
| A-798 | 3-Cl | 3-NO₂, 4-CH₃ |
| A-799 | 3-CH₃ | 3-NO₂, 4-CH₃ |
| A-800 | 3-CF₃ | 3-NO₂, 4-CH₃ |
| A-801 | 3-OCH₃ | 3-NO₂, 4-CH₃ |
| A-802 | - | 2-CF₃, 4-Cl |
| A-803 | 3-F | 2-CF_{3,} 4-Cl |
| A-804 | 3-Cl | 2-CF₃, 4-Cl |
| A-805 | 3-CH₃ | 2-CF₃, 4-Cl |
| A-806 | 3-CF₃ | 2-CF₃, 4-Cl |
| A-807 | 3-OCF₃ | 2-CF₃, 4-Cl |
| A-808 | - | 2-Cl, 4-CF₃ |
| A-809 | 3-F | 2-Cl, 4-CF₃ |
| A-810 | 3-Cl | 2-Cl, 4-CF₃ |
| A-811 | 3-CH₃ | 2-Cl, 4-CF₃ |
| A-812 | 3-CF₃ | 2-Cl, 4-CF₃ |
| A-813 | 3-OCF₃ | 2-Cl, 4-CF₃ |
| A-814 | - | 2-CF₃, 4-OCH₃ |
| A-815 | 3-F | 2-CF₃, 4-OCH₃ |
| A-816 | 3-Cl | 2-CF₃, 4-OCH₃ |
| A-817 | 3-CH₃ | 2-CF₃, 4-OCH₃ |
| A-818 | 3-CF₃ | 2-CF₃, 4-OCH₃ |
| A-819 | 3-OCF₃ | 2-CF₃, 4-OCH₃ |
| A-820 | - | 3-Cl, 4-CF₃ |
| A-821 | 3-F | 3-Cl, 4-CF₃ |
| A-822 | 3-Cl | 3-Cl, 4-CF₃ |
| A-823 | 3-CH₃ | 3-Cl, 4-CF₃ |
| A-824 | 3-CF₃ | 3-Cl, 4-CF₃ |
| A-825 | 3-OCF₃ | 3-Cl, 4-CF₃ |
| A-826 | - | 2-CF₃, 5-F |
| A-827 | 3-F | 2-CF₃, 5-F |
| A-828 | 3-Cl | 2-CF₃, 5-F |
| A-829 | 3-CH₃ | 2-CF₃, 5-F |
| A-830 | 3-CF₃ | 2-CF₃, 5-F |
| A-831 | 3-OCF₃ | 2-CF₃, 5-F |
| A-832 | - | 2-CF₃, 5-Cl |
| A-833 | 3-F | 2-CF₃, 5-Cl |
| A-834 | 3-Cl | 2-CF₃, 5-Cl. |
| A-835 | 3-CH₃ | 2-CF₃, 5-Cl |
| A-836 | 3-CH₃ | 2-CF₃, 5-Cl |
| A-837 | 3-OCF₃ | 2-CF₃, 5-Cl |
| A-838 | - | 2-Cl, 5-CF₃ |
| A-839 | 3-F | 2-Cl, 5-CF₃ |
| A-840 | 3-Cl | 2-Cl, 5-CF₃ |
| A-841 | 3-CH₃ | 2-Cl, 5-CF₃ |
| A-842 | 3-CF₃ | 2-Cl, 5-CF₃ |
| A-843 | 3-OCF₃ | 2-Cl, 5-CF₃ |
| A-844 | - | 2-F, 5-CF₃ |
| A-845 | 3-F | 2-F, 5-CF₃ |
| A-846 | 3-Cl | 2-F, 5-CF₃ |
| A-847 | 3-CH₃ | 2-F, 5-CF₃ |
| A-848 | 3-CF₃ | 2-F, 5-CF₃ |
| A-849 | 3-OCF₃ | 2-F, 5-CF₃ |
| A-850 | - | 3-F, 4-CF₃ |
| A-851 | 3-F | 3-F, 4-CF₃ |
| A-852 | 3-Cl | 3-F, 4-CF₃ |
| A-853 | 3-CH₃ | 3-F, 4-CF₃ |
| A-854 | 3-CF₃ | 3-F, 4-CF₃ |
| A-855 | 3-OCF₃ | 3-F, 4-CF₃ |
| A-856 | - | 3-Cl, 4-CF₃ |
| A-857 | 3-F | 3-Cl, 4-CF₃ |
| A-858 | 3-Cl | 3-Cl, 4-CF₃ |
| A-859 | 3-CH₃ | 3-Cl, 4-CF₃ |
| A-860 | 3-CF₃ | 3-Cl, 4-CF₃ |
| A-861 | 3-OCF₃ | 3-Cl, 4-CF₃ |
| A-862 | - | 3-Cl, 4-Cl |
| A-863 | 3-F | 3-Cl, 4-Cl |
| A-864 | 3-Cl | 3-Cl, 4-Cl |
| A-865 | 3-CH₃ | 3-Cl, 4-Cl |
| A-866 | 3-CF₃ | 3-Cl, 4-Cl |
| A-867 | 3-OCF₃ | 3-Cl, 4-Cl |
| A-868 | - | 3-F, 4-Cl |
| A-869 | 3-F | 3-F, 4-Cl |
| A-870 | 3-Cl | 3-F, 4-Cl |
| A-871 | 3-CH₃ | 3-F, 4-Cl |
| A-872 | 3-CF₃ | 3-F, 4-Cl |
| A-873 | 3-OCF₃ | 3-F, 4-Cl |
| A-874 | - | 3-CF₃, 4-Cl |
| A-875 | 3-F | 3-CF₃, 4-Cl |
| A-876 | 3-Cl | 3-CF₃, 4-Cl |
| A-877 | 3-CH₃ | 3-CF₃, 4-Cl |
| A-878 | 3-CF₃ | 3-CF₃, 4-Cl |
| A-879 | 3-OCF₃ | 3-CF₃, 4-Cl |
| A-880 | - | - |

The compounds according to the invention can be obtained by different routes. Advantageously, they are obtained from pyridine derivatives of the formula II under basic conditions by reaction with sulfonic acids or activated sulfonic acid derivatives of the formula III.

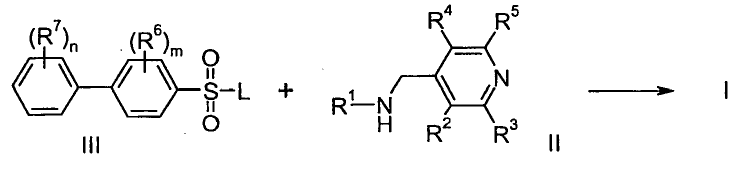

In formula III, L is a suitable leaving group such as hydroxyl or halogen, preferably chlorine.

This reaction is usually carried out at temperatures of from (-30)°C to 120°C, preferably from (-10)°C to 100°C, in an inert organic solvent in the presence of a base [cf. Lieb. Ann. Chem. 641 (1990)].

Suitable solvents are aliphatic hydrocarbons, such as pentane, hexane, cyclohexane and petroleum ether, aromatic hydrocarbons, such as toluene, o-, m- and p-xylene, halogenated hydrocarbons, such as methylene chloride, chloroform and chlorobenzene, ethers, such as diethyl ether, diisopropyl ether, tert.-butyl methyl ether, dioxane, anisole and tetrahydrofuran, nitriles, such as acetonitrile and propionitrile, ketones, such as acetone, methyl ethyl ketone, diethyl ketone and tert.-butyl methyl ketone, and also dimethyl sulfoxide, dimethylformamide and dimethylacetamide, particularly preferably diisopropyl ether, diethyl ether and tetrahydrofuran. It is also possible to use mixtures of the solvents mentioned.

Suitable bases are, in general, inorganic compounds, such as alkali metal and alkaline earth metal hydroxides, such as lithium hydroxide, sodium hydroxide, potassium hydroxide and calcium hydroxide, alkali metal and alkaline earth metal oxides, such as lithium oxide, sodium oxide, calcium oxide and magnesium oxide, alkali metal and alkaline earth metal hydrides, such as lithium hydride, sodium hydride, potassium hydride and calcium hydride, alkali metal and alkaline earth metal carbonates, such as lithium carbonate, potassium carbonate and calcium carbonate, and also alkali metal bicarbonates, such as sodium bicarbonate, moreover organic bases, for example tertiary amines, such as trimethylamine, triethylamine, triisopropylethylamine and N-methylpiperidine, pyridine, substituted pyridines, such as collidine, lutidine and 4-dimethylaminopyridine, and also bicyclic amines. Particular preference is given to pyridine, triethylamine and potassium carbonate. The bases are generally employed in catalytic amounts; however, they can also be employed in equimolar amounts, in excess or, if appropriate, as solvent.

The starting materials are generally reacted with one another in equimolar amounts. In terms of yield, it may be advantageous to use an excess of II, based on III.

The starting materials required for preparing the compounds I are commercially available or known from the literature [WO 99/42443; EP-A 483 667; WO 96/10022; WO 00/73295; J. für praktische Chemie, p. 695 (1994); Heterocycles, p. 675 (1995); Tetrahedron, p. 12, 483 (1996); Chem. Pharm. Bull., p. 1927 (1973); J. Chem. Soc., p. 426 (1942); EP-A 983 982; Synthesis, p. 852 (1986)], or they can be prepared in accordance with the literature cited.

The pyridine derivatives II can be prepared, for example, by reduction of the respective nitriles, oximes or carbonic acid amides. The synthesis and the precursors can be taken from, e.g. J. Org. Chem. 23, 714 (1958), J. Prakt. Chem. 336(8), 695 (1994), Chem. Pharm. Bull. 1927, 21 (1973), US 4,439,609, Houben-Weyl, Methoden der Organischen Chemie, Georg Thieme Verlag Stuttgart, Vol. 10/4 (1968) and Vol. 11/2 (1957) and Vol. E5 (1985), Heterocyclic Compounds, Vol. 14, part 1-4, Wiley New York 1974-1975, Methods in Science of Synthesis, Vol. 15, Tetrahedron 57, 4489 (2001), Doklady Akadem Nauk SSSR 164, 816 (1965), Eur. J. Org. Chem. 1371 (2001), Tetrahedron 57, 4059 (2001), US 2005/0239791, Heterocycles 65, 8 (2005), European Journal of Organic Chemistry 8, 1559-1568 (2003).

Alternatively, the compounds of the formula I can also be prepared by reacting a pyridine derivative of the formula II with a 4-halophenylsulfonyl halide of the formula IV, followed by construction of the biphenyl unit. The latter can be carried out under Stille or preferably Suzuki coupling conditions.

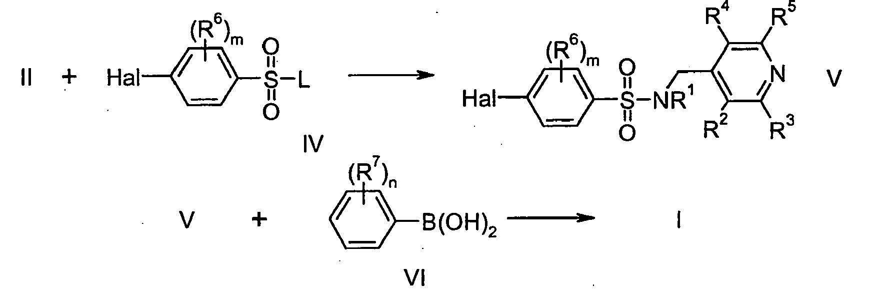

In formula IV, Hal and L are halogen, L is preferably chlorine and "Hal" is in particular bromine or iodine.

The conversion into the pyridylsulfonamides of the formula V is carried out under the same conditions as the condensation of the compounds II and III.

The Suzuki coupling of a pyridylsulfonamide V with a boronic acid derivatives VI or an ester thereof is usually carried out at temperatures of from 20°C to 180°C, preferably from 40°C to 120°C, in an inert organic solvent in the presence of a base and a platinum metal, in particular a palladium catalyst [cf. Synth. Commun. Vol. 11, p. 513 (1981); Acc. Chem. Res. Vol. 15, pp. 178-184 (1982); Chem. Rev. Vol. 95, pp. 2457-2483 (1995); Organic Letters Vol. 6 (16), p. 2808 (2004); WO 2002/42275].

Suitable catalysts are in particular tetrakis(triphenylphosphine)palladium(0); bis(triphenylphosphine)palladium (II) chloride; bis(acetonitrile)palladium (II) chloride; [1,1'-bis(diphenylphosphino)ferrocene]-palladium(II) chloride/methylene chloride (1:1) complex; bis[bis-(1,2-diphenylphosphino)ethane]palladium(0); bis(bis-(1,2-diphenylphosphino)butane]-palladium(II) chloride; palladium(II) acetate; palladium(II) chloride; and palladium(II) acetate/tri-o-tolylphosphine complex; tris-tert.-butylphosphine / palladium-dibenzylidene acetone.

Suitable solvents are aliphatic hydrocarbons, such as pentane, hexane, cyclohexane and petroleum ether, aromatic hydrocarbons, such as toluene, o-, m- and p-xylene, ethers, such as diisopropyl ether, tert.-butyl methyl ether, dioxane, anisole and tetrahydrofuran and dimethoxyethane, ketones, such as acetone, methyl ethyl ketone, diethyl ketone and tert.-butyl methyl ketone, and also dimethyl sulfoxide, dimethylformamide and dimethylacetamide, particularly preferably ethers, such as tetrahydrofuran, dioxane and dimethoxyethane. It is also possible to use mixtures of the solvents mentioned.

Suitable bases are, in general, inorganic compounds, such as alkali metal and alkaline earth metal oxides, such as lithium oxide, sodium oxide, calcium oxide and magnesium oxide, alkali metal and alkaline earth metal carbonates, such as lithium carbonate, sodium carbonate, potassium carbonate and calcium carbonate, and also alkali metal bicarbonates, such as sodium bicarbonate, alkali metal and alkaline earth metal alkoxides, such as sodium methoxide, sodium ethoxide, potassium ethoxide and potassium tert.-butoxide, moreover organic bases, for example tertiary amines, such as trimethylamine, triethylamine, triisopropylethylamine and N-methylpiperidine, pyridine, substituted pyridines, such as collidine, lutidine and 4-dimethylaminopyridine, and also bicyclic amines. Particular preference is given to bases such as sodium carbonate, potassium carbonate, cesium carbonate, triethylamine and sodium bicarbonate.

The bases are generally employed in equimolar amounts; however, they can also be employed in excess or, if appropriate, as solvent.

The starting materials are generally reacted with one another in equimolar amounts. In terms of yield, it may be advantageous to use an excess of VI, based on V.

The intermediate IV can be prepared from the respective phenylhalide VII by treatment with alkylmagnesiumhalogenide such as (CH₃)₂CHMgCl, SO₂ and SO₂Cl₂ as shown in the reaction scheme below:

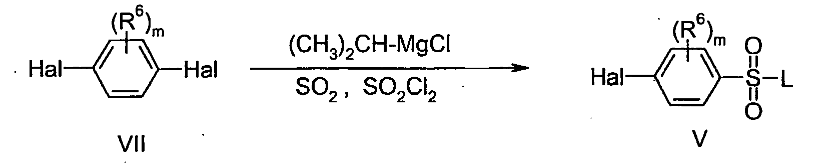

The boronic acid derivatives VI or their esters can be obtained as described in Synlett 8, 1204 (2003), J. Org. Chem. 68, 3729 (2003), Synthesis, 442 (2000), J. Org. Chem. 60, 7508 (1995) or WO 2002/42275.

The reaction mixtures are worked up in the customary manner, for example by mixing with water, separating the phases and, if appropriate, chromatographic purification of the crude products. Some of the intermediates and end products are obtained in the form of colorless or slightly brownish viscous oils which can be purified or freed from volatile components under reduced pressure and at moderately elevated temperature. If the intermediates and end products are obtained as solids, purification can also be carried out by recrystallization or digestion.

The N-oxides may be prepared from the compounds I according to conventional oxidation methods, for example by treating a biphenylsulfonamide I with an organic peracid such as metachloroperbenzoic acid [Journal of Medicinal Chemistry, 38(11), 1892-1903 (1995); WO 03/64572] or with inorganic oxidizing agents such as hydrogen peroxide [cf. Jounal of Heterocyclic Chemistry, 18(7), 1305-8 (1981)] or oxone [cf. Journal of the American Chemical Society, 123(25), 5962-5973 (2001)]. The oxidation may lead to pure mono-N-oxides or to a mixture of different N-oxides in case a second pyridine ring is present in the molecule. Different N-oxides can be separated by conventional methods such as chromatography.

If individual compounds I cannot be obtained by the routes described above, they can be prepared by derivatization of other compounds I.

If the synthesis yields mixtures of isomers, a separation is generally not necessarily required since in some cases the individual isomers can be interconverted during work-up for use or during application (for example under the action of light, acids or bases). Such conversions may also take place after use, for example in the treatment of plants in the treated plant, or in the harmful fungus to be controlled.

The compounds I are suitable as fungicides. They are distinguished by an outstanding effectiveness against a broad spectrum of phytopathogenic fungi, especially from the classes of the *Ascomycetes, Basidiomycetes, Deuteromycetes* and *Peronosporomycetes* (syn. *Oomycetes*)*.* Some are systemically effective and they can be used in crop protection as foliar fungicides, fungicides for seed dressing and soil fungicides.

They are particularly important in the control of a multitude of fungi on various cultivated plants, such as wheat, rye, barley, oats, rice, corn, lawns, bananas, cotton, soybeans, coffee, sugar cane, grapevines, fruit and ornamental plants, and vegetables, such as cucumbers, beans, tomatoes, potatoes and cucurbits, and on the seeds of these plants.

They are especially suitable for controlling the following plant diseases:
- *Alternaria* species on vegetables, rape, sugar beets, fruit and rice (e.g. *A. solani* or *A. alternata* on potatoes and tomatoes),
- *Aphanomyces* species on sugar beets and vegetables,
- *Ascochyta* species on cereals and vegetables,
- *Bipolaris* and *Drechslera* species on corn, cereals, rice and lawns (e.g.. *D. maydis* on corn),
- *Blumeria graminis* (powdery mildew) on cereals,
- *Botrytis cinerea* (gray mold) on strawberries, vegetables, ornamental plants and grapevines,
- *Bremia lactucae on lettuce,*
- *Cerospora* species on corn, soybeans, rice and sugar beets,
- *Cochliobolus* species on corn, cereals, rice (e.g. *Cochliobolus sativus* on cereals, *Cochliobolus miyabeanus* on rice),
- *Colletotricum* species on soybeans and cotton,
- *Drechslera species, Pyrenophora* species on corn, cereals, rice and lawns (e.g. *D. teres* on barley or *D. tritici-repentis* on wheat),
- *Esca* on grapevines, caused by *Phaeoacremonium chlamydosporium, Ph. Aleophilum* and *Formitipora punctata* (syn. *Phellinus punctatus*),
- *Exserohilum* species on corn,
- *Erysiphe cichoracearum* and *Sphaerotheca fuliginea* on cucurbits,
- *Fusarium* and *Verticil*/*ium* species on various plants (e.g. *F. graminearum* or *F*. *culmorum* on cereals or *F. oxysporum* on various plants, e.g. tomatoes),
- *Gaeumanomyces graminis* on cereals,
- *Gibberella* species on cereals and rice (e.g. *Gibberella fujikuroi* on rice),
- *Grainstaining complex* on rice,
- *Helminthosporium* species on corn and rice,
- *Michrodochium nivale* on cereals,
- *Mycosphaerella* species on cereals, bananas and peanuts (e.g. *M. graminicola* on wheat or *M*. *fijiesis* on bananas),
- *Peronospora* species on cabbage and onion plants (e.g. *P. brassicae* on cabbage or *P. destructor* on onions),
- *Phakopsara pachyrhizi* and *Phakopsara meibomiae* on soybeans,
- *Phomopsis* species on soybeans and sun flowers,
- *Phytophthora infestans* on potatoes and tomatoes,
- *Phytophthora* species on various plants (e.g. *P. capsici* on paprika),
- *Plasmopara viticola* on grapevines,
- *Podosphaera leucotricha* on apples,
- *Pseudocercosporella herpotrichoides* on cereals,
- *Pseudoperonospora* on various plants (e.g. *P. cubensis* on cocumber or *P. humili* on hops),
- *Puccinia* species on various plants (e.g. *P. triticina, P. striformins , P. hordei* or *P. graminis* on cereals or *P. asparagi* on asparagus),
- *Pyricularia oryzae, Corticium sasakii , Sarocladium oryzae, S. attenuatum, Entyloma oryzae* on rice,
- *Pyricularia grisea* on lawns and cereals,
- *Pythium spp.* on lawns, rice, corn, cotton, rape, sun flowers, sugar beets, vegetables and other plants (e.g. *P. ultiumum* on various plants, *P*. *aphanidermatum* on lawns),
- *Rhizoctonia* species on cotton, rice, potatoes, lawns, corn, rape, sugar beets, vegetables and on various plants (e.g. *R. solani* on beets and various plants),
- *Rhynchosporium secalis* on barley, rye and triticale,
- *Sclerotinia* species on rape and sun flowers,
- *Septoria tritici* and *Stagonospora nodorum* on wheat,
- *Erysiphe* (syn. *Uncinula) necator* on grapevines,
- *Setospaeria* species on corn and lawns,
- *Sphacelotheca reilinia* on corn,
- *Thievaliopsis* species on soybeans and cotton,
- *Tilletia* species on cereals,
- *Ustilago* species on cereals, corn and sugar cane (e.g. *U. maydis* on corn),
- *Venturia* species (scab) on apples and pears (e.g. *V. inaequalis* on apples).

The compounds I are also suitable for controlling harmful fungi in the protection of materials (e.g. wood, paper, paint dispersions, fiber or fabrics) and in the protection of stored products. As to the protection of wood, the following harmful fungi are worthy of note: Ascomycetes such as *Ophiostoma* spp., *Ceratocystis* spp., *Aureobasidium pullulans, Sclerophoma* spp., *Chaetomium* spp., *Humicola* spp., *Petriella* spp., *Trichurus* spp.; Basidiomycetes such as *Coniophora* spp., *Coriolus* spp., *Gloeophyllum* spp., *Lentinus* spp., *Pleurotus* spp., *Poria* spp., *Serpula* spp. and *Tyromyces* spp., Deuteromycetes such as *Aspergillus* spp., *Cladosporium* spp., *Penicillium* spp., *Trichoderma* spp., *Alternaria* spp., *Paecilomyces* spp. and Zygomycetes such as *Mucor* spp., and in addition in the protection of stored products the following yeast fungi are worthy of note: *Candida* spp. and *Saccharomyces cerevisae.*

The compounds I are employed by treating the fungi or the plants, seeds, materials or the soil to be protected from fungal attack with a fungicidally effective amount of the active compounds. The application can be carried out both before and after the infection of the materials, plants or seeds by the fungi.

The fungicidal compositions generally comprise between 0.1 and 95%, preferably between 0.5 and 90%, by weight of active compound. The active compounds are employed in a purity of from 90% to 100%, preferably from 95% to 100% (according to NMR spectrum).

When employed in plant protection, the amounts applied are, depending on the kind of effect desired, between 0.01 and 2.0 kg of active compound per ha.
In seed treatment, for example by dusting, coating or drenching seed, amounts of active compound of from 1 to 1000 g, preferably from 5 to 100 g, per 100 kilogram of seed are generally required.

When used in the protection of materials or stored products, the amount of active compound applied depends on the kind of application area and on the desired effect. Amounts customarily applied in the protection of materials are, for example, 0.001 g to 2 kg, preferably 0.005 g to 1 kg, of active compound per cubic meter of treated material.

The compounds of the formula I are furthermore suitable for controlling pests from the classes of the insects, arachnids and nematodes effectively. They can be used as pesticides in crop protection and in the sectors of hygiene and the protection of stored products and the veterinary sector.

They may act by contact or may be stomach-acting, or have systemic or residual action. Contact action means that the pest is killed by coming into contact with a compound I or with material that releases compound I. Stomach-acting means that the pest is killed if it ingests a pesticidially effective amount of the compound I or material containing a pesticidally effective amount of compound I. Systemic action means that the compound is absorbed into the plant tissues of treated plant and the pest is controlled, if it eats plant tissue or sucks plant-sap. Compounds I are in particular suitable for controlling the following insect pests:, such as

### Insects:

insects from the order of *Lepidoptera,* for example *Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, L eucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera eridania, Spodoptera frugiperda, Spodoptera littoralis, Spodoptera litura, Thaumatopoea pityocampa, Tortrix viridana, Trichoplusia ni* and *Zeiraphera canadensis,*
from the order of Coleoptera (beetles), for example *Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, lps typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus o vatus, Phaedon cochleariae, Phyllotreta chrysocephala, Phyllophaga sp., Phyllopertha horticola, Phyllotreta nemorum, Phyllotreta striolata, Popillia japonica, Sitona lineatus* and *Sitophilus granaria,*
from the order of Diptera, for example *Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, L ucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea* and *Tipula paludosa,*
from the order of Thysanoptera (thrips), e.g. *Dichromothrips spp., Frankliniella fusca, Frankliniella occidentalis, Frankliniella tritici, Scirtothrips citri, Thrips oryzae, Thrips palmi* and *Thrips tabaci,*
ants, bees, wasps, sawflies from the order of (Hymenoptera) e.g. *Athalia rosae, Atta cephalotes, Atta cephalotes, Atta laevigata, Atta robusta, Atta capiguara, Atta sexdens, Atta texana, Crematogaster spp., Hoplocampa minuta, Hoplocampa testudinea, Monomorium pharaonis, Solenopsis geminata,* and *Solenopsis invicta, Solenopsis richteri, Solenopsis xyloni, Pogonomyrmex barbatus, Pogonomyrmex californicus, Pheidole megacephala, Dasymutilla occidentalis, Bombus* spp. *Vespula squamosa, Para vespula vulgaris, Paravespula pennsylvanica, Para vespula germanica, Dolichovespula maculata, Vespa crabro, Polistes rubiginosa, Camponotus floridanus,* and *Linepithema humile,*
from the order of Homoptera, e.g. *Acyrthosiphon onobrychis, Adelges laricis, Aphidula nasturtii, Aphis craccivora, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisa tabaci, Bemisa argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzodes persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion avenae, Trialeurodes vaporariorum, Toxoptera aurantiiand,* and *Viteus vitifolii,*
from the order of Isoptera (termites), e.g. *Calotermes flavicollis, Heterotermes aureus, L eucotermes flavipes, Reticulitermes flavipes, Reticulitermes virginicus, Reticulitermes lucifugus,* aund *Termes natalensis,* and *Coptotermes formosanus,*
cockroaches (Blattaria - Blattodea), e.g. *Blattella germanica, Blattella asahinae, Periplaneta americana, Periplaneta japonica, Periplaneta brunnea, Periplaneta fuligginosa, Periplaneta australasiae,* and *Blatta orientalis,*
true bugs (*Hemiptera*)*,* e.g. *Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor Acyrthosiphon onobrychis, Adelges laricis, Aphidula nasturtii, Aphis fabae, Aphis forbesi, Aphis pomi, Aphis gossypii, Aphis grossulariae, Aphis schneideri, Aphis spiraecola, Aphis sambuci, Acyrthosiphon pisum, Aulacorthum solani, Bemisia argentifolii, Brachycaudus cardui, Brachycaudus helichrysi, Brachycaudus persicae, Brachycaudus prunicola, Brevicoryne brassicae, Capitophorus horni, Cerosipha gossypii, Chaetosiphon fragaefolii, Cryptomyzus ribis, Dreyfusia nordmannianae, Dreyfusia piceae, Dysaphis radicola, Dysaulacorthum pseudosolani, Dysaphis plantaginea, Dysaphis pyri, Empoasca fabae, Hyalopterus pruni, Hyperomyzus lactucae, Macrosiphum avenae, Macrosiphum euphorbiae, Macrosiphon rosae, Megoura viciae, Melanaphis pyrarius, Metopolophium dirhodum, Myzus persicae, Myzus ascalonicus, Myzus cerasi, Myzus varians, Nasonovia ribis-nigri, Nilaparvata lugens, Pemphigus bursarius, Perkinsiella saccharicida, Phorodon humuli, Psylla mali, Psylla piri, Rhopalomyzus ascalonicus, Rhopalosiphum maidis, Rhopalosiphum padi, Rhopalosiphum insertum, Sappaphis mala, Sappaphis mali, Schizaphis graminum, Schizoneura lanuginosa, Sitobion a venae, Trialeurodes vaporariorum, Toxoptera aurantiiand, Viteus vitifolii, Cimex lectularius, Cimex hemipterus, Reduvius senilis, Triatoma* spp., and *Arilus critatus,*
crickets, grasshoppers, locusts from the order of (Orthoptera), e.g. *Acheta domestica, Blatta orientalis, Blattella germanica, Calliptamus italicus, Chortoicetes terminifera, Dociostaurus maroccanus, Forficula auricularia, Gryllotalpa gryllotalpa, Hieroglyphus daganensis, Kraussaria angulifera, Locusta migratoria, Locustana pardalina, Melanoplus bivittatus, Melanoplus femur-rubrum, Melanoplus mexicanus, Melanoplus sanguinipes, Melanoplus spretus, Nomadacris septemfasciata, Oedaleus senegalensis, Periplaneta americana, Schistocerca americana, Schistocerca peregrina, Schistocerca gregaria, Stauronotus maroccanus* and, *Tachycines asynamorus, Tachycines asynamorus, Zonozerus variegatus.*

The compounds of the formula I and their salts are also useful for controlling arachnids (Arachnoidea), such as acarians (*Acarina*)*,* e.g. of the families *Argasidae, Ixodidae* and *Sarcoptidae,* such as *Amblyomma americanum, Amblyomma variegatum, Ambryomma maculatum, Argas persicus, Boophilus annulatus, Boophilus decoloratus, Boophilus microplus, Dermacentor silvarum, Dermacentor andersoni, Dermacentor variabilis, Hyalomma truncatum, Ixodes ricinus, Ixodes rubicundus, Ixodes scapularis, Ixodes holocyclus, Ixodes pacificus, Ornithodorus hermsi, Ornithodorus turicata, Ornithonyssus bacoti, Ornithodorus moubata, Otobius megnini, Dermanyssus gallinae, Psoroptes ovis, Rhipicephalus sanguineus, Rhipicephalus appendiculatus, Rhipicephalus evertsi, Sarcoptes scabiei,* and Eriophyidae spp. such as *Aculus schlechtendali, Phyllocoptrata oleivora* and *Eriophyes sheldoni,* Tarsonemidae spp. such as *Phytonemus pallidus* and *Polyphagotarsonemus latus,* Tenuipalpidae spp. such as *Brevipalpus phoenicis; Tetra*nychidae spp. such as *Tetranychus cinnabarinus, Tetranychus kanzawai, Tetranychus pacificus, Tetranychus telarius* and *Tetranychus urticae, Panonychus ulmi, Panonychus citri, and oligonychus pratensis* and *Oligonychus pratensis,* Araneida, e.g. *Latrodectus mactans,* and *Loxosceles reclusa,*
fleas (Siphonaptera), e.g. *Ctenocephalides felis, Ctenocephalides canis, Xenopsylla cheopis, Pulex irritans, Tunga penetrans,* and *Nosopsyllus fasciatus,*
silverfish, firebrat (Thysanura), e.g. *Lepisma saccharina* and *Thermobia domestica,*
centipedes (Chilopoda), e.g. *Scutigera coleoptrata,*
millipedes (Diplopoda), e.g. *Narceus spp.,*
earwigs (Dermaptera*),* e.g. *forficula auricularia,*
lice (Phthiraptera), e.g. *Pediculus humanus capitis, Pediculus humanus corporis, Pthirus pubis, Haematopinus eurysternus, Haematopinus suis, Linognathus vituli, Bovicola bovis, Menopon gallinae, Menacanthus stramineus* and *Solenopotes capillatus.*

In general, the insecticidal compositions comprise from 0.01 to 95% by weight, preferably from 0.1 to 90% by weight, of the active compound. The active compounds are employed in a purity of from 90% to 100%, preferably from 95% to 100% (according to NMR spectrum).

Under outdoor conditions, the active compound application rate for controlling pests is from 0.1 to 2.0, preferably from 0.2 to 1.0, kg/ha.

The compounds I can be converted into the customary formulations, for example solutions, emulsions, suspensions, dusts, powders, pastes and granules. The application form depends on the particular intended purpose; in each case, it should ensure a fine and uniform distribution of the compound according to the invention.

The formulations are prepared in a known manner, for example by extending the active compound with solvents and/or carriers, if desired using emulsifiers and dispersants. Solvents/auxiliaries which are suitable are essentially:
- water, aromatic solvents (for example Solvesso^{®} products, xylene), paraffins (for example mineral oil fractions), alcohols (for example methanol, butanol, pentanol, benzyl alcohol), ketones (for example cyclohexanone, gamma-butyrolactone), pyrrolidones (N-methylpyrrolidone, N-octylpyrrolidone), acetates (glycol diacetate), glycols, fatty acid dimethylamides, fatty acids and fatty acid esters. In principle, solvent mixtures may also be used.
- carriers such as ground natural minerals (for example kaolins, clays, talc, chalk) and ground synthetic minerals (for example highly disperse silica, silicates); emulsifiers such as nonionic and anionic emulsifiers (for example polyoxyethylene fatty alcohol ethers, alkylsulfonates and arylsulfonates) and dispersants such as lignosulfite waste liquors and methylcellulose.

Suitable surfactants are alkali metal, alkaline earth metal and ammonium salts of lignosulfonic acid, naphthalenesulfonic acid, phenolsulfonic acid, dibutylnaphthalenesulfonic acid, alkylarylsulfonates, alkyl sulfates, alkylsulfonates, fatty alcohol sulfates, fatty acids and sulfated fatty alcohol glycol ethers, furthermore condensates of sulfonated naphthalene and naphthalene derivatives with formaldehyde, condensates of naphthalene or of naphthalenesulfonic acid with phenol and formaldehyde, polyoxyethylene octylphenol ether, ethoxylated isooctylphenol, octylphenol, nonylphenol, alkylphenol polyglycol ethers, tributylphenyl polyglycol ether, tristearylphenyl polyglycol ether, alkylaryl polyether alcohols, alcohol and fatty alcohol/ethylene oxide condensates, ethoxylated castor oil, polyoxyethylene alkyl ethers, ethoxylated polyoxypropylene, lauryl alcohol polyglycol ether acetal, sorbitol esters, lignosulfite waste liquors and methylcellulose.

Suitable for the preparation of directly sprayable solutions, emulsions, pastes or oil dispersions are mineral oil fractions of medium to high boiling point, such as kerosene or diesel oil, furthermore coal tar oils and oils of vegetable or animal origin, aliphatic, cyclic and aromatic hydrocarbons, for example toluene, xylene, paraffin, tetrahydronaphthalene, alkylated naphthalenes or their derivatives, methanol, ethanol, propanol, butanol, cyclohexanol, cyclohexanone, isophorone, strongly polar solvents, for example dimethyl sulfoxide, N-methylpyrrolidone and water.

Also anti-freezing agents such as glycerin, ethylene glycol and propylene glycol can be added to the formulation.

Suitable antifoaming agents are, for example, those based on silicone or magnesium stearate.

Powders, materials for spreading and dustable products can be prepared by mixing or concomitantly grinding the active substances with a solid carrier.

Granules, for example coated granules, impregnated granules and homogeneous granules, can be prepared by binding the active compounds to solid carriers. Examples of solid carriers are mineral earths such as silica gels, silicates, talc, kaolin, attaclay, limestone, lime, chalk, bole, loess, clay, dolomite, diatomaceous earth, calcium sulfate, magnesium sulfate, magnesium oxide, ground synthetic materials, fertilizers, such as, for example, ammonium sulfate, ammonium phosphate, ammonium nitrate, ureas, and products of vegetable origin, such as cereal meal, tree bark meal, wood meal and nut-shell meal, cellulose powders and other solid carriers.

Formulations for the treatment of seed may additionally comprise binders and/or gelling agents and, if appropriate, colorants.

Binders may be added to increase the adhesion of the active compounds on the seed after the treatment. Suitable binders are, for example, EO/PO block copolymer surfactants, but also polyvinyl alcohols, polyvinylpyrrolidones, polyacrylates, polymethacrylates, polybutenes, polyisobutylenes, polystyrenes, polyethylenamines, polyethylenamides, polyethylenimines (Lupasol^{®}, Polymin^{®}), polyethers, polyurethanes, polyvinyl acetates, tylose and copolymers of these polymers.

A suitable gelling agent is, for example, carrageen (Satiagel^{®}).

In general, the formulations comprise from 0.01 to 95% by weight, preferably from 0.1 to 90% by weight, of the active compound. The active compounds are employed in a purity of from 90% to 100%, preferably from 95% to 100% (according to NMR spectrum).

The concentrations of active compound in the ready-for-use preparations can be varied within relatively wide ranges. In general, they are between 0.0001 and 10%, preferably between 0.01 and 1%.

The active compounds can also be used with great success in the ultra-low volume (ULV) process, it being possible to apply formulations with more than 95% by weight of active compound or even the active compound without additives.

For the treatment of seed, the formulations in question give, after two-to-tenfold dilution, active compound concentrations of from 0.01 to 60% by weight, preferably from 0.1 to 40% by weight, in the ready-to-use preparations.

The following are examples of formulations: 1. Products for dilution with water
A) Water-soluble concentrates (SL, LS)
   10 parts by weight of a compound I according to the invention are dissolved in 90 parts by weight of water or in a water-soluble solvent. As an alternative, wetting agents or other auxiliaries are added. The active compound dissolves upon dilution with water. In this way, a formulation having a content of 10% by weight of active compound is obtained.
B) Dispersible concentrates (DC)
   20 parts by weight of a compound I according to the invention are dissolved in 70 parts by weight of cyclohexanone with addition of 10 parts by weight of a dispersant, for example polyvinylpyrrolidone. Dilution with water gives a dispersion. The active compound content is 20% by weight.
C) Emulsifiable concentrates (EC)
   15 parts by weight of a compound I according to the invention are dissolved in 75 parts by weight of xylene with addition of calcium dodecylbenzenesulfonate and castor oil ethoxylate (in each case 5 parts by weight). Dilution with water gives an emulsion. The formulation has an active compound content of 15% by weight.
D) Emulsions (EW, EO, ES)
   25 parts by weight of a compound I according to the invention are dissolved in 35 parts by weight of xylene with addition of calcium dodecylbenzenesulfonate and castor oil ethoxylate (in each case 5 parts by weight). This mixture is introduced into 30 parts by weight of water by means of an emulsifying machine (Ultraturrax) and made into a homogeneous emulsion. Dilution with water gives an emulsion. The formulation has an active compound content of 25% by weight.
E) Suspensions (SC, OD)
   In an agitated ball mill, 20 parts by weight of a compound I according to the invention are comminuted with addition of 10 parts by weight of dispersants and wetting agents and 70 parts by weight of water or an organic solvent to give a fine active compound suspension. Dilution with water gives a stable suspension of the active compound. The active compound content in the formulation is 20% by weight.
F) Water-dispersible granules and water-soluble granules (WG, SG, SS, WS)
   50 parts by weight of a compound I according to the invention are ground finely with addition of 50 parts by weight of dispersants and wetting agents and prepared as water-dispersible or water-soluble granules by means of technical appliances (for example extrusion, spray tower, fluidized bed). Dilution with water gives a stable dispersion or solution of the active compound. The formulation has an active compound content of 50% by weight.
G) Water-dispersible powders and water-soluble powders (WP, SP)
   75 parts by weight of a compound I according to the invention are ground in a rotor-stator mill with addition of 25 parts by weight of dispersants, wetting agents and silica gel. Dilution with water gives a stable dispersion or solution of the active compound. The active compound content of the formulation is 75% by weight.

### 2. Products to be applied undiluted

H) Dustable powders (DP, DS)
   5 parts by weight of a compound I according to the invention are ground finely and mixed intimately with 95 parts by weight of finely divided kaolin. This gives a dustable product having an active compound content of 5% by weight.
J) Granules (GR, FG, GG, MG)
   0.5 part by weight of a compound I according to the invention is ground finely and associated with 99.5 parts by weight of carriers. Current methods are extrusion, spray-drying or the fluidized bed. This gives granules to be applied undiluted having an active compound content of 0.5% by weight.
K) ULV solutions (UL, LS)
   10 parts by weight of a compound I according to the invention are dissolved in 90 parts by weight of an organic solvent, for example xylene. This gives a product to be applied undiluted having an active compound content of 10% by weight.

The active compounds can be used as such, in the form of their formulations or the use forms prepared therefrom, for example in the form of directly sprayable solutions, powders, suspensions or dispersions, emulsions, oil dispersions, pastes, dustable products, materials for spreading, or granules, by means of spraying, atomizing, dusting, spreading or pouring. The use forms depend entirely on the intended purposes; the intention is to ensure in each case the finest possible distribution of the active compounds according to the invention.

Aqueous use forms can be prepared from emulsion concentrates, pastes or wettable powders (sprayable powders, oil dispersions) by adding water. To prepare emulsions, pastes or oil dispersions, the substances, as such or dissolved in an oil or solvent, can be homogenized in water by means of a wetter, tackifier, dispersant or emulsifier. Alternatively, it is possible to prepare concentrates composed of active substance, wetter, tackifier, dispersant or emulsifier and, if appropriate, solvent or oil, and such concentrates are suitable for dilution with water.

The active compound concentrations in the ready-to-use preparations can be varied within relatively wide ranges. In general, they are from 0.0001 to 10%, preferably from 0.01 to 1%.

The active compounds may also be used successfully in the ultra-low-volume process (ULV), by which it is possible to apply formulations comprising over 95% by weight of active compound, or even to apply the active compound without additives.

Various types of oils, wetters, adjuvants, herbicides, fungicides, other pesticides, or bactericides may be added to the active compounds, if appropriate not until immediately prior to use (tank mix). These agents can be admixed with the agents according to the invention in a weight ratio of 1:10 to 10:1.

The compositions according to the invention can, in the use form as fungicides, also be present together with other active compounds, e.g. with herbicides, insecticides, growth regulators, fungicides or else with fertilizers. Mixing the compounds I or the compositions comprising them in the use form as fungicides with other fungicides results in many cases in an expansion of the fungicidal spectrum of activity being obtained.

The following list of fungicides, in conjunction with which the compounds according to the invention can be used, is intended to illustrate the possible combinations but does not limit them:
- acylalanines, such as benalaxyl, metalaxyl, ofurace or oxadixyl,
- amine derivatives, such as aldimorph, dodine, dodemorph, fenpropimorph, fenpropidin, guazatine, iminoctadine, spiroxamine or tridemorph,
- anilinopyrimidines, such as pyrimethanil, mepanipyrim or cyprodinil,
- antibiotics, such as cycloheximide, griseofulvin, kasugamycin, natamycin, polyoxin or streptomycin,
- azoles, such as bitertanol, bromoconazole, cyproconazole, difenoconazole, dinitroconazole, enilconazole, epoxiconazole, fenbuconazole, fluquinconazole, flusilazole, flutriafol, hexaconazole, imazalil, ipconazole, metconazole, myclobutanil, penconazole, propiconazole, prochloraz, prothioconazole, simeconazole, tebuconazole, tetraconazole, triadimefon, triadimenol, triflumizole or triticonazole,
- dicarboximides, such as iprodione, myclozolin, procymidone or vinclozolin,
- dithiocarbamates, such as ferbam, nabam, maneb, mancozeb, metam, metiram, propineb, polycarbamate, thiram, ziram or zineb,
- heterocyclic compounds, such as anilazine, benomyl, boscalid, carbendazim, carboxin, oxycarboxin, cyazofamid, dazomet, dithianon, famoxadone, fenamidone, fenarimol, fuberidazole, flutolanil, furametpyr, isoprothiolane, mepronil, nuarimol, picobenzamide, probenazole, proquinazid, pyrifenox, pyroquilon, quinoxyfen, silthiofam, thiabendazole, thifluzamide, thiophanate-methyl, tiadinil, tricyclazole or triforine,
- copper fungicides, such as Bordeaux mixture, copper acetate, copper oxychloride or basic copper sulfate,
- nitrophenyl derivatives, such as binapacryl, dinocap, dinobuton or nitrophthalisopropyl,
- phenylpyrroles, such as fenpiclonil or fludioxonil,
- sulfur,
- other fungicides, such as acibehzolar-S-methyl, benthiavalicarb, carpropamid, chlorothalonil, cyflufenamid, cymoxanil, diclomezine, diclocymet, diethofencarb, edifenphos, ethaboxam, fenhexamid, fentin acetate, fenoxanil, ferimzone, fluazinam, fosetyl, fosetyl-aluminum, phosphorous acid, iprovalicarb, hexachlorobenzene, metrafenone, pencycuron, penthropyrad, propamocarb, phthalide, toloclofosmethyl, quintozene or zoxamide,
- strobilurins, such as azoxystrobin, dimoxystrobin, enestroburin, fluoxastrobin, kresoxim-methyl, metominostrobin, orysastrobin, picoxystrobin, pyraclostrobin or trifloxystrobin,
- sulfenic acid derivatives, such as captafol, captan, dichlofluanid, folpet or tolylfluanid,
- cinnamides and analogous compounds, such as dimethomorph, flumetover or flumorph.

The insecticidal compositions of this invention may also contain other active ingredients, for example other pesticides such as insecticides and herbicides, fertilizers such as ammonium nitrate, urea, potash, and superphosphate, phytotoxicants and plant growth regulators, safeners and nematicides. These additional ingredients may be used sequentially or in combination with the above-described compositions, if appropriate also added only immediately prior to use (tank mix). For example, the plant(s) may be sprayed with a composition of this invention either before or after being treated with other active ingredients.
These agents usually are admixed with the agents according to the invention in a weight ratio of 1:100 to 100:1.

The following list of pesticides together with which the compounds according to the invention can be used, is intended to illustrate the possible combinations, but not to impose any limitation:
A.1. Organo(thio)phosphates: e.g. acephate, azamethiphos, azinphos-methyl, chlorpyrifos, chlorpyrifos-methyl, chlorfenvinphos, diazinon, dichlorvos, dicrotophos, dimethoate, disulfoton, ethion, fenitrothion, fenthion, isoxathion, malathion, methamidophos, methidathion, methyl-parathion, mevinphos, monocrotophos, oxydemetonmethyl, paraoxon, parathion, phenthoate, phosalone, phosmet, phosphamidon, phorate, phoxim, pirimiphos-methyl, profenofos, prothiofos, sulprophos, tetrachlorvinphos, terbufos, triazophos, trichlorfon;
A.2. Carbamates: e.g. alanycarb, aldicarb, bendiocarb, benfuracarb, carbaryl, carbofuran, carbosulfan, fenoxycarb, furathiocarb, methiocarb, methomyl, oxamyl, pirimicarb, propoxur, thiodicarb, triazamate;
A.3. Pyrethroids: e.g. allethrin, bifenthrin, cyfluthrin, cyhalothrin, cyphenothrin, cypermethrin, alpha-cypermethrin, beta-cypermethrin, zeta-cypermethrin, deltamethrin, esfenvalerate, etofenprox, fenpropathrin, fenvalerate, imiprothrin, lambda-cyhalothrin, permethrin, prallethrin, pyrethrin I and II, resmethrin, silafluofen, tau-fluvalinate, tefluthrin, tetramethrin, tralomethrin, transfluthrin, profluthrin, dimefluthrin;
A.4. Growth regulators: a) chitin synthesis inhibitors: e.g. benzoylureas: chlorfluazuron, diflubenzuron, flucycloxuron, flufenoxuron, hexaflumuron, lufenuron, novaluron, teflubenzuron, triflumuron; buprofezin, diofenolan, hexythiazox, etoxazole, clofentazine; b) ecdysone antagonists: e.g. halofenozide, methoxyfenozide, tebufenozide, azadirachtin; c) juvenoids: e.g. pyriproxyfen, methoprene, fenoxycarb; d) lipid biosynthesis inhibitors: e.g. spirodiclofen, spiromesifen or spirotetramat;
A.5. Nicotinic receptor agonists/antagonists compounds (nicotinoid insecticides or neonicotinoids): e.g. clothianidin, dinotefuran, imidacloprid, thiamethoxam, nitenpyram, acetamiprid, thiacloprid or the thiazol compound of formula P1 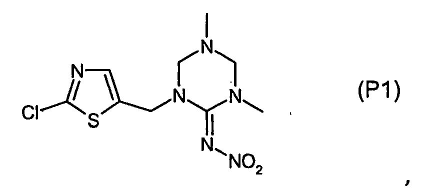
A.6. GABA antagonist compounds: e.g. acetoprole, endosulfan, ethiprole, fipronil, vaniliprole, pyrafluprole, pyriprole, 5-amino-3-(aminothiocarbonyl)-1-(2,6-dichloro-4-trifluoromethylphenyl)-4-(trifluoromethylsulfinyl)-pyrazole;
A.7. Macrocyclic lactone insecticides: abamectin, emamectin, milbemectin, lepimectin, spinosad,
A.8. Mitochondrial complex I electron transport inhibitors (METI I compounds): e.g. fenazaquin, pyridaben, tebufenpyrad, tolfenpyrad, flufenerim;
A.9. Mitochondrial complex II and/or complex III electron transport inhibitors (METI II and III compounds): e.g. acequinocyl, fluacyprim, hydramethylnon;
A.10. Uncoupler compounds: e.g. chlorfenapyr;
A.11. Oxidative phosphorylation inhibitor compounds: cyhexatin, diafenthiuron, fenbutatin oxide, propargite;
A.12. Moulting disruptor compounds: e.g. cyromazine;
A.13. Mixed function oxidase inhibitor compounds: e.g. piperonyl butoxide;
A.14. Sodium channel blocker compounds: e.g. indoxacarb, metaflumizone,
A.15. Various: benclothiaz, bifenazate, cartap, flonicamid, pyridalyl, pymetrozine, sulfur, thiocyclam, flubendiamide, cyenopyrafen, flupyrazofos, cyflumetofen, amidoflumet, compounds of the formula P2: 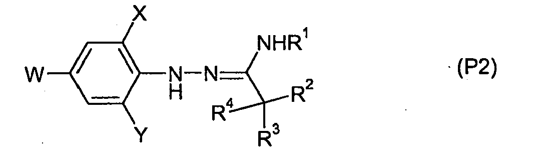 wherein X and Y are each independently halogen, in particular chlorine; W is halogen or C₁-C₂-haloalkyl, in particular trifluoromethyl;
   R¹ is C₁-C₆-alkyl, C₂-C₆-alkenyl, C₂-C₆-alkynyl, C₁-C₄-alkoxy-C₁-C₄-alkyl or C₃-C₆-cycloalkyl each of which may be substituted with 1, 2, 3, 4 or 5 halogen atoms; in particular R¹ is methyl or ethyl;
   R² and R³ are C₁-C₆-alkyl, in particular methyl, or may form together with the adjacent carbon atom a C₃-C₆-cycloalkyl moiety, in particular a cyclopropyl moiety, which may carry 1, 2 or 3 halogen atoms, examples including 2,2-dichlorocyclopropyl and 2,2-dibromocyclopropyl; and
   R⁴ is hydrogen or C₁-C₆-alkyl, in particular hydrogen methyl or ethyl;
anthranilamide compounds of formula P3 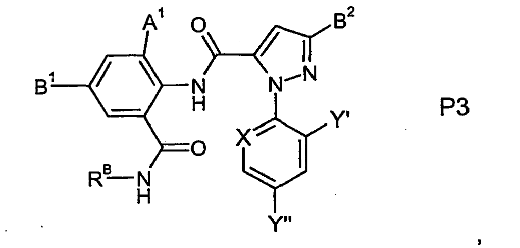 wherein A¹ is CH₃, Cl, Br, I, X is C-H, C-Cl, C-F or N, Y' is F, Cl, or Br, Y" is F, Cl, CF₃, B¹ is hydrogen, Cl, Br, I, CN, B² is Cl, Br, CF₃, OCH₂CF₃, OCF₂H, and R^{B} is hydrogen, CH₃ or CH(CH₃)₂;
and malononitrile compounds as described in JP 2002 284608, WO 02/89579, WO 02/90320, WO 02/90321, WO 04/06677, WO 04/20399, or JP 2004/99597.

Suitable pesticides compounds also include microorganisms such as *Bacillus thuringiensis, Bacillus tenebrionis* and *Bacillus subtilis.*

The aforementioned compositions are particularly useful for protecting plants against infestation of said pests and also for protecting plants against infections of phytopathogenic fungi or to combat these pests/fungi in infested/infected plants.

However, the compounds of formula I are also suitable for the treatment of seeds. Application to the seeds is carried out before sowing, either directly on the seeds or after having pregerminated the latter.

Compositions which are useful for seed treatment are e.g.:
A Soluble concentrates (SL, LS)
D Emulsions (EW, EO, ES)
E Suspensions (SC, OD, FS)
F Water-dispersible granules and water-soluble granules (WG, SG)
G Water-dispersible powders and water-soluble powders (WP, SP, WS)
H Dustable powders (DP, DS)

Preferred FS formulations of compounds of formula I for seed treatment usually comprise from 0.5 to 80% of the active ingredient, from 0,05 to 5 % of a wetter, from 0.5 to 15 % of a dispersing agent, from 0,1 to 5 % of a thickener, from 5 to 20 % of an antifreeze agent, from 0,1 to 2 % of an anti-foam agent, from 1 to 20 % of a pigment and/or a dye, from 0 to 15 % of a sticker /adhesion agent, from 0 to 75 % of a filler/vehicle, and from 0,01 to 1 % of a preservative.

Suitable pigments or dyes for seed treatment formulations are pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108.

Stickers / adhesion agents are added to improve the adhesion of the active materials on the seeds after treatment. Suitable adhesives are block copolymers EO/PO surfactants but also polyvinylalcohols, polyvinylpyrrolidones, polyacrylates, polymethacrylates, polybutenes, polyisobutylenes, polystyrene, polyethyleneamines, polyethyleneamides, polyethyleneimines (Lupasol®, Polymin®), polyethers and copolymers derived from these polymers.

For use against ants, termites, wasps, flies, mosquitos, crickets, or cockroaches, compounds of formula I are preferably used in a bait composition.

The bait can be a liquid, a solid or a semisolid preparation (e.g. a gel). Solid baits can be formed into various shapes and forms suitable to the respective application e.g. granules, blocks, sticks, disks. Liquid baits can be filled into various devices to ensure proper application, e.g. open containers, spray devices, droplet sources, or evaporation sources. Gels can be based on aqueous or oily matrices and can be formulated to particular necessities in terms of stickyness, moisture retention or aging characteristics.

The bait employed in the composition is a product which is sufficiently attractive to incite insects such as ants, termites, wasps, flies, mosquitos, crickets etc. or cockroaches to eat it. The attractiveness can be manipulated by using feeding stimulants or sex pheromones. Food stimulants are chosen, for example, but not exclusively, from animal and/or plant proteins (meat-, fish- or blood meal, insect parts, egg yolk), from fats and oils of animal and/or plant origin, or mono-, oligo- or polyorganosaccharides, especially from sucrose, lactose, fructose, dextrose, glucose, starch, pectin or even molasses or honey. Fresh or decaying parts of fruits, crops, plants, animals, insects or specific parts thereof can also serve as a feeding stimulant. Sex pheromones are known to be more insect specific. Specific pheromones are described in the literature and are known to those skilled in the art.

Formulations of compounds of formula I as aerosols (e.g in spray cans), oil sprays or pump sprays are highly suitable for the non-professional user for controlling pests such as flies, fleas, ticks, mosquitos or cockroaches. Aerosol recipes are preferably composed of the active compound, solvents such as lower alcohols (e.g. methanol, ethanol, propanol, butanol), ketones (e.g. acetone, methyl ethyl ketone), paraffin hydrocarbons (e.g. kerosenes) having boiling ranges of approximately 50 to 250 °C, dimethylformamide, N-methylpyrrolidone, dimethyl sulfoxide, aromatic hydrocarbons such as toluene, xylene, water, furthermore auxiliaries such as emulsifiers such as sorbitol monooleate, oleyl ethoxylate having 3-7 mol of ethylene oxide, fatty alcohol ethoxylate, perfume oils such as ethereal oils, esters of medium fatty acids with lower alcohols, aromatic carbonyl compounds, if appropriate stabilizers such as sodium benzoate, amphoteric surfactants, lower epoxides, triethyl orthoformate and, if required, propellants such as propane, butane, nitrogen, compressed air, dimethyl ether, carbon dioxide, nitrous oxide, or mixtures of these gases.

The oil spray formulations differ from the aerosol recipes in that no propellants are used.

The compounds of formula I and its respective compositions can also be used in mosquito and fumigating coils, smoke cartridges, vaporizer plates or long-term vaporizers and also in moth papers, moth pads or other heat-independent vaporizer systems.

The compounds of formula I and its compositions can be used for protecting non-living material, in particular cellulose-based materials such as wooden materials e.g. trees, board fences, sleepers, etc. and buildings such as houses, outhouses, factories, but also construction materials, furniture, leathers, fibers, vinyl articles, electric wires and cables etc. from ants and/or termites, and for controlling ants and termites from doing harm to crops or human being (e.g. when the pests invade into houses and public facilities). The compounds of formula I are applied not only to the surrounding soil surface or into the under-floor soil in order to protect wooden materials but it can also be applied to lumbered articles such as surfaces of the under-floor concrete, alcove posts, beams, plywoods, furniture, etc., wooden articles such as particle boards, half boards, etc. and vinyl articles such as coated electric wires, vinyl sheets, heat insulating material such as styrene foams, etc. In case of application against ants doing harm to crops or human beings, the ant controller of the present invention is applied,to the crops or the surrounding soil, or is directly applied to the nest of ants or the like.

In the methods according to the invention the pests are controlled by contacting the target parasite/pest, its food supply, habitat, breeding ground or its locus with a pesticidally effective amount of at least one compounds I, or the N-oxide or salt thereof, or with a composition, containing a pesticidally effective amount of at least one compound I, or the N-oxide or salt thereof.

"Locus" means a habitat, breeding ground, plant, seed, soil, area, material or environment in which a pest or parasite is growing or may grow.

In general, "pesticidally effective amount" means the amount of active ingredient needed to achieve an observable effect on growth, including the effects of necrosis, death, retardation, prevention, and removal, destruction, or otherwise diminishing the occurrence and activity of the target organism. The pesticidally effective amount can vary for the various compounds/compositions used in the invention. A pesticidally effective amount of the compositions will also vary according to the prevailing conditions such as desired pesticidal effect and duration, weather, target species, locus, mode of application, and the like.

The compounds of the invention can also be applied preventively to places at which occurrence of the pests is expected.

The compounds of formula I may be also used to protect growing plants from attack or infestation by pests by contacting the plant with a pesticidally effective amount of compounds of formula I. As such, "contacting" includes both direct contact (applying the compounds/compositions directly on the pest and/or plant - typically to the foliage, stem or roots of the plant) and indirect contact (applying the compounds/compositions to the locus of the pest and/or plant).

The compounds I are employed by treating the fungi, pests or the plants, seeds, materials or the soil to be protected from fungal attack or pesticidal attack with a fungicidally or pesticidally effective amount of at least one active compound I, its N-oxide or salt. The application can be carried out both before and after the infection/infestation of the materials, plants or seeds by the fungi or pest.

When employed in plant protection, the amounts applied are, depending on the kind of effect desired, in the range of 0.1 g to 4000 g per hectare, desirably from 25 g to 600 g per hectare, more desirably from 50 g to 500 g per hectare.

In the treatment of seed, the application rates of the active compounds are generally from 0.001 g to 100 g per kg of seed, preferably from 0.01 g to 50 g per kg of seed, in particular from 0.01 g to 2 g per kg of seed.

In the case of soil treatment or of application to the pests dwelling place or nest, the quantity of active ingredient ranges from 0.0001 to 500 g per 100 m², preferably from 0.001 to 20 g per 100 m².

Customary application rates in the protection of materials are, for example, from 0.01 g to 1000 g of active compound per m² treated material, desirably from 0.1 g to 50 g per m².

Insecticidal compositions for use in the impregnation of materials typically contain from 0.001 to 95 weight %, preferably from 0.1 to 45 weight %, and more preferably from 1 to 25 weight % of at least one repellent and / or insecticide.

For use in bait compositions, the typical content of active ingredient is from 0.001 weight % to 15 weight %, desirably from 0.001 weight % to 5% weight % of active compound.

For use in spray compositions, the content of active ingredient is from 0.001 to 80 weights %, preferably from 0.01 to 50 weight % and most preferably from 0.01 to 15 weight %.

When used in the protection of materials or stored products, the amount of active compound applied depends on the kind of application area and on the desired effect. Amounts customarily applied in the protection of materials are, for example, 0.001 g to 2 kg, preferably 0.005 g to 1 kg, of active compound per cubic meter of treated material.

Under outdoor conditions, the active compound application rate for controlling pests is from 0.1 to 2.0, preferably from 0.2 to 1.0, kg/ha.

Various types of oils, wetters, adjuvants, herbicides, fungicides, other pesticides, or bactericides may be added to the active compounds, if appropriate not until immediately prior to use (tank mix). These agents can be admixed with the agents according to the invention in a weight ratio of 1:100 to 100:1, preferably 1:10 to 10:1.

Adjuvants which can be used are in particular organic modified polysiloxanes such as Break Thru S 240^{®}; alcohol alkoxylates such as Atplus 245^{®}, Atplus MBA 1303^{®}, Plurafac LF 300^{®} and Lutensol ON 30^{®}; EO/PO block polymers, z. B. Pluronic RPE 2035^{®} and Genapol B^{®}; alcohol ethoxylates such as Lutensol XP 80^{®}; and dioctyl sulfosuccinate sodium such as Leophen RA^{®}.

Compounds of formula I, their N-oxides and veterinarily acceptable salts as well as the compositions comprising them can also be used for controlling and preventing infestations and infections in animals including warm-blooded animals (including humans) and fish. They are for example suitable for controlling and preventing infestations and infections in mammals such as cattle, sheep, swine, camels, deer, horses, pigs, poultry, rabbits, goats, dogs and cats, water buffalo, donkeys, fallow deer and reindeer, and also in fur-bearing animals such as mink, chinchilla and raccoon, birds such as hens, geese, turkeys and ducks and fish such as fresh- and salt-water fish such as trout, carp and eels.

Infestations in warm-blooded animals and fish include, but are not limited to, lice, biting lice, ticks, nasal bots, keds, biting flies, muscoid flies, flies, myiasitic fly larvae, chiggers, gnats, mosquitoes and fleas.

The compounds of formula I and compositions comprising them are suitable for systemic and/or non-systemic control of ecto- and/or endoparasites. They are active against all or some stages of development.

Administration can be carried out both prophylactically and therapeutically. Administration of the active compounds is carried out directly or in the form of suitable preparations, orally, topically/dermally or parenterally.

For oral administration to warm-blooded animals, the formula I compounds may be formulated as animal feeds, animal feed premixes, animal feed concentrates, pills, solutions, pastes, suspensions, drenches, gels, tablets, boluses and capsules. In addition, the formula I compounds may be administered to the animals in their drinking water. For oral administration, the dosage form chosen should provide the animal with 0.01 mg/kg to 100 mg/kg of animal body weight per day of the formula I compound, preferably with 0.5 mg/kg to 100 mg/kg of animal body weight per day.

Alternatively, the formula I compounds, their N-oxides and salts may be administered to animals parenterally, for example, by intraruminal, intramuscular, intravenous or subcutaneous injection. The formula I compounds may be dispersed or dissolved in a physiologically acceptable carrier for subcutaneous injection. Alternatively, the formula I compounds may be formulated into an implant for subcutaneous administration. In addition the formula I compound may be transdermally administered to animals. For parenteral administration, the dosage form chosen should provide the animal with 0.01 mg/kg to 100 mg/kg of animal body weight per day of the formula I compound.

The formula I compounds may also be applied topically to the animals in the form of dips, dusts, powders, collars, medallions, sprays, shampoos, spot-on and pour-on formulations and in ointments or oil-in-water or water-in-oil emulsions. For topical application, dips and sprays usually contain 0.5 ppm to 5,000 ppm and preferably 1 ppm to 3,000 ppm of the formula I compound. In addition, the formula I compounds may be formulated as ear tags for animals, particularly quadrupeds such as cattle and sheep.

Suitable preparations are:
- Solutions such as oral solutions, concentrates for oral administration after dilution, solutions for use on the skin or in body cavities, pouring-on formulations, gels;
- Emulsions and suspensions for oral or dermal administration; semi-solid preparations;
- Formulations in which the active compound is processed in an ointment base or in an oil-in-water or water-in-oil emulsion base;
- Solid preparations such as powders, premixes or concentrates, granules, pellets, tablets, boluses, capsules; aerosols and inhalants, and active compound-containing shaped articles.

Generally it is favorable to apply solid formulations which release compounds of formula I in total amounts of 10 mg/kg to 300 mg/kg, preferably 20 mg/kg to 200 mg/kg. The active compounds can also be used as a mixture with synergists or with other active compounds which act against pathogenic endo- and ectoparasites.

In general, the compounds of formula I are applied in parasiticidally effective amount-meaning the amount of active ingredient needed to achieve an observable effect on growth, including the effects of necrosis, death, retardation, prevention, and removal, destruction, or otherwise diminishing the occurrence and activity of the target organism. The parasiticidally effective amount can vary for the various compounds/compositions used in the invention. A parasiticidally effective amount of the compositions will also vary according to the prevailing conditions such as desired parasiticidal effect and duration, target species, mode of application, and the like.

### Synthesis examples

The procedures described in the synthesis examples below were used to prepare further compounds I by appropriate modification of the starting compounds. The compounds thus obtained are listed in the tables below, together with physical data.

### Example 1: Preparation of N-(pyridin-4-ylmethyl)-4'-cyanobiphenyl-4-sulfonamide

0.73 g of triethylamine was added to a solution of 2 g of 4'-cyanobiphenyl-4-sulfonyl chloride (cf. EP-A 483 667) in 80 ml of diethyl ether, and this was followed by addition of a solution of 0.78 g of 4-(aminomethyl)pyridine in 15 ml of dioxane. After 18 hours of stirring at about 23°C, the solvent was distilled off and the residue was chromatographed on silica gel (methyl tert.-butyl ether[MtBE]/methanol mixture). This gave 0.9 g of the title compound of m.p. 198-202°C.

### Example 2: Preparation of N-(pyridin-4-ylmethyl)-2'-chlorobiphenyl-4-sulfonamide

### 2a: 4-Iodo-N-pyridin-4-ylmethylphenylsulfonamide

At 0-5°C, 7 ml of pyridine were added to a solution of 50 g of 4-iodophenylsulfonyl chloride in 400 ml of diethyl ether, and this was followed by dropwise addition of a solution of 17.8 g of 4-(aminomethyl)pyridine in 20 ml of dioxane. After 18 hours of stirring at 23°C, the precipitate was filtered off, washed with diluted NaHCO₃ solution and then dried. 29.9 g of the title compound of m.p. 174-176°C remained.

### 2b: N-(pyridin-4-ylmethyl)-2'-chlorobiphenyl-4-sulfonamide

4.2 g of 2-chlorophenylboronic acid and then a solution of 7.1 g of Na₂CO₃ in 100 ml of water were added to a solution of 5 g of the sulfonamide from Ex. 2a in 200 ml of tetrahydrofuran (THF). After addition of 1 g of palladium acetate and about 100 mg of Pd[P(phenyl)]₃, the mixture was heated under reflux for 5 hours and then added to water. After extraction with methyl-tert.-butyl ether, the organic phases were washed with water and then dried, and the solvent was removed. The residue gave, after chromatography on silica gel (methyl-tert.-butyl ether/methanol mixture), 3.8 g of the title compound of m.p. 138-141°C.

### Example 3: Preparation of 4'-cyanobiphenyl-4-(sulfonic acid)-(2-methoxypyridin-4-yl-methyl)amide

### 3a: 2-methoxy-isonicotinonitrile

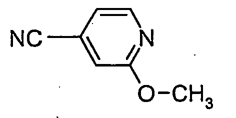

At 5-10°C, a solution of 60 g (0.433 mol) 2-chloro-isonicotinonitrile in 200 ml dimethylformamide was added dropwise to 25 g (0.46 mol) solid sodium methoxide in 100 ml dimethylformamide. Subsequently, the mixture was stirred for 2 hours at 5°C and then for 12 hours at 23°C. The reaction mixture was poured onto water and then extracted with methyl tert.-butyl ether. The combined organic phases were washed with water and dried. The solvent was removed at reduced pressure. Yield: 47.5 g useful product. ¹H-NMR (in CDCl₃): δ [ppm] = 4.0 (s), 6.9 (s), 7.9 (m) and 8.3 (m).

### 3b: C-(2-methoxypyridin-4-yl)methylamine

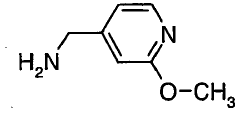

A solution of 57.5 g (0.42 mol) 2-methoxy-isonicotinonitrile from 3a in 1,000 ml ethanol was treated with 500 ml 25 % aqueous ammonia solution. Hydrogenation was then carried out for 7 hours in the presence of 14 g Pd/C (10 %) at 0-5°C and a pressure of 0.1 bar. After filtration and removal of solvent the residue was taken up in methylene dichloride. It was then washed once with water, dried and the solvent was removed at reduced pressure. The residue was chromatographed with methyl tert.-butyl ether over silica gel. Yield: 17 g useful product in the form of an oil.
¹H-NMR (in CDCl₃): δ [ppm] = 3.7 (s), 3.9 (s), 6.7 (s), 6.8 (m) and 8.1 (m).

### 3c: 4-Iodo-N-(2-methoxypyridin-4-ylmethyl)benzenesulfonamide

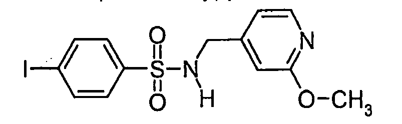

40 g (0.29 mol) C-(2-methoxypyridin-4-yl)methylamine from Example 3b in 150 ml acetonitrile were mixed at 0-5°C with 29.4 g (0.29 mol) triethylamine. A solution of 79.7 g (0.263 mol) 4-iodophenylsulfonyl chloride in 300 ml acetonitrile was added dropwise to this and the mixture was stirred for 18 hours at 23°C. The mixture was then filtered and the filtrate evaporated. The residue was mixed with water and stirred for four hours.
The product obtained was filtered off after which it was washed with a little methylene dichloride and dried. Yield: 43 g useful product.
¹H-NMR (in d⁶-dimethylsulfoxide): δ [ppm] = 3.8 (s), 4.0 (s), 7.5 (m), 7.9 (m) and 8.4 (m).

### 3d: 4'-Cyanobiphenyl-4-(sulfonic acid)-(2-methoxypyridin-4-ylmethyl)amide

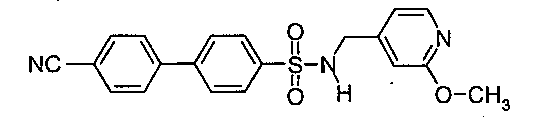

A solution of 25 g (0.061 mol) 4-iodo-N-(2-methoxypyridin-4-ylmethyl)benzenesulfonamide from Example 3c in 300 ml tetrahydrofuran was mixed with 18.18 g (0.122 mol) 4-cyanophenylboronic acid and then with 32.8 g (0.30 mol) sodium carbonate in 130 ml water. After adding 0.6 g [1,4 -bis-(diphenylphosphino)-butane]-palladium(II) chloride the mixture was refluxed for 2 hours and then added to water. Following extraction with methyl tert.-butyl ether the organic phases were washed with water, then dried and freed of solvent. The residue was stirred with a small quantity of methylene dichloride, separated off and dried. Yield: 20.4 g useful product; m.p.: 120-125°C.

### Example 4: Preparation of 4'-Chlorobiphenyl-4-(sulfonic acid)-(3-methoxypyridin-4-yl-methyl)amide

### 4a: 3-methoxy-isonicotinonitrile

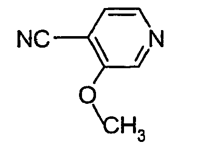

At 5°C, a solution of 62.8 g (0.453 mol) 3-chloro-isonicotinonitrile (for preparation see J. Heterocyclic Chem. 15, 683 (1978)) in 200 ml dimethylformamide was added dropwise to 26.3 g (0.48 mol) solid sodium methoxide in 100 ml dimethylformamide. Subsequently, the mixture was stirred for 2 hours at 5 °C and then for 18 hours at 23°C. The reaction solution was poured onto water and then extracted with methyl tert.-butyl ether. The combined organic phases were washed with water, dried and the solvent was removed at reduced pressure. Yield: 39.5 g useful product.
¹H-NMR (in CDCl₃): δ [ppm] = 4.0 (s), 7.5 (m), 8.4 (m) and 8.5 (s).

### 4b: C-(3-methoxypyridin-4-yl)methylamine

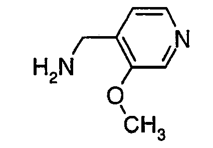

A solution of 34 g (0.254 mol) 3-methoxy-isonicotinonitrile from Example 4a in 1,000 ml ethanol was treated with 336 ml 25 % aqueous ammonia solution after which hydrogenation was carried out for 6.5 hours in the presence of 18 g Pd/C (10 %) at 0-5°C and a pressure of 0.1 bar. The solids were then filtered off. The filtrate was evaporated. The residue was taken up in methylene dichloride. The organic phase was washed once with water, then dried and the solvent was finally removed at reduced pressure. After chromatography over silica gel using methyl tert.-butyl ether as solvent 26.9 g useful product in the form of oil was obtained.
¹H-NMR (in CDCl₃): δ [ppm] = 3.7 (s), 3.9 (s), 7.2 (m) and 8.3 (m).

### 4c: 4'-Chlorobiphenyl-4-(sulfonic acid)-(3-methoxypyridin-4-ylmethyl)amide

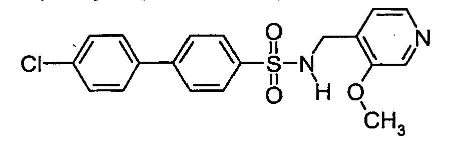

8 g (0.059 mol) C-(3-methoxypyridin-4-yl)methylamine from Example 4b in 100 ml acetonitrile were mixed at 0-5°C with 5.9 g (0.057 mol) triethylamine. A solution of 15.15 g (0.052 mol) 4'-chlorobiphenyl-4-sulfonyl chloride in 100 ml acetonitrile was added dropwise to this. The reaction mixture was then stirred for a further 18 hours at 23°C after which the solids were filtered off. The filtrate was evaporated. The residue was stirred for 7 hours with water/n-pentane. The solid portion was then separated off, washed with a little methyl tert.-butyl ether and dried. Yield: 14.3 g useful product; m.p.: 127-131 °C.

### Example 5: Preparation of 2,2,4'-trifluorobiphenyl-4-(sulfonic acid)-(pyridin-4-yl-methyl)-amide

### 5a: 4-Bromo-3-fluoro-N-pyridin-4-ylmethylbenzenesulfonamide

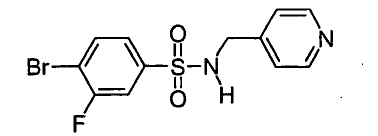

2.17 g (0.020 mol) picolinamine in 80 ml acetonitrile were mixed at 0-5 °C with 2 g (0.020 mol) triethylamine. A solution of 15.15 g (0.052 mol) 4-bromo-3-fluoro-phenylsulfonyl chloride in 20 ml acetonitrile was added dropwise to this. Subsequently, the reaction mixture was stirred for a further 18 hours at 23°C and then evaporated. The residue was mixed with water. After stirring for 3 hours the solid portion was separated off, washed with n-pentane and dried. Yield: 6.1 g useful product.
¹H-NMR (in d⁶-dimethylsulfoxide): δ [ppm] = 4.1 (s), 7.2 (s), 7.6 (m), 7.7 (m), 7.9 (m), 8.5 (m) and 8.6 (m).

### 5b: 2,2,4'-Trifluorobiphenyl-4-(sulfonic acid)-(pyridin-4-ylmethyl)amide

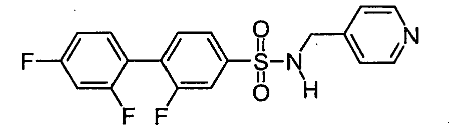

A solution of 0.5 g (1.4 mmol) 4-bromo-3-fluoro-N-pyridin-4-ylmethylbenzenesulfonamide from Example 5a in 80 ml tetrahydrofuran was mixed with 0.46 g (2.9 mmol) 2,4-difluorophenylboronic acid and then with 0.78 g (7.2 mmol) sodium carbonate in 30 ml water. After adding 0.1 g [1,4 -bis-(diphenylphosphino)butane]-palladium(II) chloride the mixture was refluxed for 5 hours and then added to water. Following extraction with methyl tert.-butyl ether, the organic phase was washed with water, then dried and freed of solvent. The residue was chromatographed with methyl tert.-butyl ether over silica gel. Yield: 90 mg useful product.
¹H-NMR (in d⁶-dimethylsulfoxide): δ [ppm] = 4.2 (s), 7.2 (m), 7.4-7.7 (m), 8.4 (m) and 8.6 (m).

Further biphenylsulfonamides I, which have been prepared or can be prepared in a way similar to the procedures described in Examples 1 to 5, are listed in the following tables B) and C):

**Table B) - Compounds of the formula I'**

| | | | | |
|---|---|---|---|---|
| 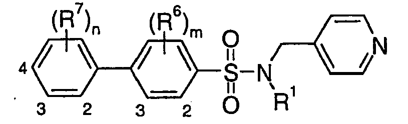 I' (= I whereas R², R³, R⁴, R⁵ are hydrogen) | | | | |

| No. | (R⁷)ₙ | (R⁶)ₘ | R¹ | Phys.data (m.p.[°C]; MS M+H⁺ [m/e]) |
|---|---|---|---|---|
| I'-1 | 4-Cl | - | H | 208-212 |
| I'-2 | 4-F | - | H | 195-198 |
| I'-3 | 4-CF₃ | - | H | 196-200 |
| I'-4 | 2-CH₃ | - | H | 175-178 |
| I'-5 | 4-OCH₃ | - | H | 200-203 |
| I'-6 | - | 2-CF₃ | H | 393.0 |
| I'-7 | 3-Cl, 4-F | - | H | 225-230 |
| I'-8 | 3-CH₃, 4-F | - | H | 178-180 |
| I'-9 | 4-F | 2-CF₃ | H | 168-172 |
| I'-10 | 2-F, 4-CH₃ | - | H | 187-188 |
| I'-11 | 4-CN | - | H | 198-201 |
| I'-12 | 2-Cl | - | H | 132-137 |
| I'-13 | 4-CH₃ | - | H | 217-218 |
| I'-14 | - | 3-CF₃ | H | 393.25 |
| I'-15 | - | 2-Cl | H | 358.8 |
| I'-16 | 2-OCH₃ | 2-Cl | H | 3888 |
| I'-17 | 4-SCH₃ | 2-Cl | H | 404.8 |
| I'-18 | 3-NO₂, 4-CH₃ | 2-Cl | H | 417.8 |
| I'-19 | 4-F | 2-Cl | H | 376.8 |
| I'-20 | 3,5-Cl₂ | 2-Cl | H | 428.7 |
| I'-21 | - | 3-Cl | H | 338.9 |
| I'-22 | 2-OCH₃ | 3-CH₃ | H | 368.9 |
| I'-23 | 4-SCH₃ | 3-CH₃ | H | 384.9 |
| I'-24 | 4-F | 3-CH₃ | H | 356.9 |
| I'-25 | 3,5-Cl₂ | 3-CH₃ | H | 406.8 |
| I'-26 | 4-CH₃ | 3-CF₃ | H | 407.2 |
| I'-27 | 4-CF₃ | 3-CF₃ | H | 461.2 |
| I'-28 | 3-Cl, 4-F | 3-CF₃ | H | 445.2 |
| I'-29 | 3-NHC(O)CH₃ | 2-Cl | H | 416.25 |
| I'-30 | 4-CH₃ | 2-Cl | H | 373.2 |
| I'-31 | 3-Cl, 4-F | 2-Cl | H | 411.25 |
| I'-32 | 4-CH₃ | 3-CH₃ | H | 353.3 |
| I'-33 | 3,5-(CF₃)₂ | 3-CH₃ | H | 475.3 |
| I'-34 | 4-CF₃ | 3-CH₃ | H | 407.3 |
| I'-35 | 3-Cl, 4-F | 3-CH₃ | H | 391.2 |
| I'-36 | 4-Cl | 3-CF₃ | H | 427.0 |
| I'-37 | 4-Br | 3-CF₃ | H | 472.9 |
| I'-38 | 2-CH₃ | 3-CF₃ | H | 407.0 |
| I'-39 | 3-NO₂ | 3-CF₃ | H | 438.0 |
| I'-40 | 2,4-Cl₂ | 3-CF₃ | H | 460.9 |
| I'-41 | 4-Cl | 2-Cl | H | 393.0 |
| I'-42 | 4-Br | 2-Cl | H | 438.9 |
| I'-43 | 2-CH₃ | 2-Cl | H | 373.0 |
| I'-44 | 3-NO₂ | 2-Cl | H | 404.0 |
| I'-45 | 2,4-Cl₂ | 2-Cl | H | 428.9 |
| I'-46 | 4-Cl | 3-CH₃ | H | 373.0 |
| I'-47 | 2-CH₃ | 3-CH₃ | H | 353.1 |
| I'-48 | 3-NO₂ | 3-CH₃ | H | 384.1 |
| I'-49 | 2,4-Cl₂ | 3-CH₃ | H | 407.0 |
| I'-50 | 2-F | 3-CF₃ | H | 411.0 |
| I'-51 | 2,4-F₂ | 3-CF₃ | H | 429.0 |
| I'-52 | 3-CH₃, 4-Cl | 3-CF₃ | H | 441.0 |
| I'-53 | 4-C₆H₅ | 3-CF₃ | H | 469.1 |
| I'-54 | 4-OCH₃ | 3-CF₃ | H | 423.1 |
| I'-55 | 2,5-F₂ | 3-CF₃ | H | 429.0 |
| I'-56 | 2-F | 2-Cl | H | 337.2 |
| I'-57 | 3,5-F₂ | 2-Cl | H | 395.2 |
| I'-58 | 2,4-F₂ | 2-Cl | H | 395.2 |
| I'-59 | 3-CH₃, 4-Cl | 2-Cl | H | 407.0 |
| I'-60 | 4-C₆H₅ | 2-Cl | H | 435.2 |
| I'-61 | 4-OCH₃ | 2-Cl | H | 389.2 |
| I'-62 | 2,5-F₂ | 2-Cl | H | 395.2 |
| I'-63 | 2-F | 3-CH₃ | H | 357.2 |
| I'-64 | 3,5-F₂ | 3-CH₃ | H | 375.2 |
| I'-65 | 2,4-F₂ | 3-CH₃ | H | 375.1 |
| I'-66 | 3-CH₃, 4-Cl | 3-CH₃ | H | 387.0 |
| I'-67 | 4-C₆H₅ | 3-CH₃ | H | 415.3 |
| I'-68 | 4-OCH₃ | 3-CH₃ | H | 369.1 |
| I'-69 | 2,5-F₂ | 3-CH₃ | H | 375.2 |
| I'-70 | 2-Cl | 3-CF₃ | H | 427.0 |
| I'-71 | 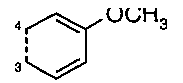 | 3-CF₃ | H | 473.1 |
| I'-72 | 4-C(O)CH₃ | 3-CF₃ | H | 435.0 |
| I'-73 | 3-CH₃ | 3-CF₃ | H | 407.0 |
| I'-74 | 2-OCH₃, 5-Br | 3-CF₃ | H | 503.0 |
| I'-75 | 2-Cl | 2-Cl | H | 393.0 |
| I'-76 | 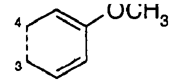 | 2-Cl | H | 439.0 |
| I'-77 | 4-C(O)CH₃ | 2-Cl | H | 401.0 |
| I'-78 | 3-CH₃ | 2-Cl | H | 373.0 |
| I'-79 | 2,6-F₂ | 2-Cl | H | 393.0 |
| I'-80 | 2-OCH₃, 5-Br | 2-Cl | H | 468.9 |
| I'-81 | 2-Cl | 3-CH₃ | H | 373.0 |
| I'-82 | 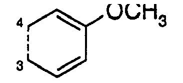 | 3-CH₃ | H | 180-182 |
| I'-83 | 4-C(O)CH₃ | 3-CH₃ | H | 381.1 |
| I'-84 | 3-CH₃ | 3-CH₃ | H | 353.1 |
| I'-85 | 2-OCH₃, 5-Br | 3-CH₃ | H | 449.0 |
| I'-86 | 2-OCH₃ | 3-F | H | 373.1 |
| I'-87 | 4-SCH₃ | 3-F | H | 389.0 |
| I'-88 | 3-NO₂, 4-CH₃ | 3-F | H | 402.0 |
| I'-89 | 4-C(O)CH₃ | 3-F | H | 399.0 |
| I'-90 | - | - | H | 180-182 |
| I'-91 | - | - | CH₂CH₃ | |
| I'-92 | - | - | CH₂CH₂CH₃ | |
| I'-93 | - | - | CH₂CH=CH₂ | |
| I'-94 | - | - | CH₂CH≡CH | |
| I'-95 | - | - | CH₂C₆H₅ | |
| I'-96 | 4-F | 3-F | H | 361.0 |
| I'-97 | H | 3-F | H | 343.1 |
| I'-98 | 2-OCH₃ | 3-Cl | H | 389.0 |
| I'-99 | 4-SCH₃ | 3-Cl | H | 405.0 |
| I'-100 | 3-NO₂, 4-CH₃ | 3-Cl | H | 418.0 |
| I'-101 | 3-NHC(O)CH₃ | 3-Cl | H | 416.0 |
| I'-102 | H | 3-Cl | H | 359.0 |
| I'-103 | H | 4-CH(CH₃)₂ | H | oil |
| I'-104 | 2-OCH₃ | 2,6-Cl₂ | H | 423.0 |
| I'-105 | 4-SCH₃ | 2,6-Cl₂ | H | 439.0 |
| I'-106 | 3-NO₂, 4-CH₃ | 2,6-Cl₂ | H | 452.0 |
| I'-107 | 3-NHC(O)CH₃ | 2,6-Cl₂ | H | 450.0 |
| I'-108 | 4-F | 2,6-Cl₂ | H | 411.0 |
| I'-109 | H | 2,6-Cl₂ | H | 427.0 |
| I'-110 | 4-Cl | 3-Cl | H | 393.25 |
| I'-111 | 4-Br | 3-Cl | H | 439.15 |
| I'-112 | 4-CH₃ | 2,6-Cl₂ | H | 407.25 |
| I'-113 | 3,5-Cl₂ | 2,6-Cl₂ | H | 463.15 |
| I'-114 | 3,5-(CF₃)₂ | 2,6-Cl₂ | H | 529.25 |
| I'-115 | 4-CF₃ | 2,6-Cl₂ | H | 461.25 |
| I'-116 | 3-Cl, 4-F | 2,6-Cl₂ | H | 445.15 |
| I'-117 | 4-Cl | 2,6-Cl₂ | H | 429.15 |
| I'-118 | 4-Br | 2,6-Cl₂ | H | 473.15 |
| I'-119 | 2-CH₃ | 3-F | H | 357.25 |
| I'-120 | 4-CO₂H | 3-F | H | 387.25 |
| I'-121 | 3-NO₂ | 3-F | H | 388.25 |
| I'-122 | 2,4-Cl₂ | 3-F | H | 411.15 |
| I'-123 | 2-F | 3-F | H | 361.25 |
| I'-124 | 3,5-F₂ | 3-F | H | 379.25 |
| I'-125 | 2,4-F₂ | 3-F | H | 379.25 |
| I'-126 | 2-CH₃ | 3-Cl | H | 373.25 |
| I'-127 | 4-CO₂H | 3-Cl | H | 363.15 |
| I'-128 | 3-NO₂ | 3-Cl | H | 404.25 |
| I'-129 | 2,4-Cl₂ | 3-Cl | H | 428.9 |
| I'-130 | 2-F | 3-Cl | H | 377.25 |
| I'-131 | 3,5-F₂ | 3-Cl | H | 395.25 |
| I'-132 | 2,4-F₂ | 3-Cl | H | 395.25 |
| I'-133 | 2-CH₃ | 2,6-Cl₂ | H | 407.25 |
| I'-134 | 3-NO₂ | 2,6-Cl₂ | H | 438.15 |
| I'-135 | 2,4-Cl₂ | 2,6-Cl₂ | H | 463.15 |
| I'-136 | 2-F | 2,6-Cl₂ | H | 411.50 |
| I'-137 | 3,5-F₂ | 2,6-Cl₂ | H | 429.25 |
| I'-138 | 2,4-F₂ | 2,6-Cl₂ | H | 429.15 |
| I'-139 | 3-CH₃, 4-Cl | 3-F | H | 391.25 |
| I'-140 | 4-C₆H₅ | 3-F | H | 419.25 |
| I'-141 | 4-OCH₃ | 3-F | H | 373.25 |
| I'-142 | 2,5-F₂ | 3-F | H | 379.25 |
| I'-143 | 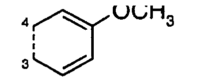 | 3-F | H | 423.25 |
| I'-144 | 3-CH₃, 4-Cl | 3-Cl | H | 407.15 |
| I'-145 | 2,5-F₂ | 3-Cl | H | 395.25 |
| I'-146 | 2-Cl | 3-Cl | H | 393.15 |
| I'-147 | 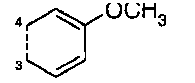 | 3-Cl | H | 439.25 |
| I'-148 | 3-CH₃, 4-Cl | 2,6-Cl₂ | H | 441.15 |
| I'-149 | 4-C₆H₅ | 2,6-Cl₂ | H | 469.25 |
| I'-150 | 4-OCH₃ | 2,6-Cl₂ | H | 423.25 |
| I'-151 | 2,5-F₂ | 2,6-Cl₂ | H | 429.25 |
| I'-152 | 2-Cl | 2,6-Cl₂ | H | 425.15 |
| I'-153 | 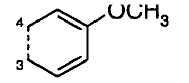 | 2,6-Cl₂ | H | 473.25 |
| I'-154 | 4-OCF₃ | 3-CF₃ | H | 477.25 |
| I'-155 | 3,5-(CH₃)₂ | 3-CF₃ | H | 421.25 |
| I'-156 | 3,4-(OCH₃)₂ | 3-CF₃ | H | 453.35 |
| I'-157 | 3-F | 3-CF₃ | H | 411.25 |
| I'-158 | 4-OCF₃ | 3-CH₃ | H | 423.25 |
| I'-159 | 3,5-(CH₃)₂ | 3-CH₃ | H | 367.35 |
| I'-160 | 3,4-(OCH₃)₂ | 3-CH₃ | H | 399.35 |
| I'-161 | 3-F | 3-CH₃ | H | 357.25 |
| I'-162 | 4-OCF₃ | 3-F | H | 427.25 |
| I'-163 | 3,5-(CH₃)₂ | 3-F | H | 371.25 |
| I'-164 | 3,4-(OCH₃)₂ | 3-F | H | 403.25 |
| I'-165 | 3-F | 3-F | H | 361.25 |
| I'-166 | 4-OCF₃ | 3-Cl | H | 443.25 |
| I'-167 | 3,5-(CH₃)₂ | 3-Cl | H | 387.25 |
| I'-168 | 3,4-(OCH₃)₂ | 3-Cl | H | 419.25 |
| I'-169 | 3-F | 3-Cl | H | 377.25 |
| I'-170 | 4-CH₂CH₃ | 3-CF₃ | H | 421.35 |
| I'-171 | 2,3-(CH₃)₂ | 3-CF₃ | H | . 421.25 |
| I'-172 | 2,5-(OCH₃)₂ | 3-CF₃ | H | 453.35 |
| I'-173 | 2-CF₃ | 3-CH₃ | H | 407.25 |
| I'-174 | 4-CH₂CH₃ | 3-CH₃ | H | 367.25 |
| I'-175 | 2,3-(CH₃)₂ | 3-CH₃ | H | 367.25 |
| I'-176 | 2,4-(OCH₃)₂ | 3-CH₃ | H | 399.35 |
| I'-177 | 4-CH₂CH₃ | 3-F | H | 371.25 |
| I'-178 | 2,3-(CH₃)₂ | 3-F | H | 371.25 |
| I'-179 | 2,5-(OCH₃)₂ | 3-F | H | 403.1 |
| I'-180 | 2-CF₃ | 3-Cl | H | 425.9 |
| I'-181 | 4-CH₂CH₃ | 3-Cl | H | 387.25 |
| I'-182 | 2,3-(CH₃)₂ | 3-Cl | H | 387.25 |
| I'-183 | 2,5-(OCH₃)₂ | 3-Cl | H | 419.25 |
| I'-184 | 4-CH₃ | 3-F | H | 357.25 |
| I'-185 | 3,5-Cl₂ | 3-F | H | 411.15 |
| I'-186 | 3,5-(CF₃)₂ | 3-F | H | 479.25 |
| I'-187 | 4-CF₃ | 3-F | H | 411.25 |
| I'-188 | 3-Cl, 4-F | 3-F | H | 395.25 |
| I'-189 | 4-CH₃ | 3-Cl | H | 373.25 |
| I'-190 | 3,5-Cl₂ | 3-Cl | H | 429.17 |
| I'-191 | 3,5-(CF₃)₂ | 3-Cl | H | 495.25 |
| I'-192 | 4-CF₃ | 3-Cl | H | 427.25 |
| I'-193 | 3-Cl, 4-F | 3-Cl | H | 411.15 |
| I'-194 | CO₂CH₃ | - | H | 190-200 |
| I'-195 | 2,5-(CH₃)₂ | 3-CF₃ | H | 421.10 |
| I'-196 | 2-naphthyl | 3-CF₃ | H | 443.0 |
| I'-197 | 2,5-(OCH₃)₂ | 3-CF₃ | H | 453.10 |
| I'-198 | 3-OCH₃ | 3-CF₃ | H | 423.10 |
| I'-199 | 2,5-(CH₃)₂ | 3-CH₃ | H | 367.10 |
| I'-200 | 2-naphthyl | 3-CH₃ | H | 389.10 |
| I'-201 | 2,5-(OCH₃)₂ | 3-CH₃ | H | 399.10 |
| I'-202 | 3-OCH₃ | 3-CH₃ | H | 369.10 |
| I'-203 | 2,5-(CH₃)₂ | 3-F | H | 371.10 |
| I'-204 | 2-naphthyl | 3-F | H | 393.10 |
| I'-205 | 2,5-(OCH₃)₂ | 3-F | H | 403.10 |
| I'-206 | 3-OCH₃ | 3-F | H | 373.10 |
| I'-207 | 2,5-(CH₃)₂ | 3-Cl | H | 387.10 |
| I'-208 | 2-naphthyl | 3-Cl | H | 409.0 |
| I'-209 | 2,5-(OCH₃)₂ | 3-Cl | H | 419.0 |
| I'-210 | 3-OCH₃ | 3-Cl | H | 389.0 |
| I'-211 | 3,5-Br₂ | 3-CF₃ | H | 550.9 |
| I'-212 | 2-OCH₃, 5-Cl | 3-CF₃ | H | 457.0 |
| I'-213 | 2-OCH₃, 5-F | 3-CF₃ | H | 441.0 |
| I'-214 | 3-CH₃, 4-F | 3-CF₃ | H | 425.0 |
| I'-215 | 3,5-Br₂ | 3-CH₃ | H | 496.9 |
| I'-216 | 2-OCH₃, 5-Cl | 3-CH₃ | H | 403.0 |
| I'-217 | 2-OCH₃, 5-F | 3-CH₃ | H | 387.1 |
| I'-218 | 3-CH₃, 4-F | 3-CH₃ | H | 371.1 |
| I'-219 | 2-OCH₃, 5-Cl | 3-F | H | 407.0 |
| I'-220 | 2-OCH₃, 5-F | 3-F | H | 391.1 |
| I'-221 | 3-CH₃, 4-F | 3-F | H | 375.1 |
| I'-222 | 3,5-Br₂ | 3-Cl | H | 517.2 |
| I'-223 | 2-OCH₃, 5-Cl | 3-Cl, | H | 423.0 |
| I'-224 | 2-OCH₃, 5-F | 3-Cl | H | 407.0 |
| I'-225 | 3-CH₃, 4-F | 3-Cl | H | 391.0 |
| I'-226 | 3-C(O)CH₃ | 3-CH₃ | H | 381.1 |
| I'-227 | 4-OCH₂CH₃ | 3-CH₃ | H | 383.1 |
| I'-228 | 3-Br | 3-CH₃ | H | 419.0 |
| I'-229 | 3,4-F₂ | 3-CH₃ | H | 375.1 |
| I'-230 | 3,4-Cl₂ | 3-CH₃ | H | 407.0 |
| I'-231 | 3-OCH₂CH₃ | 3-CH₃ | H | 383.0 |
| I'-232 | 3-C(O)CH₃ | 3-F | H | 385.1 |
| I'-233. | 4-OCH₂CH₃ | 3-F | H | 387.1 |
| I'-234 | 3-Br | 3-F | H | 422.9 |
| I'-235 | 3,4-F₂ | 3-F | H | 379.1 |
| I'-236 | 3,4-Cl₂ | 3-F | H | 411.0 |
| I'-237 | 3-OCH₂CH₃ | 3-F | H | 387.1 |

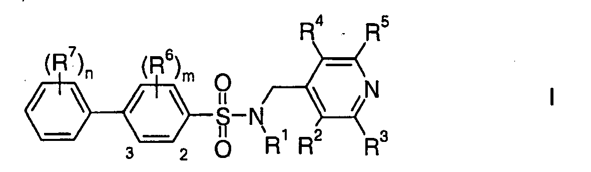

**Table C) - Compounds of the formula I (R¹ = H)**

| No. | (R⁷)ₙ | (R⁶)ₘ | R² | R³ | R⁴ | R⁵ | Phys. data (m.p. [°C]; MS M+H⁺ [m/e] RT [min]); ¹H-NMR [ppm} |
|---|---|---|---|---|---|---|---|
| I-1 | - | - | CF₃ | H | H | H | 155-157 |
| I-2 | - | - | H | OCH₃ | H | H | 105-109 |
| I-3 | 4-Cl | - | H | OCH₃ | H | H | 127-130 |
| I-4 | 4-C(O)CH₃ | - | H | OCH₃ | H | H | 98-100 |
| I-5 | 4-C(CH₃)=NOCH₃ | - | H | OCH₃ | H | H | 350.10 |
| I-6 | 4- C(CH₃)=NOCH₂CH₃ | - | H | OCH₃ | H | H | 364.10 |
| I-7 | - | - | Cl | H | Cl | H | 148-151 |
| I-8 | 4-C(O)CH₃ | - | Cl | H | Cl | H | 132-135 |
| I-9 | - | - | CH₃ | H | H | H | 133-135 |
| I-10 | - | - | OCH₃ | H | H | H | 187-189 |
| I-11 | - | - | H | Cl | H | Cl | 165-167 |
| I-12 | 4-F | - | OCH₃ | H | H | H | 78-82 |
| I-13 | 4-CN | - | OCH₃ | H | H | H | 135-138 |
| I-14 | 4-Cl | - | OCH₃ | H | H | H | 170-174 |
| I-15 | 4-CH₃ | - | OCH₃ | H | H | H | 2,7; 369,15 |
| I-16 | 3,5-Cl₂ | - | OCH₃ | H | H | H | 3,0; 422,95 |
| I-17 | 3,5-(CF₃)₂ | - | OCH₃ | H | H | H | 3,1; 491,05 |
| I-18 | 4-CF₃ | - | OCH₃ | H | H | H | 2,89; 423,05 |
| I-19 | 3-Cl, 4-F | - | OCH₃ | H | H | H | 2,7; 406,9 |
| 1-20 | 4-Br | - | OCH₃ | H | H | H | 182-190 |
| I-21 | 2-CH₃ | - | OCH₃ | H | H | H | 2,61; 369 |
| I-22 | 3,5-F₂ | - | OCH₃ | H | H | H | 2,66; 391,05 |
| I-23 | 2-F | - | OCH₃ | H | H | H | 2,52; 373,05 |
| I-24 | 2,4-Cl₂ | - | OCH₃ | H | H | H | 2,94; 422,95 |
| I-25 | 2,4-F₂ | - | OCH₃ | H | H | H | 2,61; 391,05 |
| I-26 | 3-CH₃-4-Cl | - | OCH₃ | H | H | H | 2,95; 403,05 |
| I-27 | 4-OCH₃ | - | OCH₃ | H | H | H | 2,54; 385,05 |
| I-28 | 3-CH₃ | - | OCH₃ | H | H | H | 2,72; 369,15 |
| I-29 | 2-CH₃-5-Br | - | OCH₃ | H | H | H | 2,85; 463,05 |
| I-30 | 4-OCF₃ | - | OCH₃ | H | H | H | 2,97; 439,05 |
| I-31 | 3,5-(CH₃)₂ | - | OCH₃ | H | H | H | 2,92; 383,15 |
| I-32 | 3,4-(OCH₃)₂ | - | OCH₃ | H | H | H | 2,3; 415,05 |
| I-33 | 3-F | - | OCH₃ | H | H | H | 2,57; 373,05 |
| I-34 | 2-CF₃ | - | OCH₃ | H | H | H | 2,71; 423,05 |
| I-35 | 4-CH₂CH₃ | - | OCH₃ | H | H | H | 2,94; 383,15 |
| I-36 | 2,3-(CH₃)₂ | - | OCH₃ | H | H | H | 2,83; 383,15 |
| I-37 | 2,5-(OCH₃)₂ | - | OCH₃ | H | H | H | 2,54; 415,15 |
| I-38 | 2,5-(CH₃)₂ | - | OCH₃ | H | H | H | 2,86; 383,15 |
| I-39 | 3-OCF₃ | - | OCH₃ | H | H | H | 2,85; 438,9 |
| I-40 | 3-OCF₃ | - | OCH₃ | H | H | H | 2,81; 422,9 |
| I-41 | 3-F, 4-OCH₃ | - | OCH₃ | H | H | H | 2,50; 403,0 |
| I-42 | 3-F, 5-OCH₃ | - | OCH₃ | H | H | H | 2,60; 402,9 |
| I-43 | 3-CH₃, 4-F | - | OCH₃ | H | H | H | 2,77; 387,05 |
| I-44 | 4-OCH₂CH₃ | - | OCH₃ | H | H | H | 2,73; 399,05 |
| I-45 | 3-Br | - | OCH₃ | H | H | H | 2,81; 434,85 |
| I-46 | 3,4-F₂ | - | OCH₃ | H | H | H | 2,65; 390,95 |
| I-47 | 3,4-Cl₂ | - | OCH₃ | H | H | H | 2,98; 422,85 |
| I-48 | 3-OCH₂CH₃ | - | OCH₃ | H | H | H | 2,72; 399,00 |
| I-49 | 2,5-Cl₂ | - | OCH₃ | H | H | H | 2,89; 422,80 |
| I-50 | 2,5-(OCH₃)₂ | - | OCH₃ | H | H | H | 2,60; 414,95 |
| I-51 | 3-OCH₃ | - | OCH₃ | H | H | H | 2,58; 384,95 |
| I-52 | 3,5-Br₂ | - | OCH₃ | H | H | H | 3,16; 512,75 |
| I-53 | 2-OCH₃-5-Cl | - | OCH₃ | H | H | H | 2,82; 418,95 |
| I-54 | 2-F, 4-Cl; 5-CH₃ | - | OCH₃ | H | H | H | 3,05; 420,85 |
| I-55 | 2-CH₃-4-F | - | OCH₃ | H | H | H | 2,75; 386,95 |
| I-56 | 4-C(CH₃)₃ | - | OCH₃ | H | H | H | 3,22; 411,05 |
| I-57 | 3,4-(-OCH₂O-) | - | OCH₃ | H | H | H | 2,48; 398,95 |
| I-58 | 2-F-4-Cl-5-OH | - | OCH₃ | H | H | H | 2,26; 404,85 |
| I-59 | 2-F, 4-Cl | - | OCH₃ | H | H | H | 2,53; 406,90 |
| I-60 | 2-F, 4-CH₃ | - | OCH₃ | H | H | H | 2,73; 387,0 |
| I-61 | 3,4-(-OCH₂CH₂O-) | - | OCH₃ | H | H | H | 2,49; 412,90 |
| I-62 | 2,4-(OCF₃)₂ | - | OCH₃ | H | H | H | 124 |
| I-63 | 4-CN | - | H | OCH₃ | H | H | 120-123 |
| I-64 | 4-F | - | H | OCH₃ | H | H | 123-129 |
| I-65 | 2-OCH₃ | - | H | OCH₃ | H | H | 2,89; 385,05 |
| I-66 | 4-SCH₃ | - | H | OCH₃ | H | H | 3,10; 400,90 |
| I-67 | 4-CH₃ | - | H | OCH₃ | H | H | 3,11; 369,15 |
| I-68 | 3,5-Cl₂ | - | H | OCH₃ | H | H | 3,56; 426,10 |
| I-69 | 3,5-(CF₃)₂ | - | H | OCH₃ | H | H | 3,62; 491,05 |
| I-70 | 4-CF₃ | - | H | OCH₃ | H | H | 3,30; 423,05 |
| I-71 | 3-Cl, 4-F | - | H | OCH₃ | H | H | 3,26; 406,9 |
| I-72 | 4-Br | - | H | OCH₃ | H | H | 134-144 |
| I-73 | 2-CH₃ | - | H | OCH₃ | H | H | 3,02; 369,0 |
| I-74 | 3,5-F₂ | - | H | OCH₃ | H | H | 3,06; 391,05 |
| I-75 | 2-F | - | H | OCH₃ | H | H | 2,90; 373,05 |
| I-76 | 2,4-Cl₂ | - | H | OCH₃ | H | H | 3,40; 423,05 |
| I-77 | 2,4-F₂ | - | H | OCH₃ | H | H | 2,98; 391,05 |
| I-78 | 3-CH₃, 4-Cl | - | H | OCH₃ | H | H | 3,41; 403,05 |
| I-79 | 4-OCH₃ | - | H | OCH₃ | H | H | 2,85; 385,05 |
| I-80 | 2,5-F₂ | - | H | OCH₃ | H | H | 2,96; 391,05, |
| I-81 | 3-CH₃ | - | H | OCH₃ | H | H | 3,11; 369,15 |
| I-82 | 2-OCH₃, 5-Br | - | H | OCH₃ | H | H | 3,24; 462,95 |
| I-83 | 4-OCF₃ | - | H | OCH₃ | H | H | 3,38; 439,05 |
| I-84 | 3,5-(CH₃)₂ | - | H | OCH₃ | H | H | 3,34; 383,15 |
| I-85 | 3,4-(OCH₃)₂ | - | H | OCH₃ | H | H | 2,67; 415,15 |
| I-86 | 3-F | - | H | OCH₃ | H | H | 2,94; 373,05 |
| I-87 | 2-CF₃ | - | H | OCH₃ | H | H | 3,14; 423,05 |
| I-88 | 4-CH₂CH₃ | - | H | OCH₃ | H | H | 3,30; 383,15 |
| I-89 | 2,3-(CH₃)₂ | - | H | OCH₃ | H | H | 3,22; 383,15 |
| I-90 | 2,5-(OCH₃)₂ | - | H | OCH₃ | H | H | 2,87; 415,05 |
| I-91 | 4-N(CH₃)₂ | - | H | OCH₃ | H | H | Öl |
| I-92 | 2,5-(CH₃)₂ | - | H | OCH₃ | H | H | 3,30; 383,15 |
| I-93 | 4-OCH₃ | - | H | OC₂H₅ | H | H | 143 |
| I-94 | 3-OCF₃ | - | H | OCH₃ | H | H | 3,19; 438,90 |
| I-95 | 3-CF₃ | - | H | OCH₃ | H | H | 3,16; 422,90 |
| I-96 | 3-F, 4-OCH₃ | - | H | OCH₃ | H | H | 2,78; 403,00 |
| I-97 | 2-OCH₃, 5-F | - | H | OCH₃ | H | H | 2,91; 403,95 |
| I-98 | 3-CH₃, 4-F | - | H | OCH₃ | H | H | 3,11; 386,95 |
| I-99 | 4-OCH₂CH₃ | - | H | OCH₃ | H | H | 3,05; 399,05 |
| I-10C | 3-Br | - | H | OCH₃ | H | H | 3,18; 434,85 |
| I-101 | 3,4-F₂ | - | H | OCH₃ | H | H | 2,99; 390,95 |
| I-102 | 3,4-Cl₂ | - | H | OCH₃ | H | H | 3,38; 422,85 |
| I-103 | 3-OCH₂CH₃ | - | H | OCH₃ | H | H | 3,12; 399,00 |
| I-104 | 2,5-Cl₂ | - | H | OCH₃ | H | H | 3,27; 422,90 |
| I-105 | 2,4-(OCH₃)₂ | - | H | OCH₃ | H | H | 2,85; 415,05 |
| I-106 | 3-OCH₃ | - | H | OCH₃ | H | H | 2,83; 384,95 |
| I-107 | 3,5-Br₂ | - | H | OCH₃ | H | H | 3,55; 512,85 |
| I-108 | 2-OCH₃, 5-Cl | - | H | OCH₃ | H | H | 3,12; 418,95 |
| I-109 | 2-F, 4-Cl, 5-CH₃ | - | H | OCH₃ | H | H | 3,54; 420,95 |
| I-110 | 2-CH₃, 4-F | - | H | OCH₃ | H | H | 3,18; 387,25 |
| I-111 | 4-C(CH₃)₃ | - | H | OCH₃ | H | H | 3,70; 411,05 |
| I-112 | 3,4-(-OCH₂O-) | - | H | OCH₃ | H | H | 2,77; 398,95 |
| I-113 | 4-SCH₂CH₃ | - | H | OCH₃ | H | H | 3,30; 414,95 |
| I-114 | 2-F, 4-Cl | - | H | OCH₃ | H | H | 2,97; 406,9 |
| I-115 | 2-F, 4-CH₃ | - | H | OCH₃ | H | H | 3,02; 386,90 |
| I-116 | 3,4-(-OCH₂CH₂O-) | - | H | OCH₃ | H | H | 115-119 |
| I-117 | 4-F | - | CF₃ | H | H | H | 130-132 |
| I-118 | 4-CN | - | CF₃ | H | H | H | 83-85 |
| I-119 | 4-Cl | - | CF₃ | H | H | H | 149-151 |
| I-120 | 4-F | - | CH₃ | H | H | H | 135-140 |
| I-121 | 4-Cl | - | CH₃ | H | H | H | 168-172 |
| I-122 | 4-CN | - | CH₃ | H | H | H | 183-186 |
| I-123 | 2,4-Cl₂ | - | CF₃ | H | H | | ¹H-NMR (400 MHz in d₆-DMSO): δ = 8.8 (s,1H), 8.7 (m,1H),8.6 (br.,s,1H), 7.9-7.8 (m,3H), 7.6-7.4 (m,4H), 7.4 (m,1H), 4.3 (s,2H) |
| I-124 | 2,4-Cl₂ | - | H | Cl | H | H | ¹H-NMR (400 MHz in d₆-DMSO): δ = 8.2 (m,1H).7.8 (m,2H), 7.6-7.5 (m,3H), 7.5-7.4 (m,3H), 7.2 (m,1H),7.1 (m,1H),4.2 (s,2H) |
| I-125 | 2-CF₃, 4-CF₃ | - | CH₃ | CH₃ | H | H | 161-162°C; ¹H-NMR (400 MHz in d⁸-THF): δ 8.2 (m,2H), 8.1 (m,1H),7.9 (m,2H), 7.6 (m,1H), 7.5 (m,2H), 7.1 (t.1H).6.9 (m,1H), 4.2 (t,2H), 2.4 (s,3H), 2.2 (s,3H) |
| I-126 | 2,4-Cl₂ | - | CH₃ | CH₃ | H | H | 172-173°C; ¹H-NMR (400 MHz in d⁸-THF): δ = 8.2 (m,1H), 7.9 (m,2H), 7.6 (m,3H), 7.5-7.4 (m,2H), 7.1 (t,1 H), 6.9 (m, 1H), 4.1 (t,2H), 2.4 (s,3H), 2.2 (s,3H) |
| I-127 | 2-CF₃, 4-CF₃ | - | OCH₃ | H | H | H | 124°C |

### Examples of the action against harmful fungi

The fungicidal action of the compounds of the formula I was demonstrated by the following experiments:

The active compounds were formulated separately or together as a stock solution with 0.25% by weight of active compound in acetone or DMSO. 1% by weight of the emulsifier Uniperol^{®} EL (wetting agent having emulsifying and dispersing action based on ethoxylated alkylphenols) was added to this solution and diluted with water to the desired concentration.

### Use example 1 - Activity against early blight of tomatoes caused by Alternaria solani

Leaves of potted plants of the cultivar "Golden Queen" were sprayed to run-off point with an aqueous suspension having the concentration of active compound stated below. The next day, the leaves were infected with an aqueous spore suspension of *Alternaria solani* in a 2% biomalt solution having a density of 0.17 x 10⁶ spores/ml. The plants were then placed in a water-vapor-saturated chamber at temperatures between 20 and 22°C. After 5 days, the disease on the untreated, but infected control plants had developed to such an extent that the infection could be determined visually in %.

In this test, the plants which had been treated with 250 ppm of the compound I-5, 1-6, I-8, I-9, I-10, I-12, I-15, I-16, I-24, I-45, I-56, I-61, I-63, I-64, I-65 or I-68 showed an infection of not more than 20%, whereas the untreated plants were 90% infected.

### Use example 2: Activity against late blight of tomatoes caused by Phytophthora infestans, protective treatment

Leaves of potted tomato plants were sprayed to runoff point with an aqueous suspension having the concentration of active compounds stated below. The next day, the leaves were infected with an aqueous sporangia suspension of *Phytophthora infestans*. The plants were then placed in a water-vapor-saturated chamber at temperatures between 18°C and 20°C. After 6 days, the late blight on the untreated, but infected control plants had developed to such an extent that the infection could be determined visually in %.

In this test, the plants which had been treated with 250 ppm of the compound I-5, I-6, I-8 to I-12, I-15, I-16, I-21, I-23, I-24, I-43, I-45, I-46, I-48, I-53, I-54, I-56, I-57, I-58, I-61, I-63 to I-68, I-78, I-84, I-87 or I-88 showed an infection of not more than 20%, whereas the untreated plants were 90% infected.

### Use example 3: Curative activity against brown rust of wheat caused by Puccinia recondita

Leaves of potted wheat seedlings of the cultivar "Kanzler" were inoculated with a spore suspension of brown rust (*Puccinia recondita*). The pots were then placed into a chamber with high atmospheric humidity (90-95%) at 20-22°C for 24 hours. During this time, the spores germinated and the germ tubes penetrated into the leaf tissue. The next day, the infected plants were sprayed to runoff point with an aqueous suspension having the concentration of active compound stated below. The suspension or emulsion was prepared as described above. After the spray coating had dried on, the test plants were cultivated in a greenhouse at temperatures between 20 and 22°C and at 65 to 70% relative atmospheric humidity for 7 days. The extent of the rust fungus development on the leaves was then determined.

In this test, the plants which had been treated with 250 ppm of the compound I-24, I-46, I-47, I-48, I-58, I-63 to I-65, I-69 or I-87 showed an infection of not more than 20%, whereas the untreated plants were 90% infected.

The action of the compounds of the formula I against harmful pests was demonstrated by the following experiments:

### 1. Cotton aphid (aphisgossypil₎, mixed life stages

The active compounds were formulated in 50:50 acetone / water and 100 ppm Kinetic^{®} surfactant.

Cotton plants at the cotyledon stage were infested prior to treatment by placing a heavily infested leaf from the main aphid colony on top of each cotyledon. The aphids were allowed to transfer overnight and the host leaf was removed. The infested cotyledons were then dipped and agitated in the test solution for 3 seconds and allowed to dry in a fume hood. Test plants were maintained under fluorescent lighting in a 24-hr photoperiod at 25°C and 20-40% relative humidity. Aphid mortality on the treated plants, relative to mortality on untreated check plants, was determined after 5 days.

### 2. Southern Armyworm (spodoptera eridania), 2^{nd}-3^{rd} instar larvae

The active compounds were formulated as a 10.000 ppm solution in a mixture of 35% acetone and water, which was diluted with water, if needed.

Sieva lima bean foliage, expanded to the first true leaves, were dipped and agitated in the test solution for 3 seconds and then allowed to dry in a fume hood. The treated plant was then placed in 25-cm plastic perforated zip enclosure bags, ten 2^{nd}-instar larvae were added, and the bags sealed. After 4 days, observations were made of mortality, plant feeding, and of any interference with larval growth.

### 3. Tobacco Budworm (Heliothis virescens)

Two-leaf cotton plants are utilized for bioassays. Excised plant leaves are dipped into 1:1 acetone / water dilutions of the active compounds. After the leaves have dried, they are individually placed onto water-moistened filter paper on the bottoms of Petri dishes. Each dish is infested with 5 - 7 larvae and covered with a lid. Each treatment dilution is replicated 4 times. Test dishes are held at approximately 27°C and 60% humidity.

Numbers of live and morbid larvae are assessed in each dish at 5 days after treatment application, and percent mortality is calculated.

### 4. Colorado Potato Beetle (Leptinotarsa decemlineata)

Potato plants are utilized for bioassays. Excised plant leaves are dipped into 1:1 acetone/water dilutions of the active compounds. After the leaves have dried, they are individually placed onto water-moistened filter paper on the bottoms of Petri dishes. Each dish is infested with 5 - 7 larvae and covered with a lid. Each treatment dilution is replicated 4 times. Test dishes are held at approximately 27°C and 60% humidity. Numbers of live and morbid larvae are assessed in each dish at 5 days after treatment application, and percent mortality is calculated.

### 5. Green Peach Aphid (Myzus persicae)

The active compounds were formulated in 50:50 acetone:water and 100 ppm Kinetic^{®} surfactant.

Pepper plants in the 2^{nd} leaf-pair stage (variety 'California Wonder') were infested with approximately 40 laboratory-reared aphids by placing infested leaf sections on top of the test plants. The leaf sections were removed after 24 hours. The leaves of the intact plants were dipped into gradient solutions of the test compound and allowed to dry. Test plants were maintained under fluorescent light (24 hour photoperiod) at about 25_{°}C and 20-40% relative humidity. Aphid mortality on the treated plants, relative to mortality on check plants, was determined after 5 days.

### 6. Silverleaf whitefly (bemisia argentifolii)

The active compounds are formulated in 50:50 acetone:water and 100 ppm Kinetic^{®} surfactant.

Selected cotton plants are grown to the cotyledon state (one plant per pot). The cotyledons are dipped into the test solution to provide complete coverage of the foliage and placed in a well-vented area to dry. Each pot with treated seedling is placed in a plastic cup and 10 to 12 whitefly adults (approximately 3-5 day old) are introduced. The insects are colleted using an aspirator and an 0.6 cm, non-toxic Tygon tubing connected to a barrier pipette tip. The tip, containing the collected insects, is then gently inserted into the soil containing the treated plant, allowing insects to crawl out of the tip to reach the foliage for feeding. The cups are covered with a re-usable screened lid (150 micron mesh polyester screen PeCap from Tetko Inc). Test plants are maintained in the holding room at about 25_{°}C and 20-40% relative humidity for 3 days avoiding direct exposure to the fluorescent light (24 hour photoperiod) to prevent trapping of heat inside the cup. Mortality is assessed 3 days after treatment of the plants.

### 7. 2-spotted spider mite (tetranychus urticae, OP-resistant strain)

The active compounds are formulated in 50:50 acetone:water and 100 ppm Kinetic^{®} surfactant.

Sieva lima bean plants with primary leaves expanded to 7-12 cm are infested by placing on each a small piece from an infested leaf (with about 100 mites) taken from the main colony. This is done at about 2 hours before treatment to allow the mites to move over to the test plant to lay eggs. The piece of leaf used to transfer the mites is removed. The newly-infested plants are dipped in the test solution and allowed to dry. The test plants are kept under fluorescent light (24 hour photoperiod) at about 25°C and 20 - 40% relative humidity. After 5 days, one leaf is removed and mortality counts are made.

### 8. Activity against cowpea aphid (aphis craccivora)

The active compounds were formulated in 50:50 acetone / water. Potted cowpea plants colonized with 100 - 150 aphids of various stages were sprayed after the pest population has been recorded. Population reduction was recorded after 24, 72, and 120 hours.

### 9. Activity against diamond back moth (plutella xylostella)

The active compounds were formulated in 50:50 acetone / water and 0.1 % (vol/vol) Alkamuls^{®} EL 620 surfactant. A 6 cm leaf disk of cabbage leaves was dipped in the test solution for 3 seconds and allowed to air dry in a Petri plate lined with moist filter paper. The leaf disk was inoculated with 10 third instar larvae and kept at 25-27°C and 50-60% humidity for 3 days. Mortality was assessed after 72 hours of treatment.

### 10. Yellowfever mosquitos (aedes aegypti)

The test compound (1 Vol% in acetone) is applied to water in glass dishes containing 4th instar aedes aegypti. The test dishes are maintained at about 25°C and observed daily for mortality. Each test is replicated in 3 test dishes.

### 11. Eastern subterranean termites (Reticulitérmes flávipes)

Toxicant treatments (1.0% test compound w/w) are applied to 4.25 cm (diam.) filter papers in acetone solution. Treatment levels (% test compound) are calculated on basis of a mean weight per filter paper of 106.5 mg. Treatment solutions are adjusted to provide the quantity of toxicant (mg) required per paper in 213 ml of acetone. Acetone only is applied for untreated controls. Treated papers are vented to evaporate the acetone, moistened with 0.25 ml water, and enclosed in 50x9 mm Petri dishes with tight-fit lids.

Termite bioassays are conducted in 100x15 mm Petri dishes with 10 g fine sand spread in a thin layer over the bottom of each dish. An additional 2.5 g sand is piled against the side of each dish. The sand is moistened with 2.8 ml water applied to the piled sand. Water is added to dishes as needed over the course of the bioassays to maintain high moisture content. Bioassays are done with one treated filter (inside enclosure) and 30 termite workers per test dish. Each treatment level is replicated in 2 test dishes. Test dishes are maintained at about 25°C and 85% humidity for 12 days and observed daily for mortality.

### 12. Orchid thrips (dichromothrips corbetti)

Dichromothrips corbetti adults used for bioassay are obtained from a colony maintained continuously under laboratory conditions. For testing purposes, the test compound is diluted to a concentration of 500 ppm (wt compound: vol diluent) in a 1:1 mixture of acetone : water, plus 0.01% Kinetic surfactant.

Thrips potency of each compound is evaluated by using a floral-immersion technique. Plastic petri dishes are used as test arenas. All petals of individual, intact orchid flowers are dipped into treatment solution for approximately 3 seconds and allowed to dry for 2 hours. Treated flowers are placed into individual petri dishes along with 10 - 15 adult thrips. The petri dishes are then covered with lids. All test arenas are held under continuous light and a temperature of about 28°C for duration of the assay. After 4 days, the numbers of live thrips are counted on each flower, and along inner walls of each petri dish. The level of thrips mortality is extrapolated from pre-treatment thrips numbers.

### 13. Activity against Argentine ant, harvester ant, acrobat ant, carpenter ant, fire ant, house fly, stable fly, flesh fly, yellowfever mosquito, house mosquito, malaria mosquito, German cockroach, cat flea, and brown dog tick via glass contact

Glass vials (20 ml scintillation vials) are treated with 0.5 ml of a solution of active ingredient in acetone. Each vial is rolled uncapped for ca. 10 minutes to allow the active ingredient to completely coat the vial and to allow for full drying of the acetone. Insects or ticks are placed into each vial. The vials are kept at 22°C and are observed for treatment effects at various time intervals.

### 14. Activity against Boll weevil (Anthonomus grandis)

The active compounds were formulated in 1:3 dimethylsulfoxide / water. 10 to 15 eggs were placed into microtiterplates filled with 2% agar-agar in water and 300 ppm formaline. The eggs were sprayed with 20 µl of the test solution, the plates were sealed with pierced foils and kept at 24-26°C and 75-85% humidity with a day/night cycle for 3 to 5 days. Mortality was assessed on the basis of the remaining unhatched eggs or larvae on the agar surface and/or quantity and depth of the digging channels caused by the hatched larvae. Tests were replicated 2 times.

In this test, 2500 ppm of the following compounds caused at least 75% mortality compared to 0% mortality of untreated controls:
compond A-187 of Table 36, compound A-99 of Table 36, compound A-363 of Table 36, compound A-396 of Table 25, compound A-484 of Table 25, compound A-670 of Table 25, compound A-228 of Table 1, compound A-403 of Table 1, compound A-516 of Table 3, compound A-484 of Table 3, compound A-429 of Table 36, compound A-396 of Table 36, compound A-11 of Table 36, compound A-626 of Table 36, compound A-55 of Table 36, compound A-484 of Table 36.

### 15. Activity against Mediterranean fruitfly (Ceratitis capitata)

The active compounds were formulated in 1:3 dimethylsulfoxide / water. 50 to 80 eggs were placed into microtiterplates filled with 0.5% agar-agar and 14 % diet in water. The eggs were sprayed with 5µl of the test solution, the plates were sealed with pierced foils and kept at 27-29°C and 75-85% humidity under fluorescent light for 6 days. Mortality was assessed on the basis of the agility of the hatched larvae. Tests were replicated 2 times.

In this test compound A-628 of Table 1 and compound A-484 of Table 25 at 2500 ppm showed over 75% mortality compared to 0% mortality of untreated controls.

### 16. Activity against Tobacco budworm (Heliothis virescens)

The active compounds were formulated in 1:3 dimethylsulfoxide / water. 15 to 25 eggs were placed into microtiterplates filled with diet. The eggs were sprayed with 10 µl of the test solution, the plates were sealed with pierced foils and kept at 27-29°C and 75-85% humidity under fluorescent light for 6 days. Mortality was assessed on the basis of the agility and of comparative feeding of the hatched larvae. Tests were replicated 2 times.

In this test, 2500 ppm of the following compounds caused at least 75% mortality compared to 0% mortality of untreated controls:
compond A-187 of Table 36; compound A-99 of Table 36, compound A-396 of Table 25; compound A-484 of Table 25; compound A-670 of Table 25, compound A-403 of Table 1, compound A-516 of Table 3, compound A-429 of Table 36, compound A-396 of Table 36, compound A-11 of Table 36, compound A-626 of Table 36.

### 17. Activity against Vetch aphid (Megoura viciae)

The active compounds were formulated in 1:3 dimethylsulfoxide / water. Bean leaf disks were placed into microtiterplates filled with 0.8% agar-agar and 2.5 ppm OPUS^{®}. The leaf disks were sprayed with 2.5 µl of the test solution and 5 to 8 adult aphids were placed into the microtiterplates which were then closed and kept at 22-24°C and 35-45% under fluorescent light for 6 days. Mortality was assessed on the basis of vital, reproduced aphids. Tests were replicated 2 times.

### 18. Activity against Wheat aphid (Rhopalosiphum padi)

The active compounds were formulated in 1:3 dimethylsulfoxide / water. Barlay leaf disk were placed into microtiterplates filled with 0.8% agar-agar and 2.5 ppm OPUS^{®}. The leaf disks were sprayed with 2.5 µl of the test solution and 3 to 8 adult aphids were placed into the microtiterplates which were then closed and kept at 22-24°C and 35-45% humidity under fluorescent light for 5 days. Mortality was assessed on the basis of vital aphids. Tests were replicated 2 times.

## Claims

1. Biphenylsulfonamides of the formula I 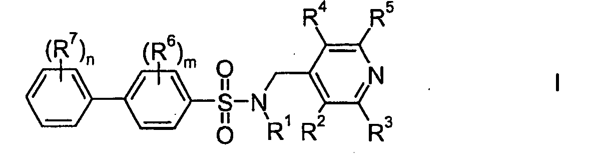 where
R¹ is hydrogen, C₁-C₄-alkyl, C₁-C₄-alkoxy, C₂-C₄-alkenyl, C₂-C₄-alkynyl or benzyl;
R2, R³, R⁴, R⁵ independently of one another are hydrogen, halogen, C₁-C₄-alkyl, halomethyl, C₁-C₄-alkoxy, halomethoxy, C₁-C₄-alkylthio, C₁-C₄-alkylamino or di(C₁-C₄-alkyl)amino;
R⁶, R⁷ independently of one another are hydrogen, hydroxy, cyano, nitro, amino, halogen, C₁-C₈-alkyl, C₁-C₆-haloalkyl, C₁-C₈-alkoxy, C₁-C₈-haloalkoxy, C₁-C₆-alkylthio, (C₁-C₄-alkyl)carbonyl, (C₁-C₄-alkoxy)carbonyl, -C(R⁸)=NOR⁹, (C₁-C₄-alkyl)amino, di(C₁-C₄-alkyl)amino, (C₁-C₄-alkyl)aminocarbonyl, di(C₁-C₄-alkyl)aminocarbonyl, phenyl or phenoxy, where the phenyl ring in the last two mentioned radicals may carry 1, 2 or 3 groups R¹⁰,
or
two radicals R⁶ or two radicals R⁷, together with two adjacent ring members of the phenyl ring to which they are attached, may form an annellated cyclopentyl, cyclohexyl or phenyl ring, whereas these annellated rings may carry themselves one or two radicals R¹⁰;
m zero, 1, 2, 3 or 4;
n zero, 1, 2, 3, 4 or 5;
with the proviso that m and n are not simultaneously zero if R² to R⁵ are all hydrogen;
R⁸ C₁-C₄-alkyl,
R⁹ C₁-C₈-alkyl, C₁-C₄-haloalkyl, C₂-C₄-alkenyl, C₂-C₄-haloalkenyl, C₂-C₄-alkinyl, C₂-C₄-haloalkinyl or benzyl;
R¹⁰ cyano, nitro, halogen, C₁-C₄-alkyl, halomethyl, C₁-C₄-alkoxy or halomethoxy;
and the N-oxides and the agriculturally acceptable salts and the veterinarily acceptable salts of the compounds I.

2. Biphenylsulfonamides of the formula I as defined in claim 1, where R¹ is hydrogen.

3. Biphenylsulfonamides of the formula I as defined in claim 1, where R², R³, R⁴ and R⁵ independently of one another, are hydrogen, methyl, ethyl, fluorine, chlorine, trifluoromethyl, methoxy, ethoxy, difluoromethoxy, trifluoromethoxy, methylthio or ethylthio.

4. Biphenylsulfonamides of the formula I as defined in claim 1, where R² and R⁵ are hydrogen.

5. Biphenylsulfonamides of the formula I as defined in claim 1, where R⁴ and R⁵ are hydrogen.

6. Biphenylsulfonamides of the formula I as defined in claim 1, where R², R³, R⁴ and R⁵ are hydrogen.

7. Biphenylsulfonamides of the formula I as defined in claim 1, where m is zero or one.

8. Biphenylsulfonamides of the formula I as defined in claim 1, where R⁶ is halogen, C₁-C₄-alkyl, C₁-C₄-haloalkyl, C₁-C₄-alkoxy or C₁-C₄-haloalkoxy.

9. Biphenylsulfonamides of the formula I as defined in claim 1, where n is zero, 1 or 2 and R⁷ is halogen, cyano, nitro, C₁-C₄-alkyl, C₁-C₆-haloalkyl, acetyl, C(CH₃)=NOCH₃, C₁-C₈-alkoxy, C₁-C₈-haloalkoxy or C₁-C₆-alkylthio.

10. Biphenylsulfonamides of the formula I as defined in claim 1, where n is 2, 3, 4 or 5.

11. A process for the preparation of biphenylsulfonamides of the formula I according to claims 1 to 10, by reacting pyridine derivatives of the formula II 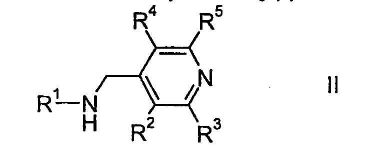 in which R¹ to R⁵ are as defined in claim 1,
under basic conditions with sulfonyl chloride of the formula III 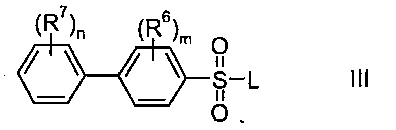 in which the variables are as defined in claim 1 and L is hydroxy or halogen.

12. A process for the preparation of biphenylsulfonamides of the formula I according to claims 1 to 10, by reacting pyridine derivatives of the formula II 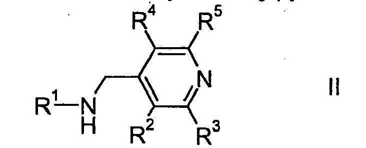 in which R¹ to R⁵ are as defined in claim 1,
with 4-halogenphenylsulfonyl halogenides of the formula IV 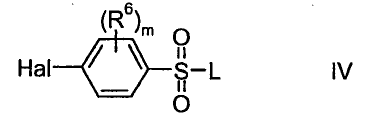 in which the variables are as defined in claim 1 and L is hydroxy or halogen,
to yield pyridine derivatives of the formula V 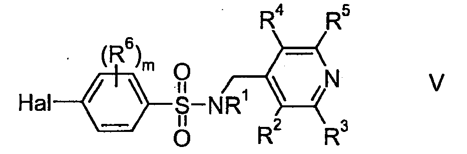 which themselves are reacted with boronic acid derivatives of the formula VI 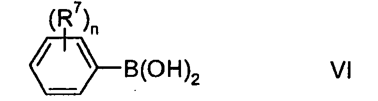 in the presence of a platinum metal catalyst.

13. A composition suitable for controlling harmful fungi, which composition comprises a solid or liquid carrier and at least one biphenylsulfonamide of the formula I or an N-oxide or an agriculturally acceptable salt thereof, according to claim 1.

14. A process for the treatment of phytopathogenic harmful fungi, which process comprises treating the fungi or the materials, plants, the soil or seeds to be protected against fungal attack, with an effective amount of a biphenylsulfonamide compound of the formula I or an N-oxide or an agriculturally acceptable salt thereof, according to claim 1.

15. The non-therapeutic use of biphenylsulfonamides of the formula I, their N-oxides, their agriculturally acceptable salts and their veterinarily acceptable salts, according to claim 1, and of compositions comprising compounds of formula I, for combating harmful arthropodes.

16. A non-therapeutic method for combating arthropodal pests, which comprises contacting said pests, their habitat, breeding ground, food supply, plant, seed, soil, area, material or environment in which the arthropodal pests are growing or may grow, or the materials, plants, seeds, soils, surfaces or spaces to be protected from an attack of or infestation by said pests, with a pesticidally effective amount of at least one biphenylsulfonamide of the formula I, an N-oxide, an agriculturally acceptable salt or a veterinarily acceptable salts thereof, according to claim 1, or with a composition comprising at least one biphenylsulfonamide of the formula I, an N-oxide, an agriculturally acceptable salt or a veterinarily acceptable salt thereof.

17. The method as claimed in claim 16, wherein the pests are insects.

18. The method as claimed in claim 16, wherein the pests are arachnids.

19. A method for protecting crops from attack or infestation by arthropodal pests, the method comprising contacting a crop with a pesticidally effective amount of at least one biphenylsulfonamide of the formula I or an N-oxide or an agriculturally acceptable salt thereof, according to claim 1.

20. A method for protecting seed from infestation by arthropodal pests and of the seedlings' roots and shoots from infestation by arthropodal pests, the method comprising contacting the seed or of the seedlings' roots and shoots with a pesticidally effective amount of at least one biphenylsulfonamide of the formula I, or an N-oxide or an agriculturally acceptable salt thereof, according to claim 1.

21. A method for protecting non-living materials from attack or infestation by arthropodal pests, the method comprising contacting the non-living material with a pesticidally effective amount of at least one biphenylsulfonamide of the formula I, or an N-oxide or an agriculturally acceptable salt thereof, according to claim 1.

22. Seed comprising a biphenylsulfonamide of the formula I, or an N-oxide or an agriculturally acceptable salt thereof, as defined in claim 1, in an amount of from 0.1 g to 10 kg per 100 kg of seed.

## Patentansprüche

1. Biphenylsulfonamide der Formel I 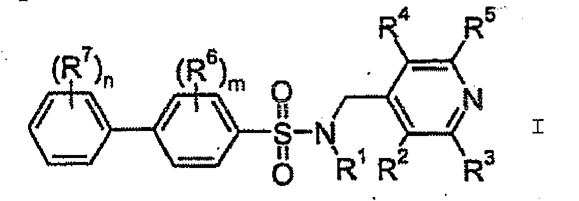 wobei
R¹ für Wasserstoff, C₁-C₄-Alkyl, C₁-C₄-Alkoxy, C₂-C₄-Alkenyl, C₂-C₄-Alkinyl oder Benzyl steht;
R², R³, R⁴, R⁵ unabhängig voneinander für Wasserstoff, Halogen, C₁-C₄-Alkyl, Halogenmethyl, C₁-C₄-Alkoxy, Halogenmethoxy, C₁-C₄-Alkylthio, C₁-C₄-Alkylamino oder Di (C₁-C₄-alkyl) amino stehen;
R⁶ R⁷ unabhängig voneinander für Wasserstoff, Hydroxy, Cyano, Nitro, Amino, Halogen, C₁-C₈-Alkyl, C₁-C₆-Halogenalkyl, C₁-C₈-Alkoxy, C₁-C₈-Halogenalkoxy, C₁-C₆-Alkylthio, (C₁-C₄-Alkyl) carbonyl, (C₁-C₄-Alkoxy) carbonyl, C(R⁸)=NOR⁹, (C₁-C₄-Alkyl) amino, Di(C₁-C₄-alkyl)amino, (C₁-C₄-Alkyl) aminocarbonyl, Di(C₁-C₄-alkyl)aminocarbonyl, Phenyl oder Phenoxy stehen, wobei der Phenylring in den beiden zuletzt erwähnten Resten 1, 2 oder 3 Gruppen R¹⁰ tragen kann,
oder
zwei Reste R⁶ oder zwei Reste R⁷ können zusammen mit den beiden benachbarten Ringgliedern des Phenylrings, an den sie gebunden sind, einen kondensierten Cyclopentyl-, Cyclohexyl- oder Phenylring bilden, wobei diese kondensierten Ringe selbst einen oder zwei Reste R¹⁰, tragen können;
m für null, 1, 2, 3 oder 4 steht;
n für null, 1, 2, 3, 4 oder 5 steht;
mit der Maßgabe, daß m und n nicht gleichzeitig für null stehen, wenn die Reste R² bis R⁵ alle für Wasserstoff stehen;
R⁸ für C₁-C₄-Alkyl steht;
R⁹ für C₁-C₈-Alkyl , C₁-C₄-Halogenalkyl , C₂-C₄-Alkenyl, C₂-C₄-Halogenalkenyl, C₂-C₄-Alkinyl, C₂-C₄-Halogenalkinyl oder Benzyl steht;
R¹⁰ für Cyano, Nitro, Halogen, C₁-C₄-Alkyl, Halogenmethyl , C₁-C₄-Alkoxy oder Halogenmethoxy steht;
und die N-Oxide und die landwirtschaftlich unbedenklichen Salze und die veterinärmedizinisch unbedenklichen Salze der Verbindungen I.

2. Biphenylsulfonamide der Formel I nach Anspruch 1, wobei R¹ für Wasserstoff steht.

3. Biphenylsulfonamide der Formel I nach Anspruch 1, wobei R², R³, R⁴ und R⁵ unabhängig voneinander für Wasserstoff, Methyl, Ethyl, Fluor, Chlor, Trifluormethyl, Methoxy, Ethoxy, Difluormethoxy, Trifluormethoxy, Methylthio oder Ethylthio stehen.

4. Biphenylsulfonamide der Formel I nach Anspruch 1, wobei wobei R² und R⁵ für Wasserstoff stehen.

5. Biphenylsulfonamide der Formel I nach Anspruch 1, wobei R⁴ und R⁵ für Wasserstoff stehen.

6. Biphenylsulfonamide der Formel I nach Anspruch 1, wobei R², R³ R⁴ und R⁵ für Wasserstoff stehen.

7. Biphenylsulfonamide der Formel I nach Anspruch 1, wobei m für null oder eins steht:

8. Biphenylsulfonamide der Formel I nach Anspruch 1, wobei R⁶ für Halogen, C₁-C₄-Alkyl, C₁-C₄-Halogenalkyl, C₁-C₄-Alkoxy oder C₁-C₄-Halogenalkoxy steht.

9. Biphenylsulfonamide der Formel I nach Anspruch 1, wobei n für null, 1 oder 2 steht und R⁷ für Halogen, Cyano, Nitro, C₁-C₄-Alkyl, C₁-C₆-Halogenalkyl, Acetyl, C(CH₃)=NOCH₃, C₁-C₈-Alkoxy, C₁-C₈-Halogenalkoxy oder C₁-C₆-Alkylthio steht.

10. Biphenylsulfonamide der Formel I nach Anspruch 1, wobei n für 2, 3, 4 oder 5 steht.

11. Verfahren zur Herstellung von Biphenylsulfonamiden der Formel I nach einem der Ansprüche 1 bis 10, durch Umsetzung von Pyridinderivaten der Formel II 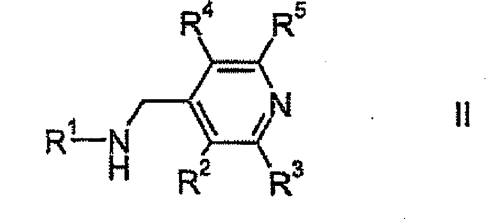 in welcher R¹ bis R⁵ wie in Anspruch 1 definiert sind,
mit Sulfonylchloriden der Formel III 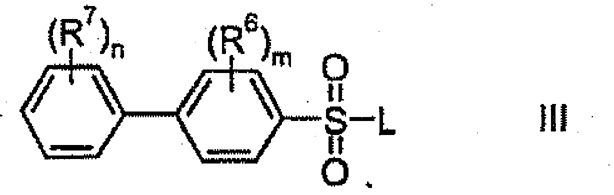 in welcher die variablen wie in Anspruch 1 definiert sind und L für Hydroxy oder Halogen steht,
unter basischen Bedingungen.

12. Verfahren zur Herstellung von Biphenylsulfonamiden der Formel I nach einem der Ansprüche 1 bis 10, durch Umsetzung von Pyridinderivaten der Formel II 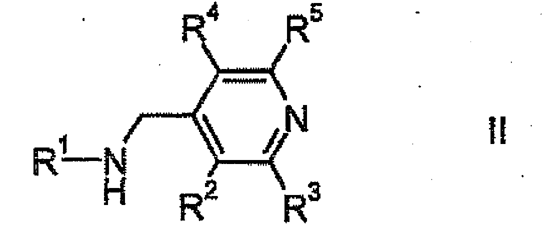 in welcher R¹ bis R⁵ wie in Anspruch 1 definiert sind,
mit 4-Halogenphenylsulfonylhalogeniden der Formel IV 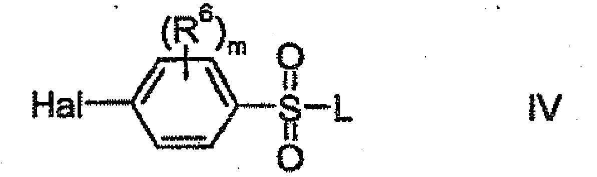 in welcher die Variablen wie in Anspruch definiert sind und L für Hydroxy oder Halogen steht,
zu Pyridinderivaten der Formel V 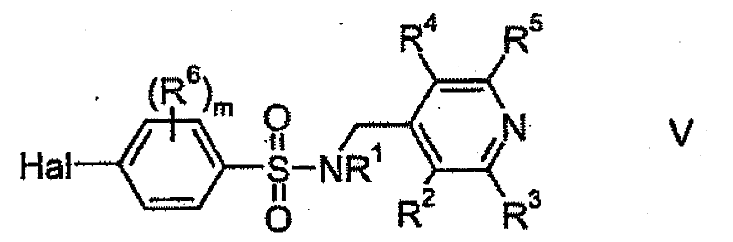 welche ihrerseits mit Boronsäurederivaten der Formel VI 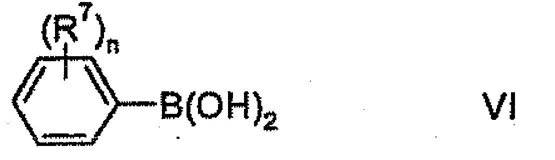 in Gegenwart eines Platinmetall-Katalysators umgesetzt werden.

13. Zur Bekämpfung von Schadpilzen geeignetes Mittel, enthaltend einen festen oder flüssigen Träger und wenigstens ein Biphenylsulfonamid der Formel I oder ein N-Oxid oder ein landwirtschaftlich unbedenkliches Salz davon nach Anspruch 1.

14. Verfahren zur Bekämpfung von pflanzenpathogenen Schadpilzen, **dadurch gekennzeichnet, daß** man die Pilze oder die vor Pilzbefall zu schützenden Materialien, Pflanzen, den Boden oder Saatgüter mit einer wirksamen Menge einer Biphenylsulfonamidverbindung der Formel I oder eines N-Oxids oder eines landwirtschaftlich unbedenklichen Salz davon nach Anspruch 1 behandelt.

15. Nicht-therapeutische Verwendung von Biphenylsulfonamiden der Formel I, ihrer N-Oxide, ihrer landwirtschaftlich unbedenklichen Salze und ihrer veterinärmedizinisch unbedenklichen Salze nach Anspruch 1 und von Verbindungen der Formel I enthaltenden Zusammensetzungen zur Bekämpfung schädlicher Arthropoden.

16. Nicht-therapeutisches Verfahren zur Bekämpfung von Arthropodenschädlingen, bei dem man diese Schädlinge, ihren Lebensraum, ihren Fortpflanzungsraum, ihre Nahrungsvorräte, Pflanzen, Saatgüter, den Boden, Flächen, Materialien oder die Umgebung, in der die Arthropodenschädlinge wachsen oder wachsen können, oder die gegen einen Angriff oder einen Befall durch diese Schädlinge zu schützenden Materialien, Pflanzen, Saatgüter, Böden, Oberflächen oder Räume mit einer pestizidwirksamen Menge wenigstens eines Biphenylsulfonamids der Formel I, eines N-Oxids, eines landwirtschaftlich unbedenklichen Salzes oder eines veterinärmedizinisch unbedenklichen Salzes davon nach Anspruch 1 oder einer Zusammensetzung, die wenigstens ein Biphenylsulfonamid der Formel I, ein N-Oxid, ein landwirtschaftlich unbedenkliches Salz oder ein veterinärmedizinisch unbedenkliches Salz davon enthält, in Kontakt bringt.

17. Verfahren nach Anspruch 16, wobei es sich bei den Schädlingen um Insekten handelt.

18. Verfahren nach Anspruch 16, wobei es sich bei den Schädlingen um Spinnentiere handelt.

19. Verfahren zum Schützen von Pflanzen gegen Angriff oder Befall durch arthropode Schädlinge, wobei man bei diesem Verfahren eine Pflanze mit einer pestizidwirksamen Menge wenigstens eines Biphenylsulfonamids der Formel I oder eines N-Oxids oder eines landwirtschaftlich unbedenklichen Salzes davon nach Anspruch 1 in Kontakt bringt.

20. Verfahren zum Schützen von Saatgut gegen einen Befall durch arthropode Schädlinge und zum Schutz der Wurzeln und Triebe von Setzlingen gegen Befall durch arthropode Schädlinge, wobei man bei diesem Verfahren das Saatgut oder die Wurzeln und Triebe der Setzlinge mit einer pestizidwirksamen Menge wenigstens eines Biphenylsulfonamids der Formel I oder eines N-Oxids oder eines landwirtschaftlich unbedenklichen Salzes davon nach Anspruch 1 in Kontakt bringt.

21. Verfahren zum Schutz von nicht lebenden Materialien gegen Angriff oder Befall durch arthropode Schädlinge, wobei man bei diesem Verfahren das nicht lebende Material mit einer pestizidwirksamen Menge wenigstens eines Biphenylsulfonamids der Formel I oder eines N-Oxids oder eines landwirtschaftlich unbedenklichen Salzes davon nach Anspruch 1 in Kontakt bringt.

22. Saatgut, enthaltend ein Biphenylsulfonamid der Formel I oder ein N-Oxid oder ein landwirtschaftlich unbedenkliches Salz davon nach Anspruch 1 in einer Menge von 0,1 g bis 10 kg pro 100 kg Saatgut.

## Revendications

1. Biphénylsulfonamides de formule I 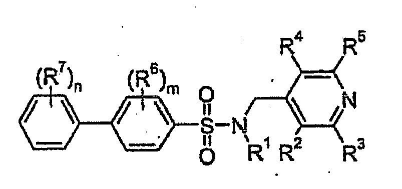 dans laquelle
R¹ est hydrogène, C₁-C₄-alkyle, C₁-C₄-alcoxy, C₂-C₄-alcényle, C₂-C₄-alcynyle ou benzyle ;
R², R³ R⁴ R⁵ indépendamment les uns des autres, sont hydrogène, halogène, C₁-C₄-alkyle, halogénométhyle, C₁-C₄-alcoxy, halogénométhoxy, C₁-C₄-alkylthio, C₁-C₄-alkylamino ou di(C₁-C₄-alkyl)amino ;
R⁶, R⁷, indépendamment l'un de l'autre, sont hydrogène, hydroxy, cyano, nitro, amino, halogène, C₁-C₈-alkyle, C₁-C₆-halogénoalkyle, C₁-C₈-alcoxy, C₁-C₈-halogénoalcoxy, C₁-C₆-alkylthio, (C₁-C₄-alkyl) carbonyle, (C₁-C₄-alcoxy) carbonyle, -C (R⁸) =NOR⁹, (C₁-C₄-alkyl)amino, di (C₁-C₄-alkyl) amino, (C₁-C₄-alkyl)aminocarbonyle, di (C₁-C₄-alkyl)aminocarbonyle, phényle ou phénoxy, où le cycle phényle dans les deux derniers radicaux mentionnés peut porter 1, 2 ou 3 groupements R¹⁰, ou
deux radicaux R⁶ ou deux radicaux R⁷, conjointement avec deux membres du cycle adjacents du cycle phényle auquel ils sont liés, peuvent former un cycle cyclopentyle, cyclohexyle ou phényle condensé, alors que ces cycles condensés peuvent eux-mêmes porter un ou deux radicaux R¹⁰ ;
m zéro, 1, 2, 3 ou 4 ;
n zéro, 1, 2, 3, 4 ou 5 ;
à condition que m et n ne soient pas simultanément zéro si R² à R⁵ sont tous hydrogène ;
R⁸ C₁-C₄-alkyle,
R⁹ C₁-C₈-alkyle, C₁-C₄-halogénoalkyle, C₂-C₄-alcényle, C₂-C₄-halogénoalcényle, C₂-C₄-alcynyle, C₂-C₄-halogénoalcynyle ou benzyle ;
R¹⁰ cyano, nitro, halogène, C₁-C₄-alkyle, halogénométhyle, C₁-C₄-alcoxy ou halogénométhoxy ;
et les N-oxydes et les sels acceptables en agriculture et les sels acceptables du point de vue vétérinaire des composés I.

2. Biphénylsulfonamides de formule I tels que définis dans la revendication 1, **caractérisés en ce que** R¹ est hydrogène.

3. Biphénylsulfonamides de formule I tels que définis dans la revendication 1, **caractérisés en ce que** R², R³, R⁴ et R⁵ indépendamment les uns des autres sont hydrogène, méthyle, éthyle, fluor, chlore, trifluorométhyle, méthoxy, éthoxy, difluorométhoxy, trifluorométhoxy, méthylthio ou éthylthio.

4. Biphénylsulfonamides de formule I tels que définis dans la revendication 1, **caractérisés en ce que** R² et R⁵ sont hydrogène.

5. Biphénylsulfonamides de formule I tels que définis dans la revendication 1, **caractérisés en ce que** R⁴ et R⁵ sont hydrogène.

6. Biphénylsulfonamides de formule I tels que définis dans la revendication 1, **caractérisés en ce que** R², R³ , R⁴ et R⁵ sont hydrogène.

7. Biphénylsulfonamides de formule I tels que définis dans la revendication 1, **caractérisés en ce que** m est zéro ou un.

8. Biphénylsulfonamides de formule I tels que définis dans la revendication 1, **caractérisés en ce que** R⁶ est halogène, C₁-C₄-alkyle, C₁-C₄-halogénoalkyle, C₁-C₄-alcoxy ou C₁-C₄-halogénoalcoxy.

9. Biphénylsulfonamides de formule I tels que définis dans la revendication 1, **caractérisés en ce que** n est zéro, 1 ou 2 et R⁷ est halogène, cyano, nitro, C₁-C₄-alkyle, C₁-C₆-halogénoalkyle, acétyle, C (CH₃) =NOCH₃, C₁-C₈-alcoxy, C₁-C₈-halogénoalcoxy ou C₁-C₆-alkylthio.

10. Biphénylsulfonamides de formule I tels que définis dans la revendication 1, **caractérisés en ce que** n est 2, 3, 4 ou 5.

11. Procédé de préparation de biphénylsulfonamides de formule I selon les revendications 1 à 10, en faisant réagir des dérivés de pyridine de formule II 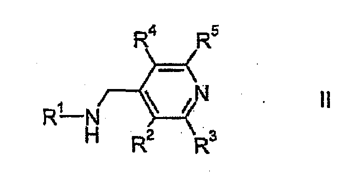 dans laquelle R¹ à R⁵ sont tels que définis dans la revendication 1,
dans des conditions basiques avec du chlorure de sulfonyle de formule III 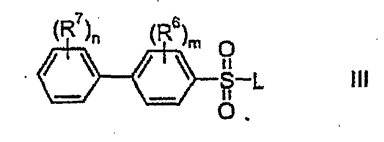 dans laquelle les variables sont telles que définies dans la revendication 1 et L est hydroxy ou halogène.

12. Procédé de préparation de biphénylsulfonamides de formule I selon les revendications 1 à 10, par la réaction de dérivés de pyridine de formule II 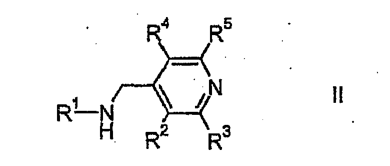 dans laquelle R¹ à R⁵ sont tels que définis dans la revendication 1,
avec des halogénures de 4-halogénophénylsulfonyle de formule IV 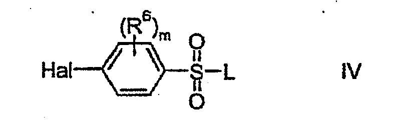 dans laquelle les variables sont telles que définies dans la revendication 1 et L est hydroxy ou halogène, pour donner les dérivés de pyridine de formule V 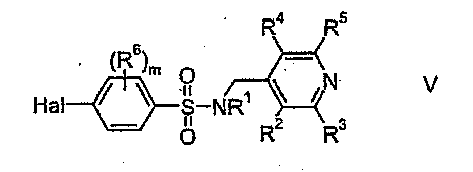 qui eux-mêmes sont mis en réaction avec des dérivés d'acide boronique de formule VI 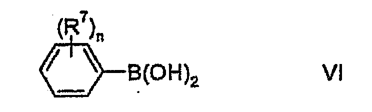 en présence d'un catalyseur de platine métallique.

13. Composition convenable pour combattre les champignons nuisibles, ladite composition comprenant un support solide ou liquide et au moins un biphénylsulfonamide de formule I ou un N-oxyde ou un sel acceptable en agriculture de celui-ci, selon la revendication 1.

14. Procédé de traitement de champignons nuisibles phytopathogènes, ledit procédé comprenant le traitement des champignons ou des matériaux, des plantes, du sol ou des semences à protéger contre une attaque par des champignons, avec une quantité efficace d'un composé biphénylsulfonamide de formule I ou d'un N-oxyde ou d'un sel acceptable en agriculture de celui-ci, selon la revendication 1.

15. Utilisation non thérapeutique des biphénylsulfonamides de formule I, leurs N-oxydes, leurs sels acceptables en agriculture et leurs sels acceptables du point de vue vétérinaire, selon la revendication 1, et de compositions comprenant des composés de formule I, pour combattre les arthropodes nuisibles.

16. Méthode non thérapeutique pour combattre les arthropodes nuisibles, qui comprend la mise en contact desdits arthropodes nuisibles, leur habitat, lieu de reproduction, source d'alimentation, d'un ou une plante, semence, sol, surface, matériau ou environnement dans lequel les arthropodes nuisibles se développent ou peuvent se développer, ou des matériaux, plantes, semences, sols, surfaces ou espaces à protéger contre une attaque ou une infestation par lesdits arthropodes nuisibles, avec une quantité efficace du point de vue pesticide d'au moins un biphénylsulfonamide de formule I, un N-oxyde, un sel acceptable en agriculture ou des sels acceptables du point de vue vétérinaire de celui-ci, selon la revendication 1, ou avec une composition comprenant au moins un biphénylsulfonamide de formule I, un N-oxyde, un sel acceptable en agriculture ou un sel acceptable du point de vue vétérinaire de celui-ci.

17. Méthode selon la revendication 16, **caractérisée en ce que** les espèces nuisibles sont les insectes.

18. Méthode selon la revendication 16, **caractérisée en ce que** les espèces nuisibles sont les arachnides.

19. Méthode de protection des cultures contre une attaque ou une infestation par des arthropodes nuisibles, la méthode comprenant la mise en contact d'une culture avec une quantité efficace du point de vue pesticide d'au moins un biphénylsulfonamide de formule I ou un N-oxyde ou un sel acceptable en agriculture de celui-ci, selon la revendication 1.

20. Méthode de protection des semences contre une infestation par des arthropodes nuisibles et des racines et des pousses des semis contre une infestation par des arthropodes nuisibles, la méthode comprenant la mise en contact des semences ou des racines et des pousses des semis avec une quantité efficace du point de vue pesticide d'au moins un biphénylsulfonamide de formule I, ou d'un N-oxyde ou d'un sel acceptable en agriculture de celui-ci, selon la revendication 1.

21. Méthode de protection des matériaux non vivants contre une attaque ou une infestation par des arthropodes nuisibles, la méthode comprenant la mise en contact du matériau non vivant avec une quantité efficace du point de vue pesticide d'au moins un biphénylsulfonamide de formule I, ou d'un N-oxyde ou d'un sel acceptable en agriculture de celui-ci, selon la revendication 1.

22. Semis comprenant un biphénylsulfonamide de formule I, ou un N-oxyde ou un sel acceptable en agriculture de celui-ci, tel que défini dans la revendication 1, dans une quantité de 0,1g à 10 kg par 100 kg de semence.
